# EUROPEAN PATENT APPLICATION

(11) **EP 4 566 631 A1**
(43) Date of publication of application: **11.06.2025**
(21) Application number: 23214775.1
(22) Date of filing: 06.12.2023
(51) Int. Cl.: A61K 47/68, A61K 47/61, A61K 47/60, A61K 47/62, A61P 35/00, C07K 16/28

(54) **NEW ANTIBODY DRUG CONJUGATE AS WELL AS METHODS OF PRODUCTION AND USES THEREOF**

(71) Applicant: Heidelberg Pharma Research GmbH, 68526 Ladenburg (DE)
(72) Inventor: The designation of the inventor has not yet been filed
(74) Representative: Ullrich & Naumann PartG mbB

(57) **Abstract**

The present invention relates to new antibody-drug-conjugates (ADCs) comprising a favourable modular linker linking the pharmaceutically active drug substance with the antibody. The invention further relates to methods of producing the antibody drug conjugate as well as to uses thereof and pharmaceutical compositions comprising the same. Embodiments of the invention have been particularly developed as antibody drug conjugates for use in the treatment of Guanylate Cyclase 2C (GUCY2C) positive gastrointestinal cancers, and will be described hereinafter with reference to this application. However, it will be appreciated that the invention is not limited to this particular field of use.

## Description

### FIELD OF THE INVENTION

The present invention relates to new antibody-drug-conjugates (ADCs) comprising a favourable modular linker linking the pharmaceutically active drug substance with the antibody. The invention further relates to methods of producing the antibody drug conjugate as well as to uses thereof and pharmaceutical compositions comprising the same. Embodiments of the invention have been particularly developed as antibody drug conjugates for use in the treatment of Guanylate Cyclase 2C (GUCY2C) positive gastrointestinal cancers, and will be described hereinafter with reference to this application. However, it will be appreciated that the invention is not limited to this particular field of use.

### BACKGROUND OF THE INVENTION

Aside from surgical resection and radiation therapy, chemotherapy remains as the therapeutic option predominantly utilized in the treatment of cancer. However, it is also well-established that chemotherapy - through its limited selectivity and specificity towards cancer cells - typically offers only a small therapeutic window for the administration of highly toxic doses of chemotherapeutic agents, which regularly lead to severe side effects for the patients through the toxic effect on non-cancer cells, while still only conveying a low efficacy towards malign cancer cells.

Targeted drug therapy solutions have been developed to increase the specific delivery of cytotoxic drug substances directly to malign cancer cells. In particular, antibody drug conjugates (ADCs) provide new treatment options that allow for the delivery of drug substances to malign cancer cells with high specificity and with very limited or no effects on non-cancer cells.

Antibody-drug conjugates (ADCs), typically consist of an antibody covalently linked to a cytotoxic drug substances via a synthetic linker. While (a) the antibody ensures the high degree of specificity and (b) the cytotoxic drug substance ensures the effect on the cancer cells once delivered, (c) the linker compound used to conjugate the antibody and the drug substance plays an important role as it can significantly affects the therapeutic index, efficacy and pharmacokinetics of an ADC.

In particular, the linker compound must (i) ensure high plasma stability of the conjugate in circulation to prevent premature drug release and consequential undesired systemic off-target effects; (ii) maintain the properties of the antibody as well as of the cytotoxic drug substance, (iii) provide a high degree of aqueous solubility of the conjugate to allow for the delivery of hydrophobic/lipophilic drug substances and to prevent aggregation of the ADCs in a pharmaceutical composition; and (iv) ensure appropriate exposure of the targeted cancer cells to the cytotoxic drug substance to maximize the therapeutic effect.

The lipophilic nature of many cytotoxic drug substances can adversely affect the properties of an ADC to the extent that the payloads are not efficiently delivered to the target cells. Modulating ADC properties by affixing polar groups in linkers may reduce aggregation during conjugation, and improve physiochemical properties of the ADCs leading to improved pharmacokinetics (PK) and an improved therapeutic index of the ADC (see, for example, Nature Biotechnology, 2015, 33, 733-735). Modulation of the linker to effect a change in the polarity or charge of the final metabolite may improve activity toward multidrug resistant (MDR) cells owing to better retention of the payloads inside the cells (see, for example, Cancer Res; 70(6) Mar. 15, 2010, p2528). The chemical moieties on linkers, e.g., polyethylene glycol (PEG), sugar, or sulfuric acid groups (See, for example, WO2014062697, US20100323973), not only affect the conjugation efficiency and the ease of production of ADCs, but also often remain as part of the metabolites and thus affect the ADC's activity and safety.

With respect to the delivery of the cytotoxic drug substance at the target site, non-cleavable as well as cleavable linkers have been developed. Non-cleavable linkers, e.g. comprising thioether or maleimido-caproyl linkages, typically require internalisation and subsequent lysosomal enzymatic degradation of the entire TBDC by the target cell to release the cytotoxic drug substance. Cleavable linkers can be divided into the groups of chemically cleavable linkers and enzymatically cleavable linkers. Chemically cleavable linkers typically rely on pH sensitivity (lysosomal/endosomal release of cytotoxic drug substance) or reduction sensitivity (cytoplasmic release of cytotoxic drug substance) while various enzymatically cleavable are known that rely on enzymatic release of the cytotoxic drug substance, typically within the lysosomal compartment of the target cell. A combination of a cleavable peptide structure and a self-immolative spacer between the targeting-binding antibody and the drug of an ADC, for example, has been described to be applicable to a payload carrying a primary or secondary amine. Jeffrey et al. 2005 (Design, Synthesis, and in Vitro Evaluation of Dipeptide-Based Antibody Minor Groove Binder Conjugates. J. Med. Chem. Vol.48: 1344-1358). Notwithstanding, in many instances, the cytotoxic drug substance released still contains linker-derived adducts. In some instances, clever design of the cleavable linkage allows for a positive modification of the drug substance. In others, efficacy of the drug substance is negatively impacted by residual linker adducts.

However, the therapeutic efficacy of an ADC is not only dependent on the appropriate release of the cytotoxic drug substance but also on the effective bio-conjugation connecting the drug substance to the antibody and, further, on the number molecules of the cytotoxic drug substance successfully linked to a single antibody, i.e. the Drug-Antibody-Ratio (DAR). A low DAR is known to decrease ADC efficacy and requires a highly toxic drug substance to be conjugated, whereas a high DAR has been linked to potential ADC instability leading to increased systemic effects, a reduced ADC half-life and a general alteration of the pharmacokinetics of the molecule. However, given that stochastic chemistries have typically been applied to conjugate drug substances to antibodies, even FDA-approved ADCs are composed of a mixture of conjugates, which are heterogeneous with respect to their DAR.

Furthermore, selection of the appropriate target and high antibody specificity greatly influence the likelihood of a new ADC being therapeutically effective. In this respect, cancer-specific or cancer-associated antigens must be identified that allow for the generation of antibodies that can target malign cancer cells with high specificity and substantially no off-target binding of non-cancer or healthy cells.

With respect to gastrointestinal cancers, it is noteworthy that they account for approximately 26% of all cancer diagnoses and are responsible for approximately 35% of cancer deaths worldwide. There were an estimated 4.8 million new cases of gastrointestinal (GI) cancers and 3.4 million related deaths, worldwide, in 2018.

Gastrointestinal cancers comprise esophageal, gastric, colorectal, liver, esophageal and pancreatic tumors, whereby colorectal cancer (CRC) is most frequent and contributes to about 10.2% of all new cases, followed by gastric cancer (5.7%), liver cancer (4.7%), esophageal cancer (3.2%) and pancreatic cancer (2.5%). CRC contributes to about 9% of all cancer related deaths, followed by gastric cancer (8.2%), liver cancer (8.2%), esophageal cancer (5.3%) and pancreatic cancer (4.5%) with about 3.4 million GI cancer deaths globally in 2018 (Arnold et al. (2020) Gastroenterology 159:335-349). Colorectal cancer (CRC) is thus, the third most common cancer worldwide after lung and breast cancer but the second leading cause of cancer death.

In women CRC is the second most common adult cancer and the third most common in men, and it is the fourth leading cause of cancer death.

The 5-year and 10-year survival rates are 65% and 58%, respectively, and incidence and mortality rates are 25% higher in men than in women. From 1975 to 2013, CRC incidence rate increased from 10-15 cases per 100,000 population of Americans between the age of 20-49 years of age. This is paralleled by an increase in CRC cases of about 20% in developing countries such as Argentina, Brazil, and China. Furthermore, 30% of all patients with CRC experience metastasis for which the prognosis still remains poor with a median 5-year survival of only 18.5% in the United States and 27.7% in Europe.

Despite the fact that the incidence rate has declined over the past 30 years due to improvements in screening and treatment paradigms it is alarming, that incidence rates in individuals younger than 50 years have been steadily increasing, with a 2.2% annual overall incidence rate rise between 2012-2016. The exact etiology for this paradigm shift is unknown, however, it is likely that this attributable to changes in diet and other lifestyle risk factors, or may be attributable to increased nonsystematic screening of young adults.

10%-20% of all patients with CRC possess a positive family history and -5% of all cases of CRC are linked to a known hereditary CRC syndrome detectable by germline. In some cases, increased sporadic CRC incidence is associated with longstanding inflammatory bowel disease and variable lifestyle factors such as physical inactivity, unhealthy diet, smoking, obesity, and heavy alcohol consumption.

Differences in clinical outcomes and drug responsiveness of CRC treatment are dependent on the location of cancer along the colon and rectum. Relevant contributing factors include their distinct physiological functions, gut microbiome, regionally resident immune cell types, dietary carcinogens, timing of disease detection. In addition, ontogeny factors may contribute to disease severity and treatment outcome:
The proximal (right) large intestine (cecum, ascending colon, and the transverse colon) derives from the embryonic midgut, whereas the distal (left) large intestine (splenic flexure, descending colon, sigmoid colon, and rectum) derives from the embryonic hindgut. These ontogenetic differences are associated with differential gene expression patterns along the proximal-distal axis. In women and in African-Americans, proximal sporadic colon tumors, are more frequently diagnosed with an incidence of 51%-62% of cases which also show a higher TNM stage at first diagnosis, display a pattern with high levels of genome-wide promoter hypermethylation referred to as CpG island methylator phenotype (CIMP), exhibit microsatellite instability (MSI) due to deficient DNA mismatch repair mechanisms (dMMR), are more frequently mutated in KRAS and BRAF and have a worse prognosis in terms of survival. Distal colorectal tumors are more likely to present with chromosomal instability (CIN) and show a more favorable prognosis.

Genomic profiling of CRC has revealed significant intratumoral and intertumoral heterogeneity resulting from the accumulation of genetic mutations and chromosomal aberrations during disease initiation and progression. Genomic instability in CRC presents as one of two major forms: CIN and MSI. CRC lacking CIN or MSI is classified as genome stable (GS) CRC.

In GS CRC, the DNA repair genes and tumor suppressors are likely to be transcriptionally silenced through CIMP, though a large proportion of MSI CRCs and a small population of CIN CRCs are also CIMP-positive, and about 10% of CRCs are negative for CIN, MSI, or CIMP.

Chromosome instability (CIN) CIN is characterized by chromosomal numerical alterations (aneuploidy) and structural alterations (somatic copy number alterations, deletions, insertions, amplifications, or loss of heterozygosity), occurring in 65%-70% of sporadic CRCs. Nearly all CIN tumors show activated Wnt signaling, and 80% harbor mutational inactivation of APC, a negative regulator of the Wnt pathway. Mutational inactivation/deletion of TP53 occurs in 60% of CIN tumors and p53 loss of function directly drives CIN and provides a permissive context for genome instability mechanisms.

With respect to genome instability, combined telomere dysfunction and p53 deficiency is a major CIN mechanism, as revealed by the occurrence of anaphase bridges in early-stage carcinomas of human CRC and spontaneous CRC occurrence in telomerase-deficient p53 mutant mice.

Microsatellite instable (MSI) CRC is characterized by the presence of microsatellites, DNA sequences which containing repetitive motifs that tend to accumulate higher mutation rates than other genomic regions. MSI is the phenotypic manifestation of dMMR resulting from mutational inactivation of MMR genes, including MLH1, MSH2, MSH3, MSH6, PMS2 and Exo1 (De' Angelis et al. Acta Biomed 2018 Dec 17;89(9-S):97-101).

Other important factors that contribute to the etiology of CRC are somatic genetic alterations which activate key signal transduction pathways that promote the proproliferative state of CRC cancer cells. In CRC, the major proproliferative signaling pathways are the EGFR-RAS and WNT-β-catenin pathways.

EGFR signaling EGFR activation triggers downstream RAS/RAF/MEK/ERK and PI3K/AKT signaling cascades which ultimately lead to proliferation. Mutations in EGFR itself are rare in CRC with 1% of cases of CRC being attributable to a mutation in CRC (Barber et al N Engl J Med 351: 2883) and instead shows overexpression in ~80% of CRCs. Enhanced EGFR activation can occur via post-translational modifications involving methylation of R198 and R200 by protein arginine methyltransferase 1 (PRMT1), which enhances its binding to EGF and consequent signaling activation, even in the presence of the EGFR inhibitor.

It has recently been demonstrated that hepatocyte growth factor (HGF), the ligand of the MET-receptor, can fully replace EGF in the outgrowth of single Lgr5+ mouse intestinal stem cells (ISCs) to intestinal organoids, comprising all differentiated intestinal cell lineages. In addition, HGF and EGF were equally effective in promoting expansion of *Apc*-mutant mouse organoids, while *Met* deletion in ISCs in vivo attenuated stem-cell fitness and the formation of *Apc*-driven intestinal adenomas. These findings indicate that EGFR and MET signaling have overlapping and partially redundant, functionality in the mouse intestinal mucosa. Recent results also indicate that HGF/MET signaling can fully overcome EGFR inhibition in human ISCs and CRC stem cells, allowing expansion of single normal ISCs, as well as *APC-*mutant cells into organoids, even in the presence of EGFR-inhibition (Joosten et al Gastroenterology 2019 Oct;157(4):1153-1155).

Another factor that is decisive for the clinical effectiveness of EGFR blockade is the mutational status of its downstream signaling components, specifically, gain-of-function mutations in RAS, RAF, MEK, or ERK, which can maintain cancer cell proliferation and survival upon EGFR inhibition. Activating mutations in KRAS, NRAS, or HRAS are collectively present in -50% of cases of CRC; these mutations involve codons 12 or 13 and, less frequently, codons 61, 117, or 146. With respect to the RAS pathway in CRC, the RAS effector, BRAF, is mutated in 10%-15% of early-stage CRC and around 5% in stage IV CRC, in the hotspotcodon 600 (V600E). Clinically, the inhibition of BRAF alone showed limited activity in metastatic BRAFV600E CRC owing to EGFR-mediated reactivation of MAPK signaling leading to the approval of combinations of EGFR inhibitors with BRAF and MEK inhibitors that have now become the standard of care in patients with metastatic BRAF mutant tumors.

Screening for CRC can be done via either direct visualization method such as colonoscopy, CT colonography, flexible sigmoidoscopy, flexible sigmoidoscopy with fecal immunochemical test (FIT) or via stool-based tests such as guaiac-based fecal occult blood test, FIT, or multi-targeted stool DNA test or via serology test such ad SEPT9 DNA test.

Once the diagnosis of CRC is made, staging is an important factor as it determines treatment options for the patient. For tumor staging the Tumor, node, metastasis (TNM) system from the combined American Joint Committee on Cancer (AJCC)/Union for International Cancer Control (UICC) is a commonly used staging system which defines the following cancer stages:
Standard conventional treatments for CRC are surgery, chemotherapy and radiotherapy. Depending on the localization and progression of the disease, these treatments can be used in combination. Treatment options include for example for stage I surgical resection alone. For stage III curative surgery is followed with adjuvant chemotherapy as standard of care. Total mesorectal excision (TME) through laparoscopic and transanal surgery approaches are often the options for localized cancer and whenever the tumor location is easy to access. However, complete removal of all cancer cells is often not possible. About 66% of stage II and and 61% of stage III colon and rectal patients have to undergo further treatments with adjuvant chemotherapy and/or radiotherapy. These treatments have many side effects due to their unspecificity and cytotoxicity toward any cells that are growing and dividing. Despite recent improvements in the diagnostic and treatment options, 54% of patients relapse even after neoadjuvant treatment. Thus, it is crucial to have more alternative and effective treatments to treat CRC patients.

Current chemotherapy for CRC includes both single-agent therapy, which is mainly fluoropyrimidine (5-FU)-based, and multipleagent regimens containing one or several drugs, including oxaliplatin (OX), irinotecan (IRI), and capecitabine (CAP or XELODA or XEL), whereby the combined therapy regimens FOLFOX (5-FU+OX), FOXFIRI (5-FU+IRI), XELOX or CAPOX (CAP+OX), and CAPIRI (CAP+OX) represent the main approaches in first-line treatment. However, chemotherapy is associated with certain limitations, such as systemic toxicity, unsatisfying response rate, as well as unpredictable innate and acquired resistance, and low tumor-specific selectivity.

Patients with metastatic colorectal cancer (mCRC) have a 5-year overall survival rate of <10%.

Currently, the main chemotherapy agents used for the treatment of the affected patients are fluoropyrimidine (intravenous or oral)-based as either single-agent treatments, such as intravenous 5-fluorouracil (5-FU) and oral capecitabine (CAP) or as multiple-agent regimens, including the combinations FOLFOX (5-FU and oxaliplatin), FOLFIRI (5-FU and irinotecan), XELOX/CAPOX (CAP and oxaliplatin), CAPIRI (CAP and irinotecan). Beyond traditional chemotherapy, the EGFR-targeted agents, cetuximab and panitumumab, are approved for first-line treatment of mCRC . combinations of cetuximab with FOLFIRI or panitumumab with FOLFOX have significantly enhanced therapeutic efficacy. However, treatment decisions for early-stage mCRC do not consider *BRAF* or *KRAS* mutations, given the dramatically poor prognosis conferred by these mutations as seen in clinical trials. Although the survival rate of patients with mCRC has improved in recent years, the response and prognosis of patients with the aforementioned mutations are still poor.

The current lack of effective therapies and non-specific cytotoxic limitations have led investigators to transition towards the development of predictive, preventative, and personalized medicine strategies to improve the treatment of CRC and mCRC given the substantial unmet need for prospective therapies for patients with *BRAF-* or *KRAS*-mutant mCRC.

Gastric cancer is the second leading cause of death from malignant disease worldwide, with especially high mortality rates in East, South, and Central Asia; Central and Eastern Europe; and South America. Treatment options for gastric cancer vary with cancer stages: Very early cancer can typically be treated by surgery. Potentially resectable cancer may typically be treated by surgery as first treatment, with either subtotal gastrectomy or total gastrectomy. Nearby lymph nodes and possibly parts of nearby organs may be removed as well. In addition, patients may receive chemotherapy alone or chemo plus radiation therapy (chemoradiation), for example patients may receive 5-fluorouracil (5-FU) in combination with cisplatin (CDDP) (FP therapy). Other possible treatment regimens include epirubicin, cisplatin, and fluorouracil (ECF therapy), or regimens in which fluorouracil was replaced by capecitabine (ECX therapy), or ECF therapy in which cisplatin is replaced by oxaliplatin (EOF therapy) may be used. Alternatively, both cisplatin and fluorouracil can be replaced by oxaliplatin and capecitabine (EOX therapy).

In metastatic gastric cancers treatment is aimed at controlling the growth of the cancer and may include chemotherapy alone, chemotherapy plus immunotherapy, or chemotherapy along with radiation therapy. The combination of a platinum and fluoropyrimidine (5-FU) is the global standard first-line chemotherapy regimen within a non-curative setting. After platinum and 5-FU failure paclitaxel plus ramucirumab has been established as standard second-line therapy. However, treatment-related neuropathy, progression during or rapid recurrence following perioperative FLOT regimen (fluorouracil, oxaliplatin, docetaxel) have raised the demand for a taxane-free second-line therapy. Trifluridine/tipiracil has recently been approved for patients with metastatic gastric cancer. In addition to chemotherapy, patients may be treated with immune checkpoint inhibitors, such as ipilimumab (anti-CTLA-4), nivolumab (anti-PD-1), pembrolizumab (anti-PD-1), or atezolizumab (anti-PD-L1).

Diagnostically gastrointestinal endoscopy is indispensable for the diagnosis of gastric cancer, as well as staging laparoscopy (SL), which is a minimally invasive, brief procedure that only requires a small incision. The advantages of SL include providing an accurate diagnosis of peritoneal dissemination and extraserosal invasion, and the ability to perform peritoneal lavage for cytology. In patients with advanced gastric cancer for whom imaging does not yield a diagnosis, peritoneal lavage cytology obtained before treatment can be very important for treatment planning. Peritoneal lavage cytology obtained by SL for evaluation of peritoneal dissemination is thought to be useful for assessing the effects of neoadjuvant chemotherapy and/or immunotherapy.

The most frequent liver tumor is hepatocellular carcinoma (HCC) among all primary liver cancers, accounting for 75%-85% of cases. HCC is usually diagnosed at an advanced stage, for which there remain limited effective treatment options. Until 2007, there were no effective treatment options for patients diagnosed with advanced-stage disease or patients who transitioned into advanced-stage disease after other treatments failed. Sorafenib was the first systemic drug approved by the U.S. Food and Drug Administration (FDA) as standard treatment for advanced HCC between 2007 and 2016. In recent years other small molecule drugs have been deleveloped which include lenvatinib, regorafenib, or cabozantinib which have either been approved or are still undergoing clinical trials. These drugs are currently also tested in combination with immune checkpoint inhibitors, such as nivolumab, or pembrolizumab. In addition, combinations of immune checkpoint inhibitors and angiogenesis inhibitors, such as atezolizumab and bevacizumab are being tested.

Esophageal cancer as one form of GI cancer is being treated according to its stage, which may include endoscopic treatments, as well as chemotherapy and radiation therapy. Common drugs and drug combinations that are being used to treat esophageal cancer but are usually not given with radiation include ECF: epirubicin (Ellence), cisplatin, and 5-FU (especially for gastroesophageal junction tumors), DCF: docetaxel (Taxotere), cisplatin, and 5-FU, or trifluridine and tipiracil (Lonsurf), a combination drug in pill form.

For pancreatic cancer several chemotherapy regimens have been approved the most widely used and best-studied agent is Gemcitabine. Gemcitabine is often administered in combination with albumin-bound (Nab) Paclitaxel, which improved survival time compared to Gemcitabine monotherapy. Alternative treatment options include the multi-drug regimen FOLFIRINOX (5-Fluorouracil, Leucovorin, Irinotecan, and Oxaliplatin). Also, for pancreatic cancer, combination therapies using immune checkpoint inhibitors such as pemprolizumab, or nivolumab in combination with gemcitabine, nab-paclitaxel, or capecitabine are being clinically assessed.

Guanylyl Cyclase 2C (GUCY2C, EC: 4.6.1.2) is a member of a family of receptor-enzyme proteins synthesizing guanosine 3',5'-cyclic monophosphate (cyclic GMP; cGMP). GUCY2C is a transmembrane receptor for the endogenous hormonal ligands:guanylin and uroguanylin. Ligand binding to the extracellular receptor catalyzes the conversion of GTP into cyclic GMP (cGMP) and initiates downstream cGMP-related signaling pathways, which are implicated in the regulation of intestinal homeostatic processes such as epithelial cell proliferation, differentiation, and apoptosis (Lisby et al. Expert Rev Precis Med Drug Dev. 2021; 6(2): 117-129.).

GUCY2C is specifically expressed by intestinal epithelial cells. GUCY2C regulates the dynamic progression of cells along the crypt-villus and crypt-surface axis, coordinating homeostatic processes including proliferation, DNA repair, metabolic programming, lineage-specific cell fate, and epithelial-mesenchymal interactions organizing that axis. Given the major role of GUCY2C in maintaining epithelial regeneration, dysregulation of the GUCY2C-cGMP axis promotes pathologies that include inflammatory bowel disease and bowel transit disorder, in addition to colorectal cancer. Importantly, silencing the GUCY2C signaling axis is associated with colorectal tumorigenesis through the loss of ligand binding.

GUCY2C protein can be detected near-universally (>95%) in all primary and metastatic human colorectal tumors regardless of anatomical location or grade, as well as a subset of gastroesophageal and pancreatic tumors, but not in tumors arising outside the GI tract. GUCY2C was also shown to be expressed in esophageal, gastric and pancreatic tumors (Danaee et al (2017) PLoS One. 2017; 12(12): e0189953).

Given its unique expression pattern in colorectal cancer (CRC) and metastatic colorectal cancer (mCRC) various approaches have been taken to utilize GUCY2C in the treatment of these diseases which have included cancer vaccines using GUCY2C, its use as a target for CAR-T cell therapy (Magee et al. Cancer Immunol Res. 6(5), 509-516 (2018)), or the use of therapeutic antibodies and antibody-drug conjugates targeting GUCY2C. WO2013/163633 A1 discloses anti-GUCY2C antibodies and anti-GUCY2C ADCs comprising auristatin E (MMAE) and auristatin F MMAE (MMAF) which inhibit mitosis by inhibiting tubulin polymerization. Clinical trials of the anti-GUCY2C ADC TAK264 (MLN0264, 5F9vcMMAE) which employed MMAE as payload have been terminated. WO2021/205325 A1 discloses anti-CD3-GUCY2C bispecific antibodies and respective use in cancer therapy to induce a cytolytic T cell response in GUCY2C positive target cells.

While these efforts indicate that GUCY2C-directed therapies may be effective, a lack of approved GUCY2C-directed therapies, and in particular of approved GUCY2C-directed ADCs still exists.

Accordingly, there is a need in the art for improved antibody drug conjugates (ADCs), which mediate stable conjugation of a cytotoxic drug substance to the antibody, while allowing for easy adaptation of the ADC's Drug-Antibody-Ratio (DAR) and/or aqueous solubility depending on the cytotoxicity and hydrophobicity of the drug substance to be conjugated, respectively. Even more so, there is a need for such improved ADC's having high potency and a good efficacy profile in the context of treating gastrointestinal cancer.

It is an object of the present invention to overcome or ameliorate at least one of the disadvantages of the prior art, or to provide a useful alternative. In particular, it is an object of the present invention to provide such improved ADC's having high potency and a good efficacy profile in the context of treating gastrointestinal cancer.

### SUMMARY OF THE INVENTION

As indicated above, the present invention aims to provide improved ADCs having a linker structure that is particularly suitable to provide ADCs with high potency and a good efficacy profile in the context of treating gastrointestinal cancer.

The here-described ADCs have a linker with an overall modular architecture providing a selection of beneficial linker functionalities for a high potency ADC. Specifically, the modular linker used in the ADC of the present invention comprises two functional units of a cleavable peptide sequence and a self-immolative spacer, wherein a solubility enhancing group and the cytotoxic drug substance are bound to each one of the self-immolative spacers in physical proximity. Further, each functional unit is connected to a corresponding branching unit in the multimeric core of the linker via a defined chemical spacer. Given that the multimeric core of the ADC of the present invention comprises two branching units each, which are connected to one cytotoxic drug substance, respectively, the linker has a defined defined Degree-of-Labelling (DOL) of 2.

Finally, the linker is conjugated to the antibody via a further chemical spacer extending from the multimeric core. Conjugation of the drug substance-carrying linker to the antibody occurs via the terminus of the spacer extending from the multimeric core by way of a reaction of a terminal maleimide group of the spacer with a sulphur atom of a cysteine residue in the constant heavy chain of the antibody.

Beneficially, thiol reactions are highly efficient and typically proceed rapidly under mild conditions. This means that thiol-reactive groups can form conjugates quickly and with a high degree of completeness. Further, the thioether bonds formed between thiol groups and thiol-reactive groups are stable under physiological conditions. While they are less prone to hydrolysis or degradation compared to some other types of chemical bonds, further stability of the conjugate can, e.g., be achieved through incorporation of a basic amino group adjacent to the maleimide as described in Lyon et al. Nat. Biotechnol. 2014, 32, 1059-1062. Accordingly, in the ADCs of the invention, the reaction product is typically a succinimidyl thioether or a derivative thereof and stably links the antibody with the drug substance-carrying linker.

Accordingly, in a first aspect the present invention relates to an antibody-drug conjugate (ADC) of Formula (I) 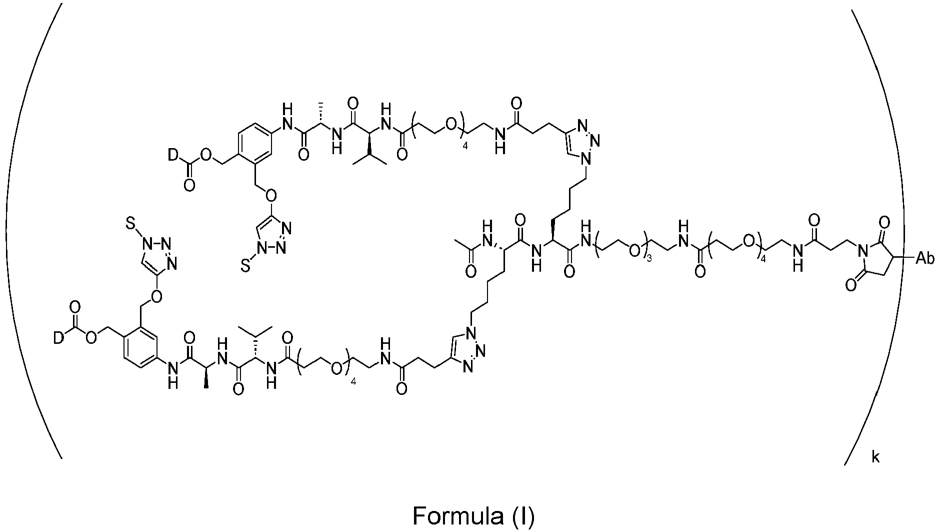 or a pharmaceutically acceptable salt or solvate thereof; wherein:
k is an integer from 2 to 4;
Ab is an antibody;
D is a pharmaceutically active substance selected from 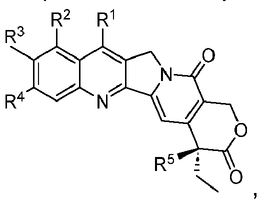 wherein:
   - R² and R¹ together form 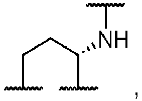 R³ is -CH₃, R⁴ is -F and R⁵ is -OH; or
   - R¹ is -H, R² is -CH₂N(CH₃)₂ ,R³ -OH, R⁴ is -H and R⁵ is -O-; or
   - R¹ is -CH₂CH₃, R² is -H ,R³ is 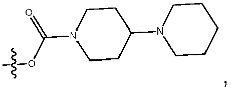 and R⁴ is -H and R⁵ is -O-; or
   - R¹ is -H, R² is -NO₂ ,R³ -H, and R⁴ is -H and R⁵ is -O-; or
   - R¹ is -CH₂CH₂NHCH(CH3)₂, R² is -H , R³ -H, and R⁴ is -H and R⁵ is -O-; or
   - R' is -CH₂CH₃, R² is -H ,R³ -OH, and R⁴ is -H and R⁵ is -O-;
S is a solubility enhancing group and comprises an alpha cyclodextrin, a beta-cyclodextrin, a gamma-cyclodextrin, a polysarcosine, a PEG, or a polyalcohol made from carbohydrates.

Preferably, D is 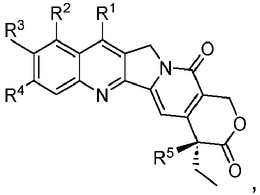 wherein:
- R² and R¹ together form 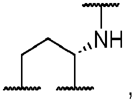 R³ is -CH₃, R⁴ is -F and R⁵ is -OH; or
- R¹ is -H, R² is -CH₂N(CH₃)₂, R³ -OH, R⁴ is -H and R⁵ is -O-; or
- R¹ is -CH₂CH₃, R² is -H R³ is 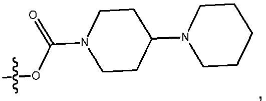 and R⁴ is -H and R⁵ is -O-.

In preferred embodiments D is 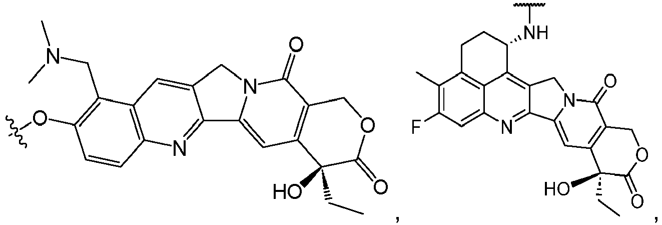 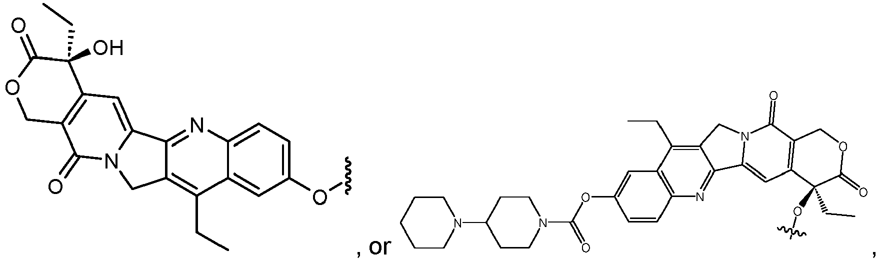 wherein the wavy line indicates the attachment site of D to the linker as disclosed herein.

Most preferably, D is

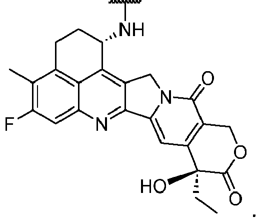

In some embodiments, the antibody is a monoclonal antibody that recognizes and binds a target sequence or epitope of Guanylate Cyclase 2C (GUCY2C).

One specifically preferred embodiment of the antibody-drug conjugate of Formula (I) of the first aspect is: 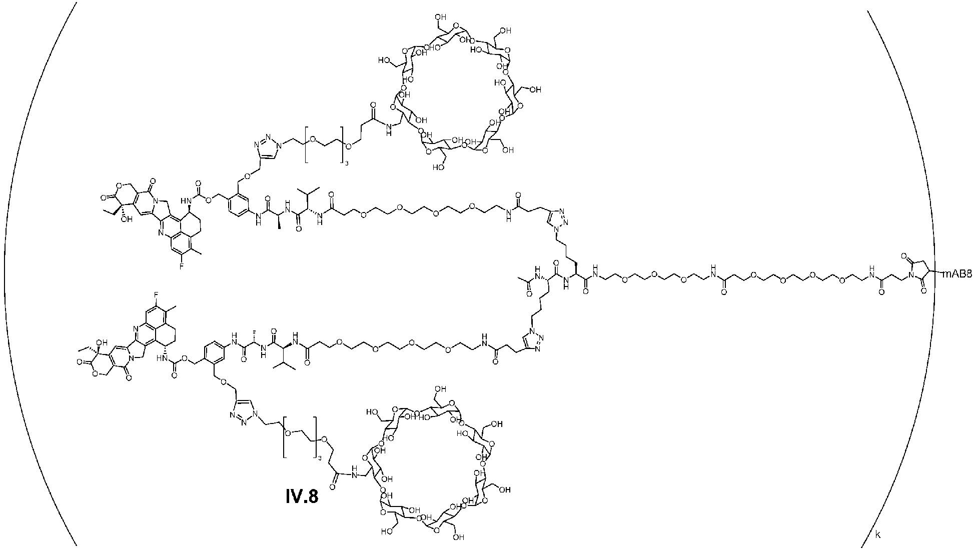 wherein k is 2.

Another specifically preferred embodiment of the antibody-drug conjugate of Formula (I) of the first aspect is: 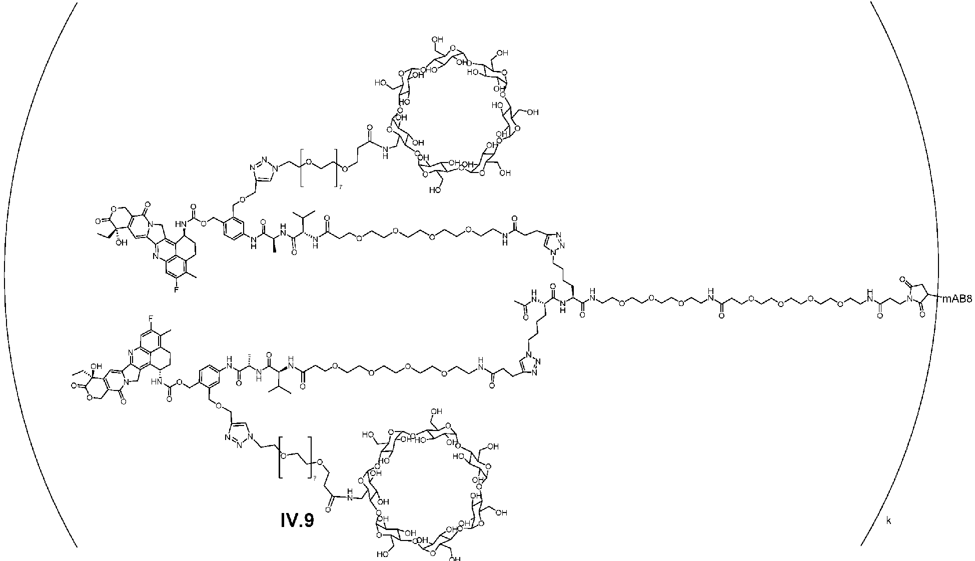 wherein k is 2.

Another specifically preferred embodiment of the antibody-drug conjugate of Formula (I) of the first aspect is: 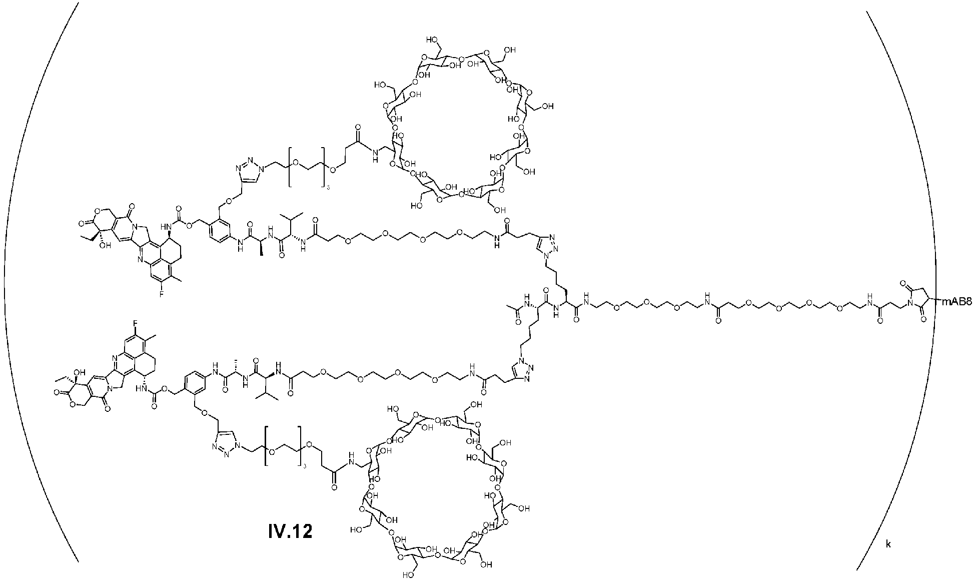 wherein k is 2.

Another specifically preferred embodiment of the antibody-drug conjugate of Formula (I) of the first aspect is: 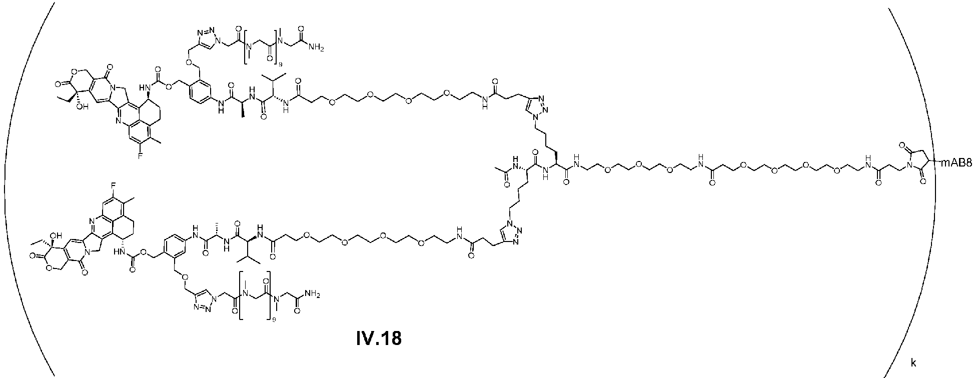 wherein k is 2.

Another specifically preferred embodiment of the antibody-drug conjugate of Formula (I) of the first aspect is: 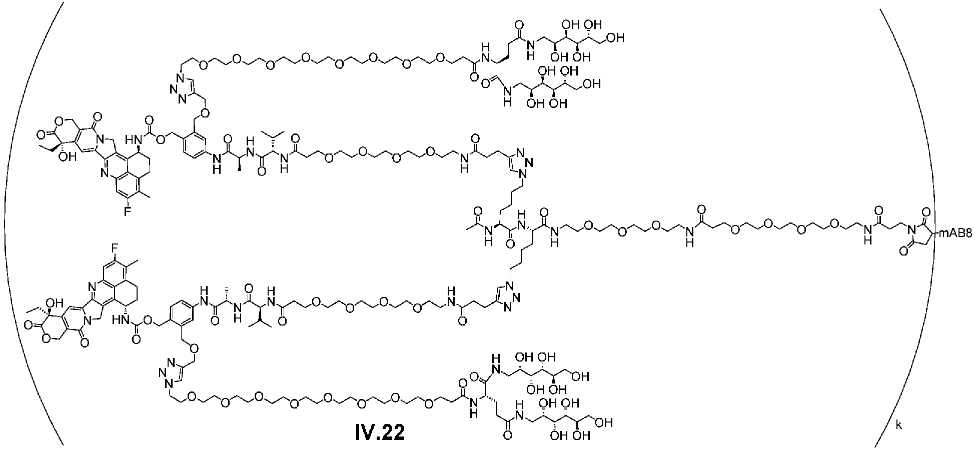 wherein k is 2.

In a second aspect, the present invention relates to the antibody-drug conjugate of the first aspect for use as a medicament, preferably for use in the treatment of cancer, more preferably for the treatment of gastrointestinal cancer, such as colorectal cancer (CRC), metastatic colorectal cancer (mCRC) or pancreatic cancer.

In a third aspect, the present invention relates to a pharmaceutical composition comprising the antibody-drug conjugate of the first aspect.

Accordingly, the second and third aspects of the present invention also encompass methods of treating cancer, preferably of treating gastrointestinal cancer, such as colorectal cancer (CRC), metastatic colorectal cancer (mCRC) or pancreatic cancer, wherein the method comprises administering a therapeutically effective amount of the antibody-drug conjugate of the first aspect or of the pharmaceutical composition of the third aspect to a patient in need thereof. Similarly, at the second aspect of the present invention also encompasses use of the antibody-drug conjugate of the first aspect in the manufacture of a medicament for the treatment of cancer, preferably for the treatment of gastrointestinal cancer, such as colorectal cancer (CRC), metastatic colorectal cancer (mCRC) or pancreatic cancer.

In such embodiments, the gastrointestinal cancer is typically characterised by the expression of a target sequence or epitope of a Guanylate Cyclase 2C (GUCY2C) antigen.

### FIGURES

Embodiments of the invention will now be described, by way of example only, with reference to the accompanying drawings in which:

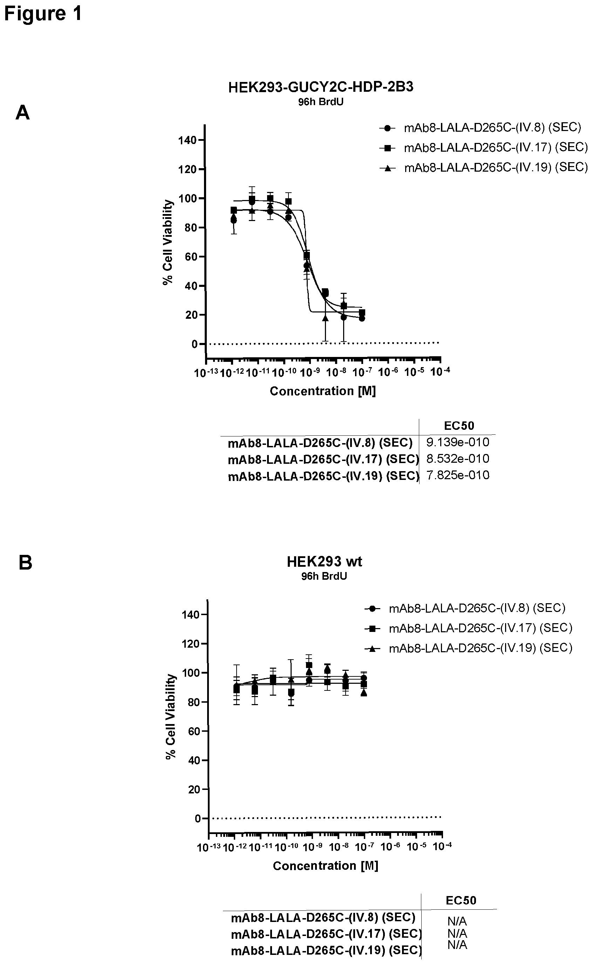

- **Fig. 1**: In vitro cytotoxicity of an anti-GCC ADC comprising the linker-payload conjugate (IV.8), in comparison to anti-GCC ADCs comprising the linker-payloads (IV.17) and (IV.19) on **(A)** HEK cells overexpressing human GUCY2C (HEK293-GUCY2C-HDP-2B3 cells, or **(B)** HEK293 wildtype cells. SEC indicates purification of ADC by size-exclusion chromatography. "N/A" indicates that no EC50 calculation was done.

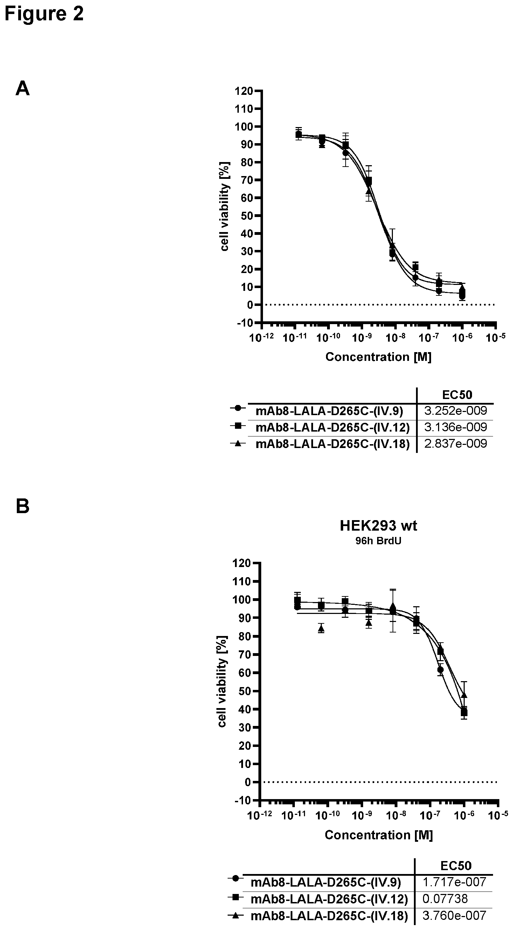

- Fig. 2: In vitro cytotoxicity of anti-GCC ADCs comprising the linker-payload conjugates (IV.9), (IV.12) and (IV.18) on (A) HEK cells overexpressing human GUCY2C (HEK293-GUCY2C-HDP-2B3 cells, or (B) HEK293 wildtype cells.

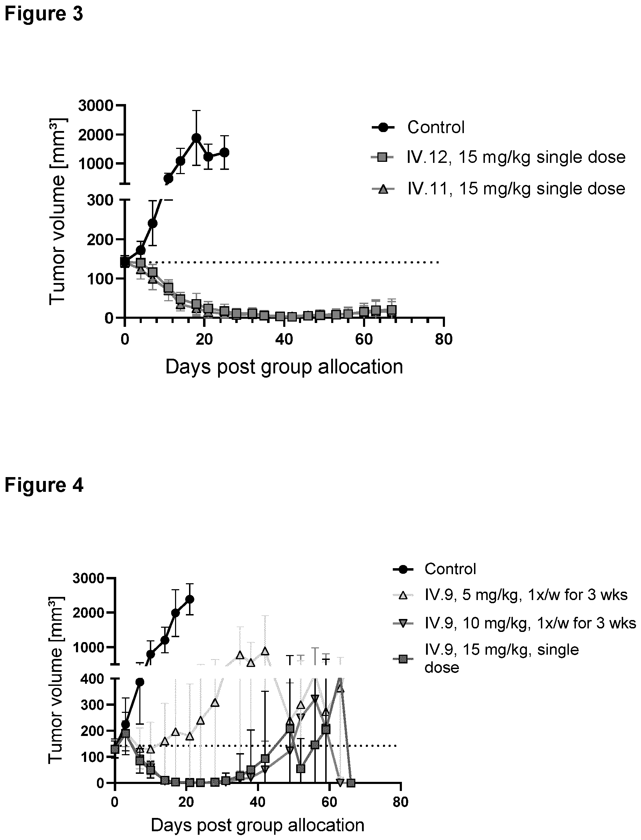

- Fig. 3: shows mean tumor volume [mm³] depicted from day 0 to day 67 post group allocation in mice treated either with vehicle control or with the Exatecan-TBDCs of the invention IV.12 and IV.11, as a single dose of 15 mg/kg on day 1 (n = 10 animals/group, mean depicted ± SD).
- Fig. 4: shows mean tumor volume [mm³] depicted from day 0 to day 66 post group allocation in mice treated either with vehicle control or with the Exatecan-TBDC of the invention IV.9 either as a single dose of 15 mg/kg on day 1 or 1x/week for 3 weeks with 10 or 15 mg/kg (n = 10 animals/group, mean depicted ± SD).

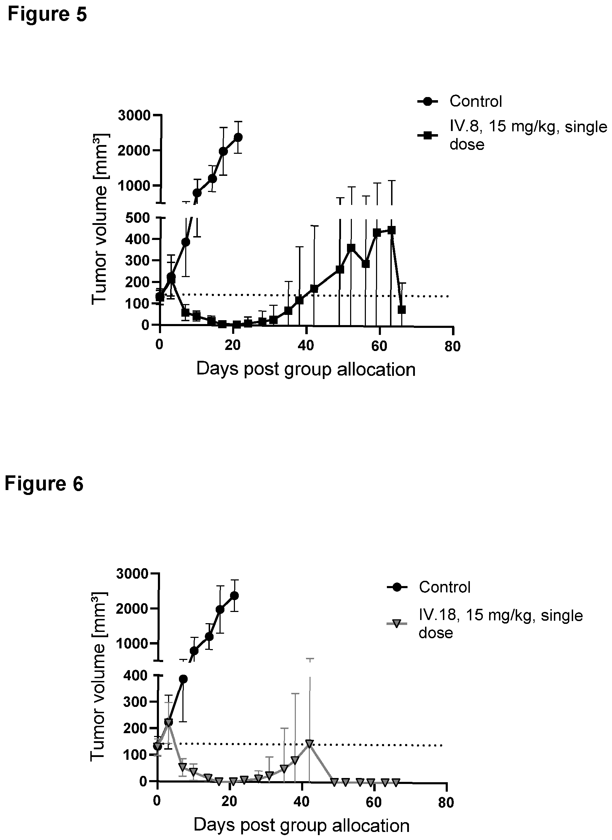

- Fig. 5: shows mean tumor volume [mm³] depicted from day 0 to day 66 post group allocation in mice treated either with vehicle control or with the Exatecan-TBDC of the invention IV.8 as a single dose of 15 mg/kg on day 1 (n = 10 animals/group, mean depicted ± SD).
- Fig. 6: shows mean tumor volume [mm³] depicted from day 0 to day 66 post group allocation in mice treated either with vehicle control or with the Exatecan-TBDC of the invention IV.18 as a single dose of 15 mg/kg on day 1 (n = 10 animals/group, mean depicted ± SD).

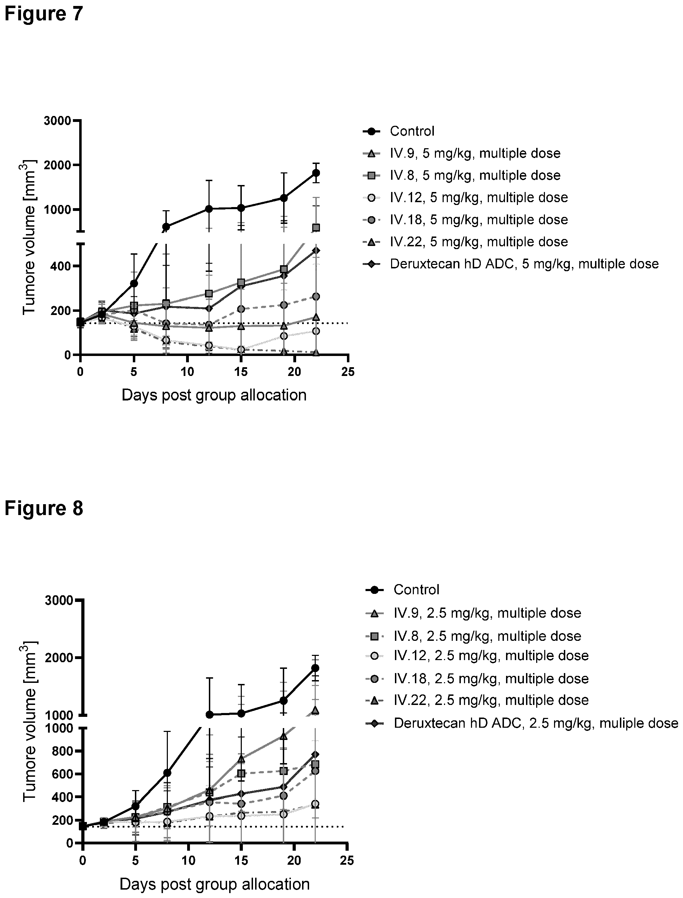

- Fig. 7: shows mean tumor volume [mm³] depicted from day 0 to day 22 post group allocation in mice treated either with vehicle control or with the Exatecan-TBDCs of the invention IV.9, IV.8, IV.12 or IV.18 or IV.20 1x/week for 3 weeks with 5 mg/kg each (n = 10 animals, mean depicted ± SD). "hD" denotes high DAR (DAR10).
- Fig. 8: shows mean tumor volume [mm³] depicted from day 0 to day 22 post group allocation in mice treated either with vehicle control or with the Exatecan-TBDCs of the invention IV.9, IV.8, IV.12 or IV.18 or IV.20 1x/week for 3 weeks with 2.5 mg/kg each (n = 10 animals, mean depicted ± SD).

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kolbl, H. eds. (1995), Helvetica Chimica Acta, CH-4010 Basel, Switzerland). The definitions of the chemical groups as used herein unless specified otherwise shall have the meaning and be defined as provided in "Compendium of Chemical Terminology" ("Gold Book") published by the International Union of Pure and 13 Applied Chemistry (IUPAC) version 2.3.3, goldbook.iupac.org, ISBN: 0-9678550-9-8), the content of which is hereby incorporated by reference.

Notwithstanding, in order to provide a clear and consistent understanding of the specification and claims, as well as of the scope to be given to certain terms, the following definitions are provided.

### Definitions

The term **"modular linker"** in the context of the present disclosure refers to a linker that comprises at least one molecule of a cytotoxic drug substance (also referred to simply as drug or payload), several further structural as well as functional modules (also referred to as spacers or functional units or groups), including at least one solubility enhancing group and a branching unit, as well as a reactive group suitable to stably connect the modular linker to a target-binding moiety such as an antibody.

The term **"solubility enhancing group"** in the context of the present disclosure refers to a module within the modular linker, which at least counters - at best negates - hydrophobic/lipophilic properties of a cytotoxic drug substance such as to provide the modular linker comprising a specific payload with a high degree of aqueous solubility. A variety of solubility enhancing groups suitable for the incorporation into the modular linker of the present disclosure are well-known to the skilled person and include, without limitation: (a) polar functional groups, such as hydroxyl (-OH), amine (-NH₂), and carboxyl (-COOH) groups; polar sulfonate (-SOsH) groups; polymeric chains, such as hydrophilic polyethylene glycol (PEG) chains; surfactant groups, such as alkyl chains; aromatic ring structures; halogen substituents such as the addition of fluorine or chlorine; ester (-COO-) or ether (-O-) groups; and cyclodextrins/cyclic oligosaccharides.

In the context of the present disclosure, the term **"self-immolative spacer"** refers to a spacer that is stably connected to the cytotoxic drug substance but undergoes triggered self-immolation, i.e. decomposition, such that its connection to the cytotoxic drug substance is abolished and the drug substance is released without any adducts or traces of the self-immolative spacer itself. In some embodiments a change in pH can trigger self-immolation of the spacer and release of the payload. In other embodiments, the self-immolative spacer is also connected to an enzymatically cleavable peptide sequence and enzymatic cleavage of the peptide triggers self-immolation of the spacer and release of the payload. An illustrative example of a enzymatically triggered self-immolative spacer is a p-aminobenzyl (PAB) spacer connected to an enzymatically cleavable Val-Ala dipeptide.

In the context of the present disclosure, the term **"cleavable linker"** refers to a linker that is cleavable (i) by an enzyme, or (ii) in a reducing environment. Further, the terms "enzymatically cleavable" or "cleavable by an enzyme" or the likes convey that the peptide, structure, sequence or spacer in question can be cleaved by an enzyme, particularly by a lysosomal protease, such as Cathepsin B, resulting in the release of the pharmaceutically active drug substance/payload.

In the context of the present disclosure, the term **"branching unit"** refers to a structure within a modular linker that allows for a furcation of the compound into a branched structure, wherein each branch is typically connected to at least one molecule of the cytotoxic drug substance via a self-immolative spacer. Therefore, a modular linker typically comprises as many drug molecules as branches. As such, a modular linker comprising between 1 and 10, preferably between 2 and 8, 2 and 6 or 2 and 4 branches, typically comprises between 1 and 10, preferably between 2 and 8, 2 and 6 or 2 and 4 cytotoxic drug substance molecules, respectively.

As used herein, the term **"antibody"** refers to a protein consisting of one or more polypeptide chains encoded by immunoglobulin genes or fragments of immunoglobulin genes or cDNAs derived from the same. Said immunoglobulin genes include the light chain kappa, lambda and heavy chain alpha, delta, epsilon, gamma and mu constant region genes as well as any of the many different variable region genes.

The basic immunoglobulin (antibody) structural unit is usually a tetramer composed of two identical pairs of polypeptide chains, the light chains (L, having a molecular weight of about 25 kDa) and the heavy chains (H, having a molecular weight of about 50-70 kDa). Each heavy chain is comprised of a heavy chain variable region (abbreviated as VH or V_{H}) and a heavy chain constant region (abbreviated as CH or C_{H}). The heavy chain constant region is comprised of three domains, namely CH1, CH2 and CH3. Each light chain contains a light chain variable region (abbreviated as VL or V_{L}) and a light chain constant region (abbreviated as CL or C_{L}). The VH and VL regions can be further subdivided into regions of hypervariability, which are also called complementarity determining regions (CDR) interspersed with regions that are more conserved called framework regions (FR). Each VH and VL region is composed of three CDRs and four FRs arranged from the amino terminus to the carboxy terminus in the order of FR1, CDR1, FR2, CDR2, FR3, CDR3, FR4. The variable regions of the heavy and light chains form a binding domain that interacts with an antigen. The constant regions (Fc regions) are not directly involved in the binding of the antibody to the antigen but exhibit various effector functions such as participation in antibody dependent cell- mediated cytotoxicity (ADCC), phagocytosis via binding to Fcg receptor, half-life/clearance rate via neonatal Fc receptor (FcRn) and complement activation via the C1q component, leading to the chemotactic, opsonic and, potentially in the case of a viable cellular antigen target, cytolytic actions of complement. Human antibodies of the IgG1 class are the most potent in activating the complement system and are therefore the desirable isotype for the therapeutic antibodies of the ADCs of the present invention.

Human Fcg receptors include FcgR (I), FcgRlla, FcgRllb, FcVRllla and neonatal FcRn for which it was demonstrated that a common set of IgG1 residues is involved in binding all FcgRs, while FcgRII and FcgRIII utilize distinct sites outside of this common set (Shields et al. (2001) J. Biol. Chem 276: 6591-6604). One group of IgG1 residues reduced binding to all FcgRs when altered to alanine: Pro-238, Asp-265, Asp-270, Asn-297 and Pro-239 (numbering according to EU numbering system, Edelman et al., Proc. Natl. Acad. Sci. USA; 63 (1969) 78-85.). All are in the IgG CH2 domain and clustered near the hinge joining CH1 and CH2. While FcgR1 utilizes only the common set of IgG1 residues for binding, FcgRII and FcgRIII interact with distinct residues in addition to the common set. Alteration of some residues reduced binding only to FcgRII (e.g. Arg-292) or FcgRIII (e.g. Glu- 293). Some variants showed improved binding to FcgRII or FcgRIII but did not affect binding to the other receptor. The neonatal FcRn receptor is believed to be involved in both antibody clearance and the transcytosis across tissues (see: Junghans (1997) Immunol. Res 16: 29-57; and Ghetie et al. (2000) Annu. Rev. Immunol. 18: 739-766). Human IgG1 residues determined to interact directly with human FcRn includes Ile253, Ser254, Lys288, Thr307, Gln311, Asn434 and His435.

The terms **"CDR", "CDR_{L}1", "CDR_{L}2", "CDR_{L}3", "CDR_{H}1", "CDR_{H}2", "CDR_{H}3"** as used herein follow the Kabat numbering convention (Kabat et al., Sequences of Proteins of Immunological Interest, 4th Ed., U.S. Department of Health and Human Services, National Institutes of Health (1987)). However, although the Kabat numbering convention for amino acid residues in variable domain sequences and full length antibody sequences is used throughout this specification, it will be apparent to those skilled in the art that there are alternative numbering conventions for amino acid residues in variable domain sequences and full length antibody sequences. There are also alternative numbering conventions for CDR sequences, for example those set out in Chothia et al. (1989) Nature 342: 877-883. The structure and protein folding of the antibody may mean that other residues based on the numbering system used are considered part of the CDR sequence and would be understood to be so by a skilled person, however, these differences functionally do not imply altered or different antigen-binding of the respective antibody. Other numbering conventions for CDR sequences available to a skilled person include "AbM" (University of Bath) and "contact" (University College London) methods.

The CDRs are most important for binding of the antibody or the antigen binding portion thereof. The FRs can be replaced by other sequences, provided the three-dimensional structure which is required for binding of the antigen is retained. Structural changes of the construct most often lead to a loss of sufficient binding to the antigen.

Antibodies typically bind specifically to their cognate antigen with high affinity, reflected by a dissociation constant (KD) of 10⁻⁵ to 10⁻¹¹ M or less. Any K_{D} greater than about 10⁻⁴ M is generally considered to indicate nonspecific binding.

As used herein, an antibody that " **specifically recognizes and** binds" to an antigen refers to an antibody that binds to the antigen and substantially identical antigens with high affinity, which means having a K_{D} of 10⁻⁷ M or less, preferably 10⁻⁸M or less, even more preferably 5×10⁻⁹ M or less, and most preferably from about 10⁻⁸, 10⁻⁹ M to about 10⁻¹⁰ M, 10⁻¹¹ or less, or e.g. from about 10⁻¹⁰ M to about 10⁻¹¹ M or less, but does not bind to unrelated (e.g. structurally or sequence unrelated) antigens with an affinity equal to the affinity for the specific target.

The antibody of the ADC of the invention which may e.g. also be referred to as immunoglobulin may be from any of the commonly known isotypes, including but not limited to IgA, secretory IgA, IgG and IgM. The IgG isotype is divided in subclasses in certain species: IgG1, IgG2, IgG3 and IgG4 in humans, and IgG1, IgG2a, IgG2b and IgG3 in mice.

The term **"antibody fragment"** or **"antigen-binding fragment"** as used herein refers to an antibody fragment or analog of an antibody which retains the binding specificity of the parent anti-GUCY2C antibody as disclosed herein and comprises a portion (for example, one or more CDRs) or variable region of the antigen binding region of the parent antibody. The antibody fragment is, for example, Fab, Fab', F(ab')₂, Fv fragment, sc-Fv, unibody, diabody, linear antibody, nanobody, domain antibody, or multispecific antibody fragment formed from the antibody fragment. A Fab fragment consists of the CH1 and variable regions of one light chain and one heavy chain. The heavy chain of a Fab molecule cannot form a disulfide bond with another heavy chain molecule. A Fab' fragment as contains a light chain and a portion of a heavy chain that contains the VH domain, the CH1 domain, and the region between the CH1 and CH2 domains. A F(ab')₂fragment contains two light chains and two heavy chains containing a portion of the constant region between the CH1 and CH2 domains, thereby forming an interchain disulfide bond between the two heavy chains. A F(ab')₂ fragment is composed of two Fab' fragments held together by the disulfide bond between the two heavy chains. A "Fv fragment" contains variable regions from both the heavy and light chains, but lacks the constant region. The term "single-chain antibody" is a single-chain recombinant protein formed by connecting the heavy chain variable region VH and the light chain variable region VL of an antibody through a connecting peptide. It is the smallest antibody fragment with a complete antigen-binding site. The term "domain antibody fragment" is an immunoglobulin fragment with immunological functions that only contains a heavy chain variable region or a light chain variable region chain. In some cases, two or more VH regions are covalently linked to a peptide linker to form a bivalent domain antibody fragment. The two VH regions of the bivalent domain antibody fragment can target the same or different antigens.

In the context of the present disclosure, the term **"reactive moiety for conjugation to an antibody"** refers to a chemically reactive group within the linker that that can react with a correspondingly reactive group of the antibody under suitable conditions such that the resulting reaction product covalently conjugates the remaining linker compound to the antibody.

In the context of the present disclosure, the term **"reaction product of the modular linker and a correspondingly reactive group of an antibody"** refers a moiety formed as the result of a reaction between the reactive moiety of the modular linker of the invention and a correspondingly reactive group of an antibody. For example, if the reactive moiety of the modular linker of the invention is a maleimide, the reaction product resulting from the reaction of the maleimide with the sulphur atom of the cysteine residue of the target-binding moiety, is a succinimidyl thioether or a derivative thereof. Further, if the reactive moiety of the modular linker of the invention is a group that selectively reacts with the thiol group of a free cysteine of the target-binding moiety, i.e. if the reactive moiety of the modular linker is a "thiol-reactive moiety", the reaction product is typically selected from: thiol-substituted acetamide; thiol-substituted succinimide; thiol-substituted succinamic acid; thiol-substituded heteroaryl, particularly thiol-substituted benzothiazole, thiol-substituted phenyltetrazole and thiol-substituted phenyloxadiazole; and a disulfide, wherein one sulphur atom is derived from a cysteine residue of the target-binding moiety.

As used herein, a **"derivative"** of a compound refers to a species having a chemical structure that is similar to the compound, yet containing at least one chemical group not present in the compound and/or deficient of at least one chemical group that is present in the compound. The compound to which the derivative is compared is known as the "parent" compound. Typically, a "derivative" may be produced from the parent compound in one or more chemical reaction steps.

In the context of the present disclosure, the term a **"pharmaceutically acceptable salt or solvates"** includes acid addition salts, formed with inorganic acids such as hydrochloric acid, hydro bromic acid, sulphuric acid, nitric acid, phosphoric acid, and the like; or formed with organic acids such as glycolic acid, pyruvic acid, lactic acid, malonic acid, malic acid, inaleic acid, fumaric acid, tartaric acid, citric acid, 3-(4-hydroxybenzoyl)benzoic acid, cinnamic acid, mandelic acid, methane sulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, lauryl sulphuric acid, gluconic acid, glutamic acid, salicylic acid, muconic acid, and the like. The term further includes basic addition salts formed with the conjugate bases of any one of the above-listed inorganic acids, wherein the conjugate bases comprise a cationic component selected from Na⁺, K⁺, Mg²⁺, Ca²⁺, and NHgR'_{4-g}⁺; in which R' is a C₁-₃ alkyl and g is a number selected from among 0, 1, 2, 3, or 4 as well as solvent addition forms (i.e. solvates) of the respective acid addition salt.

As used herein, **"treat", "treating"** or **"treatment"** of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, a **"patient"** means any mammal or bird who may benefit from a treatment with the target-binding drug conjugates described herein. Preferably, a "patient" is selected from the group consisting of laboratory animals (e.g. mouse or rat), domestic animals (including e.g. guinea pig, rabbit, chicken, pig, sheep, goat, camel, cow, horse, donkey, cat, or dog), or primates including human beings. It is particularly preferred that the "patient" is a human being.

A **"therapeutically effective amount"** is an amount of a therapeutic agent sufficient to achieve the intended purpose. The effective amount of a given therapeutic agent will vary with factors such as the nature of the agent, the route of administration, the size and species of the animal to receive the therapeutic agent, and the purpose of the administration. The effective amount in each individual case may be determined empirically by a skilled artisan according to established methods in the art.

In addition to the above definitions, and unless the context clearly requires otherwise, throughout the description and the claims, the words **"comprise", "comprising",** and the like are to be construed in an inclusive sense as opposed to an exclusive or exhaustive sense; that is to say, in the sense of "including, but not limited to".

Further, reference throughout this specification to **"one embodiment", "some embodiments"** or **"an embodiment"** means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives **"first", "second", "third",** etc., to describe a common object, merely indicate that different instances of like objects are being referred to, and are not intended to imply that the objects so described must be in a given sequence, either temporally, spatially, in ranking, or in any other manner.

As used herein, the term **"exemplary"** is used in the sense of providing examples, as opposed to indicating quality. That is, an **"exemplary embodiment"** is an embodiment provided as an example, as opposed to necessarily being an embodiment of exemplary quality.

Several documents are cited throughout the text of this specification. Each of the documents cited herein (including all patents, patent applications, scientific publications, manufacturer's specifications, instructions, GenBank Accession Number sequence submissions etc.), whether supra - or infra, is hereby incorporated by reference in its entirety to the extent possible under the respective patent law.

### Antibody-drug-conjugates (ADC) of the invention

The inventors of the present invention have developed new antibody-drug-conjugate (ADC) comprising a modular linker that harbours various modules (i.e. also referred to as groups, units, elements, etc.), and which have been implicated ADC function and efficacy.

The linker's modular architecture can accommodate pharmaceutically active drug substances having various physico-chemical properties as well as stably link the drug substance carrying linker to an antibody such that a robust and reliable ADC can be provided.

In addition, and as will become apparent form the further description below, through the conjugation chemistries employed in the ADC, both a homogeneous Degree-of-Labeling (DOL) of 2 per linker molecule and a Drug-Antibody-Ratio (DAR) of 4 to 8 is readily achievable.

Therefore, it was found that, in a relatively simple fashion, the ADC of the present disclosure provides for efficient targeting of selected cancer cells with the pharmaceutically active drug substance of the conjugate given the specificity of the antibody for a specific cancer-associated or cancer-specific antigen.

Accordingly, in a first aspect, the present invention relates to an antibody-drug conjugate (ADC) of Formula (I) 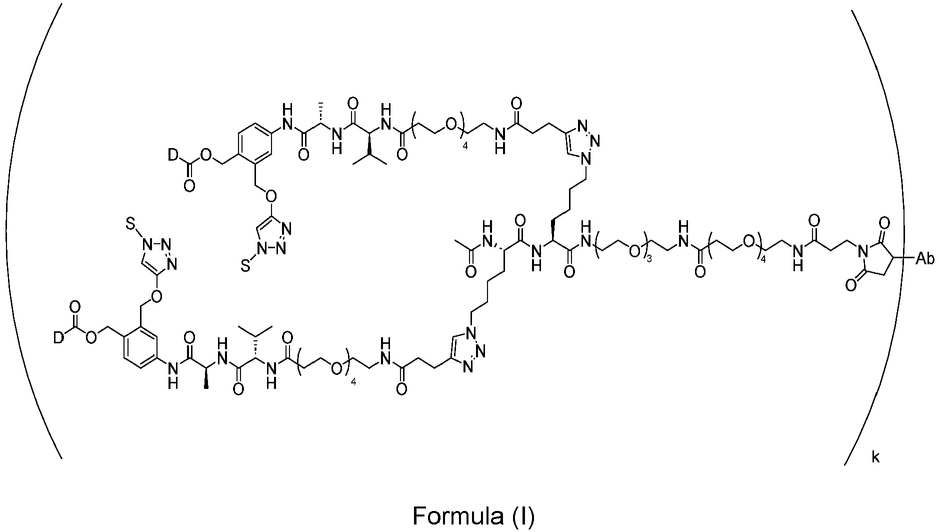 or a pharmaceutically acceptable salt or solvate thereof; wherein:
k is an integer from 2 to 4;
Ab is an antibody;
D is a pharmaceutically active substance selected from 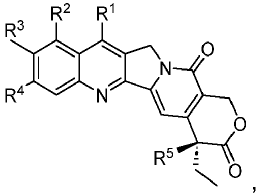 wherein:
   - R² and R¹ together form 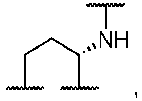 R³ is -CH₃, R⁴ is -F and R⁵ is -OH; or
   - R¹ is -H, R² is -CH₂N(CH₃)₂ ,R³ -OH, R⁴ is -H and R⁵ is -O-; or
   - R¹ is -CH₂CH₃, R² is -H ,R³ is 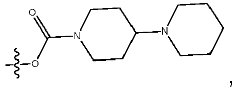 and R⁴ is -H and R⁵ is -O-; or
   - R¹ is -H, R² is -NO₂ ,R³ -H, and R⁴ is -H and R⁵ is -O-; or
   - R¹ is -CH₂CH₂NHCH(CH3)₂, R² is -H , R³ -H, and R⁴ is -H and R⁵ is -O-; or
   - R¹ is -CH₂CH₃, R² is -H ,R³ -OH, and R⁴ is -H and R⁵ is -O-;
S is a solubility enhancing group and comprises an alpha cyclodextrin, a beta-cyclodextrin, a gamma-cyclodextrin, a polysarcosine, a PEG, or a polyalcohol made from carbohydrates.

Particularly suitable pharmaceutically active drug substances D in the ADC of the present invention are camptothecins, a class of compounds that comprises both naturally-occurring as well as synthetic members, which have shown anti-cancer through inhibition of topoisomerase I thereby interfering with DNA replication and repair processes, leading to DNA damage and ultimately cell death in cancer cells.

In the ADC of the present invention, the pharmaceutically active drug substance D is a campthotecin compound selected from SN-38, topotecan, irinotecan, rubitecan, belotecan, SN38 and exatecan.

A notable member of this compound class is SN-38 (a derivative of camptothecin, generated as an active metabolite when the chemotherapeutic drug irinotecan / CPT-1 1 is metabolized in the body of the patient.

Topotecan and irinotecan are camptothecins also used as chemotherapeutic agents in the treatment of various cancers, including ovarian cancer, small cell lung cancer, and cervical cancer. As already indicated above, Irinotecan is a prodrug, which is administered in an inactive form and which converted into its active form (SN-38) within the body. Both Topotecan and irinotecan are typically administered intravenously.

Rubitecan remains an investigational chemotherapeutic agent of the camptothecin class, similar to irinotecan and topotecan. Rubitecan has been studied for its potential use in the treatment of various cancers, including colorectal cancer, ovarian cancer, and pancreatic cancer and has been extensively studied in cancer research.

Belotecan is a further camptothecin compound shown to inhibit topoisomerase 1 thereby disrupting essential DNA replication and repair processes leading to cancer cell death.

Exatecan is yet a further camptothecin compound (also known as DX-8951f or SG2000) suitable for inclusion in the ADC of the present invention.

In light of the above, preferably, D is 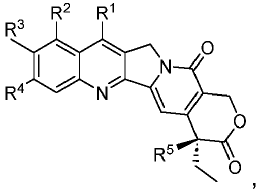 wherein:
- R² and R¹ together form 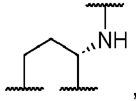 R³ is -CH₃, R⁴ is -F and R⁵ is -OH; or
- R¹ is -H, R² is -CH₂N(CH₃)₂, R³ -OH, R⁴ is -H and R⁵ is -O-; or
- R¹ is -CH₂CH₃, R² is -H R³ is 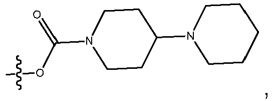 and R⁴ is -H and R⁵ is -O-.

Most preferably, D is

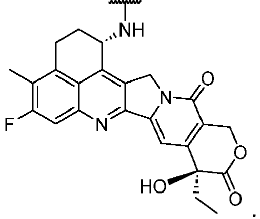

As briefly mentioned above, both the pharmaceutically active substance D and the solubility enhancing group S are connected to a self-immolative spacer of the modular linker of the ADC. Particularly, D is connected in para-position to the attached cleavable linker sequence to the self-immolate to of aminobenzyl spacer, while the solubility enhancing group S is connected in meta-position to the attached cleavable linker sequence. Accordingly, once the aminobenzyl spacer undergoes triggered self-immolation, i.e. decomposition, the pharmaceutically active drug substance D is released as a metabolite of the self-immolation reaction without any chemical moieties from the linker compound remaining. In such embodiments, the amino group of a para-aminobenzyl spacer serves as the electron donor in the electron cascade of the 1,6-self-immolation reaction releasing the cytotoxic drug substance D. Advantageously, this traceless release mechanism ensures that the cytotoxic drug substance is not altered through its connection to and subsequent release from the linker compound.

The solubility enhancing group S being linked to the self-immolative spacer in meta, while the cytotoxic drug substance is linked in the adjacent para position has been shown to be advantageous. Without wanting to be bound by theory, the close proximity of the solubility enhancing group S and the cytotoxic drug substance D likely leads to a better masking of the lipophilic/hydrophobic properties of the drug substance such as to further increase solubility of the modular linker compound.

Once the ADC recognizes and binds the target sequence or target epitope of the cancer-specific antigen, it is internalized by the cancer cell and becomes a substrate for lysosomal proteases. In the ADC of the present invention, the self-immolative spacer is linked to a cleavable di-peptide sequence, which is a target for such lysosomal proteases, namely for cysteine proteases of the cathepsin family, including cathepsin B, C, D, H, L, S and Z. Typically, the di-peptide sequence is a valine-alanine (Val-Ala) sequence, which is cleavable by cathepsin B. Once the di-peptide sequence is cleaved, the electron cascade of the self-immolation reaction is triggered, leading to the traceless release of the drug substance from the self-immolative spacer of the ADC.

In alternative instances, the peptide sequence may be a dipeptide selected from valine-citrulline (Val-Cit), or phenylalanine-lysine (Phe-Lys), or a tripeptide selected from glutamic acid-valine-alanine (Glu-Val-Ala) or glutamic acid-valine-citrulline (Glu-Val-Cit).

As already indicated above, an advantageous feature of the modular linker of the ADC of the present invention is its multimeric core comprising two sequential amino acid-based branching units, each indirectly connected to the dipeptide or tripeptide sequence and the self-immolative spacer carrying both the pharmaceutically active drug substance D and the solubility enhancing group S.

In embodiments of ADC of the first aspect, the branching unit distal to the conjugation site of the drug-carrying linker to the antibody carries a terminal acyl cap, wherein said acyl cap constitutes the terminus of the final branching unit in the modular linker's multimeric core. Further, each branching unit is separated from the dipeptide or tripeptide sequence preceding the self-immolative spacer carrying both D and S by a C₂-amide-PEG₄ spacer.

As also mentioned above, pharmaceutically active drug substances, including camptothecins, typically have an unfavorably low solubility in aqueous solutions such as in physiologic/systemic solutions. Therefore, in order to avoid undesirable aggregation of the ADCs of the invention, the modular linker necessarily comprises one solubility enhancing group S connected to each self-immolative spacer. Solubility enhancing group S may be an alpha-cyclodextrin, a beta-cyclodextrin, a gamma-cyclodextrin, a polysarcosine, a PEG, or a polyalcohol made from carbohydrates.

Cyclodextrins are cyclic oligosaccharides composed of glucose units arranged in a toroid (doughnut) shape. They have a hydrophobic interior and a hydrophilic exterior. This unique structure allows them to form inclusion complexes with hydrophobic or poorly soluble molecules and when a hydrophobic/lipophilic pharmaceutical drug substance is complexed with a cyclodextrin, its solubility in an aqueous solution is typically increased. Accordingly, a hydrophobic pharmaceutically active drug substance can be encapsulated within the hydrophobic pocket of the cyclodextrin, shielding it from the surrounding aqueous environment thereby enhancing its solubility. Notwithstanding, cyclodextrins are chemically compatible with a wide range of pharmaceutically active drug substances and typically are non-reactive with respect to molecules encapsulated via the formation of an inclusion complex. This allows for the maintenance of complex stability and integrity of the pharmaceutically active drug substance at the same time.

Further, cyclodextrins have been widely used in pharmaceuticals, food as well as cosmetics for many years and it is established that there overall safety and biocompatibility profiles are favorable.

Accordingly, cyclodextrins are particularly suitable solubility enhancing groups S in the context of the here-described linker compounds. For example, alpha-cyclodextrins (comprising six glucose molecules), beta-cyclodextrins (comprising seven glucose molecules) and gamma-cyclodextrins (comprising eight glucose molecules), when attached to an amide-PEG₁₋₁₂ spacer.

Therefore, in some embodiments of the ADC of the first aspect, S is selected from: 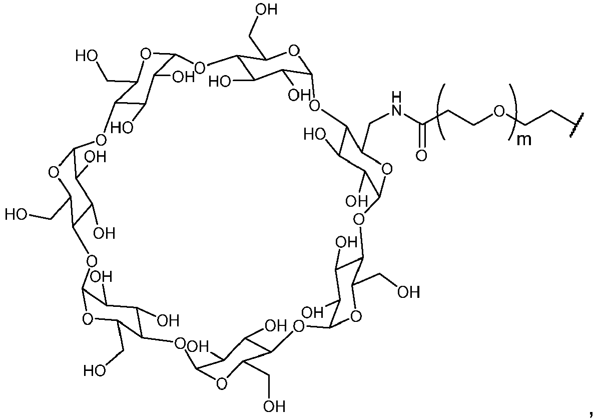 wherein m is 2 to 12, such as 2, 3, 4, 5, 6, 7, 8, 9, 10, 11 or 12; 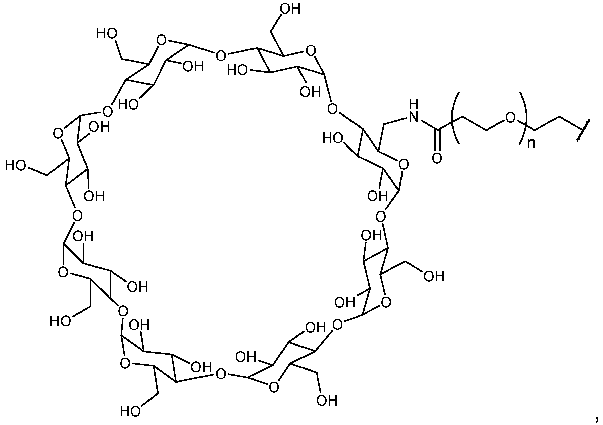 wherein n is 2 to 12, such as 2, 3, 4, 5, 6, 7, 8, 9, 10, 11 or 12; 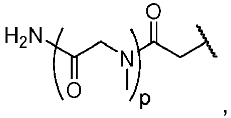 wherein p is 1 to 20, such as 2, 3, 4, 5, 6, 7, 8, 9, 10, 11, 12, 13, 14, 15, 16, 17, 18, 19 or 20;
or 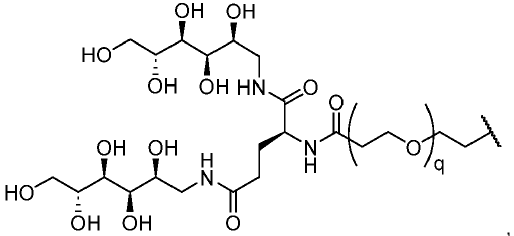 wherein q is 2 to 12, such as 2, 3, 4, 5, 6, 7, 8, 9, 10, 11 or 12.

In some embodiments, S is: 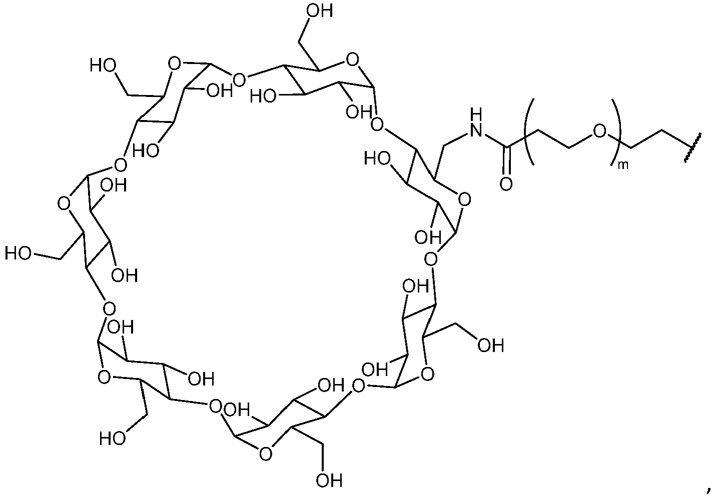 and m is 4.

Alternatively, S is: 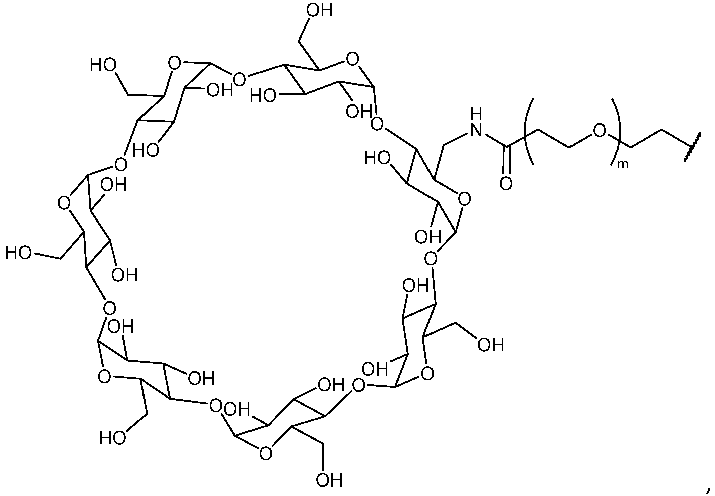 and m is 8.

Selecting the length of the PEG spacers of m = 4 or m = 8 for the above-shown beta-cyclodextrins is particularly advantageous when the pharmaceutical drug substance D is a camptothecin.

In further embodiments, S is: 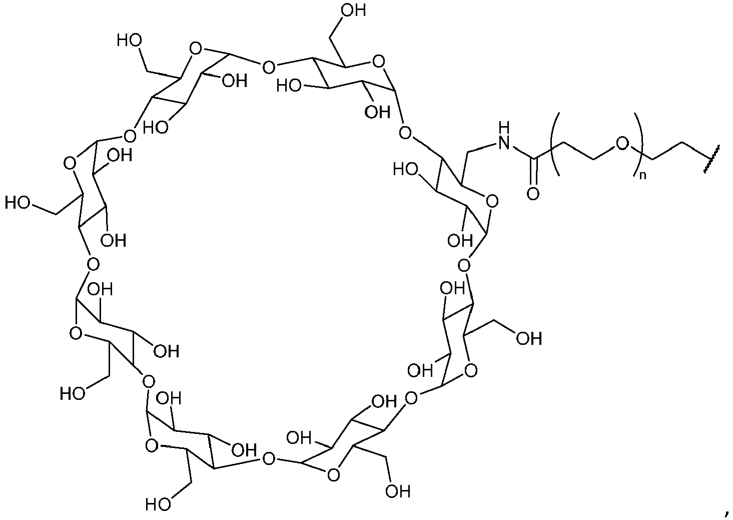 and n is 4.

Selecting the length of the PEG spacer of n = 4 for the above gamma-cyclodextrin is particularly advantageous when the pharmaceutical drug substance D is a camptothecin.

It is particularly advantageous to link a cyclodextrin-based solubility enhancing groups of the above illustrated types to the self-immolative spacer X via a PEG chain of the above indicated lengths. Such PEG chain lengths favour a conformational arrangement of the linker compound that allows for an inclusion complex placing the lipophilic drug substance into the pocket of the cyclodextrin ring structure substantially negating the hydrophobic/lipophilic properties of the camptothecin.

In yet further embodiments, S is: 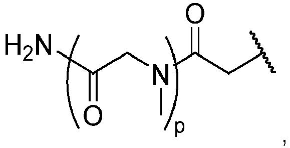 and p is 10.

In some embodiments, S is: 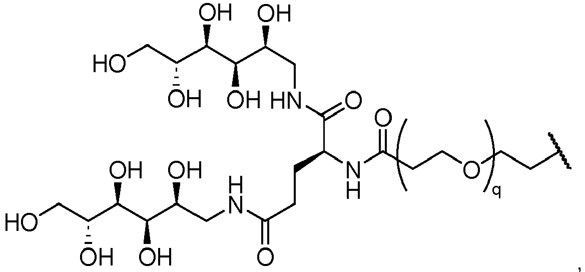 and q is 8.

As already indicated, the antibody of the ADC specifically recognises and binds a target sequence or epitope of an antigen, such as to mediate the ADC's cancer cell specificity. However, as also described above, preferably the antibody of the ADC of the first aspect is one of:
- a monoclonal antibody;
- a functional antibody fragment or antibody derivative such as a single-chain variable fragment (scFv), an antigen-binding fragment such as a Fab fragment, a F(ab'), fragment, a F(ab')2 fragment or a bi- or tri-specific antibody construct;
- a Diabody,
- a Camelid Antibody,
- a Domain Antibody,
- a Nanobody,
- a bivalent homodimer with two chains consisting of scFvs,
- a shark antibody,
- an antibody consisting of new-world primate framework sequences plus non-new world primate complementarity-determining regions (CDR) or
- a dimerised construct comprising a constant heavy chain 3 (CH₃) domain, a variable light chain (V_{L}) and a variable heavy chain (V_{H}).

Each one of the above-mentioned antibody formats must ensure highly specific recognition and binding of the target sequence or target epitope of the cancer-specific antigen such as to avoid undesirable off-target effects.

In the context of the present invention, where gastrointestinal cancer patients, such as colorectal cancer (CRC), metastatic colorectal cancer (mCRC) or pancreatic cancer patients are to benefit from the ADC of the first aspect, predominantly, the antibody typically is a monoclonal antibody that recognizes and binds a target sequence or epitope of Guanylate Cyclase 2C (GUCY2C), preferably human, or cynomolgus Guanylate Cyclase 2C, more preferably human Guanylate Cyclase 2C.

Highly specific anti-GUCY2C have been described in PCT/EP2023/080350, which is hereby incorporated by reference in its entirety.

Accordingly, in one embodiment of the first aspect, the antibody of the ADC is the monoclonal anti-GUCY2C antibody mAb1, which includes an antigen binding region comprising:
- an mAb1 light chain variable region (mAb1 V_{L}) complementarity-determining region (CDR_{L}) sequences CDR_{L}1 of SEQ ID NO: 1, CDR_{L}2 of SEQ ID NO: 2 and CDR_{L}3 of SEQ ID NO: 3 and
- an mAb1 heavy chain variable region (mAb1 V_{H}) CDR_{H}1 of SEQ ID NO: 4, CDR_{H}2 of SEQ ID NO: 5 and CDR_{H}3 of SEQ ID NO: 6.

More specifically, the mAb1 antibody includes an antigen binding region comprising:
- the mAb1 V_{L} comprises a framework region 1 (FR_{L}1) of SEQ ID NO: 14, CDR_{L}1 of SEQ ID NO: 1, a FR_{L}2 of SEQ ID NO: 15, CDR_{L}2 of SEQ ID NO: 2, a FR_{L}3 of SEQ ID NO: 16, CDR_{L}3 of SEQ ID NO: 3 and a FR_{L}4 of SEQ ID NO: 17; and
- the mAb1 V_{H} comprises a framework region 1 (FR_{H}1) of SEQ ID NO: 18, CDR_{H}1 of SEQ ID NO: 4, a FR_{H}2 of SEQ ID NO: 19, CDR_{H}2 of SEQ ID NO: 5, a FR_{H}3 of SEQ ID NO: 20, CDR_{H}3 of SEQ ID NO: 6 and a FR_{H}4 of SEQ ID NO: 21.

Further, the mAb1 antibody includes an antigen binding region comprising:
- the mAb1 V_{L} of SEQ ID NO: 32 and
- the mAb1V_{H} of SEQ ID NO: 33.

In another embodiment of the first aspect, the antibody of the ADC is the monoclonal anti-GUCY2C antibody mAb8, which includes an antigen binding region comprising:
- an mAb8 light chain variable region (mAb8 V_{L}) complementarity-determining region (CDR_{L}) sequences CDR_{L}1 of SEQ ID NO: 1, CDR_{L}2 of SEQ ID NO: 2 and CDR_{L}3 of SEQ ID NO: 7 and
- an mAb8heavy chain variable region (mAb8 V_{H}) CDR_{H}1 of SEQ ID NO: 4, CDR_{H}2 of SEQ ID NO: 5 and CDR_{H}3 of SEQ ID NO: 6.

More specifically, the mAb8 antibody includes an antigen binding region comprising:
- the mAb8 V_{L} comprises a framework region 1 (FR_{L}1) of SEQ ID NO: 22, CDR_{L}1 of SEQ ID NO: 1, a FR_{L}2 of SEQ ID NO: 15, CDR_{L}2 of SEQ ID NO: 2, a FR_{L}3 of SEQ ID NO: 23, CDR_{L}3 of SEQ ID NO: 7 and a FR_{L}4 of SEQ ID NO: 24; and
- the mAb8 V_{H} comprises a framework region 1 (FR_{H}1) of SEQ ID NO: 18, CDR_{H}1 of SEQ ID NO: 4, a FR_{H}2 of SEQ ID NO: 19, CDR_{H}2 of SEQ ID NO: 5, a FR_{H}3 of SEQ ID NO: 20, CDR_{H}3 of SEQ ID NO: 6 and a FR_{H}4 of SEQ ID NO: 21.

Further, the mAb8 antibody includes an antigen binding region comprising:
- the mAb8 V_{L} of SEQ ID NO: 34 and
- the mAb8 V_{H} of SEQ ID NO: 35.

In yet another embodiment of the first aspect, the antibody of the ADC is the monoclonal anti-GUCY2C antibody mAb41, which includes an antigen binding region comprising:
- an mAb41 light chain variable region (mAb41 V_{L}) complementarity-determining region (CDR_{L}) sequences CDR_{L}1 of SEQ ID NO: 8, CDR_{L}2 of SEQ ID NO: 9 and CDR_{L}3 of SEQ ID NO: 10 and
- an mAb41 heavy chain variable region (mAb41 V_{H}) CDR_{H}1 of SEQ ID NO: 11, CDR_{H}2 of SEQ ID NO: 12 and CDR_{H}3 of SEQ ID NO: 13.

More specifically, the mAb41 antibody includes an antigen binding region comprising:
- the mAb41 V_{L} comprises a framework region 1 (FR_{L}1) of SEQ ID NO: 25, CDR_{L}1 of SEQ ID NO: 8, a FR_{L}2 of SEQ ID NO: 26, CDR_{L}2 of SEQ ID NO: 9, a FR_{L}3 of SEQ ID NO: 27, CDR_{L}3 of SEQ ID NO: 10 and a FR_{L}4 of SEQ ID NO: 28; and
- the mAb41 V_{H} comprises a framework region 1 (FR_{H}1) of SEQ ID NO: 29, CDR_{H}1 of SEQ ID NO: 11, a FR_{H}2 of SEQ ID NO: 30, CDR_{H}2 of SEQ ID NO: 12, a FR_{H}3 of SEQ ID NO: 31, CDR_{H}3 of SEQ ID NO: 13 and a FR_{H}4 of SEQ ID NO: 21.

Further, the mAb41 antibody includes an antigen binding region comprising:
- the mAb41 V_{L} of SEQ ID NO: 36 and
- the mAb41 V_{H} of SEQ ID NO: 37.

In some embodiments of the antibody drug conjugate according to the first aspect, the antibody is: (a) a humanized or human antibody; and/or (b) an IgG type antibody, preferably an IgG1 antibody; and/or (c) a recombinant antibody.

According to one embodiment, the anti-GUCY2C antibody of the ADC is a recombinant antibody or antigen-binding fragment thereof. The term "recombinant" or "recombinantly produced" as used herein refers to a protein, such as the anti-GUCY2C antibody or antigen-binding fragment of the invention as disclosed herein, that have been expressed in heterologous cells. For example, the anti-GUCY2C antibody or antigen-binding fragment of the invention may be expressed in prokaryotic or eukaryotic cells. Prokaryotic cells for the expression of the antibody of the ADC of the invention include e.g. gram-negative bacteria such as E.coli, or gram-positive bacteria such as Bacillus subtilis. The use of heterologous prokaryotic expression systems may be particularly useful for the expression of antigen-binding fragments of the anti-GUCY2C antibody of the ADC of the invention and may be done according to established protocols known in the art, such as e.g. Kwong and Rader, Curr. Protoc. Protein Sci. 55:6.10.1-6.10.14. for the expression of Fab fragments.

The use of eukaryotic cells for the expression of the antibody or antigen-binding fragment of the invention is, however, preferred due to the glycosylation of the heterologously expressed antibody. It is even more preferred to use mammalian cells for the expression of the antibody or antigen-binding fragment of the invention. For example, Chinese hamster ovary (CHO) cells, or any of its genetically different progeny such as K1-, DukX B11-, DG44-cell lines may be used for the expression of the antibody of the invention. Other mammalian cell lines that may be used for the production of the antibody or antigen-binding fragment of the invention include e.g. NS0 cells, embryonic kidney (HEK) 293 cells, PER.C6 cells, MCF7 cells. Cells can e.g. be transfected with an expression vector comprising the coding sequence for the ati-GUCY2C antibody or antigen-binding fragment according to the invention. The expression vector or recombinant plasmid is produced by placing the coding antibody sequences under control of suitable regulatory genetic elements, including promoter and enhancer sequences like, e.g., a CMV promoter. Heavy and light chain sequences might can e.g. be expressed from individual expression vectors which are co-transfected, or from dual expression vectors. Said transfection may be a transient transfection or a stabile transfection. The transfected cells are subsequently cultivated to produce the transfected antibody construct. When stabile transfection is performed, then stable clones secreting antibodies with properly associated heavy and light chains are selected by screening with an appropriate assay, such as, e.g., ELISA, subcloned, and propagated for future production. Corresponding methods are e.g. disclosed in WO03/018771.

According to one embodiment, the anti-GUCY2C antibody of the ADC of the invention comprises an Fc region, which comprises the amino acid sequence according to SEQ ID NO: 38. Antibodies of the invention which comprise an Fc region comprising the amino acid sequence according SEQ ID NO: 38 are able to induce antibody-dependent cell-mediated cytotoxicity (ADCC), antibody-dependent cellular phagocytosis (ADCP), or complement-dependent cytotoxicity (CDC). It may, however, be desirable to reduce or eliminate effector function of the antibody of the ADC of the invention as disclosed herein for example to prevent target cell death, unwanted cytokine secretion, or killing of cells that express the Fcγ receptor such as macrophages.

Accordingly, the anti-GUCY2C antibody of the ADC of the invention as described herein may also include modifications and/or mutations that alter the properties of the antibodies and/or fragments, such as those which decrease ADCC, ADCP, or complement-dependent cytotoxicity CDC as known in the art. Preferably, ADCC, ADCP and CDC are reduced by at least 90% or more, more preferably by at least 95%, more preferably by at least 97.5%, even more preferred by at least 98%, 99% compared to an antibody comprising a wild type Fc region comprising e.g. the amino acid sequence according to SEQ ID NO: 38.

The binding of IgG1 to activating and inhibitory Fcγ receptors (FcγRs) or the first component of complement (C1q) depends on residues located in the hinge region and the CH2 domain. Two regions of the CH2 domain are critical for Fc□Rs and complement C1q binding, and have unique sequences. Substitution of human IgG1 and IgG2 residues at positions 233-236 and IgG4 residues at positions 327, 330 and 331 greatly reduced ADCC and CDC (Armour, et al., Eur. J. Immunol. 29(8) (1999) 2613-2624; Shields, et al., J. Biol. Chem. 276(9) (2001) 6591-6604, WO 2021/234402 A2).

Accordingly, in one embodiment, the anti-GUCY2C antibody of the ADC of the invention comprises a variant Fc region, wherein said variant Fc region comprises at least one amino acid modification relative to a wild-type Fc region, such that said molecule has a reduced affinity for IgG1 Fc receptors FcγRI, FcγRII and FcγRIII as well as to complement component C1q compared to a wild-type Fc region (e.g. comprising an amino acid sequence according to SEQ ID NO: 38).

Affinity to an Fc region, such as the binding of IgG1 to FcγRs, can be determined using a variety of techniques known in the art, for example but not limited to, equilibrium methods (e.g., enzyme-linked immunoabsorbent assay (ELISA); KinExA, Rathanaswami et al. Analytical Biochemistry, Vol. 373:52-60, 2008; or radioimmunoassay (RIA)), or by a surface plasmon resonance assay as disclosed in e.g. Wilkinson et al. PLoS One. 2021; 16(12): e0260954 or other mechanism of kinetics-based assay (e.g., BIACORETM. analysis or OctetTM analysis (forteBIO)), and other methods such as indirect binding assays, competitive binding assays fluorescence resonance energy transfer (FRET), gel electrophoresis and chromatography (e.g., gel filtration).

Thus, in some embodiments the anti-GUCY2C antibody of the ADC of the invention have been genetically engineered to comprise a variant Fc region which comprise modification of at least one amino acid residue that directly contacts FcγRs based on structural and crystallographic analysis. The term "genetically engineered" or "genetic engineering" as used herein relates to the modification of the amino acid sequence or part thereof of a given or natural polypeptide or protein, such as e.g. the Fc region of an antibody, in the sense of nucleotide and/or amino acid substitution, insertion, deletion or reversion, or any combinations thereof, by gene technological methods, such as, e.g., site-directed mutagenesis as described in Carter, Biochem. J. (1986) Vol. 237: 1-7, or J Biol Chem. (2015) Vol. 290(5): 2577-2592. As used herein, the term "amino acid substitution" or "mutation" relates to modifications of the amino acid sequence of the protein, wherein one or more amino acids are replaced with the same number of different amino acids, producing a protein which contains a different amino acid sequence than the original protein. A conservative amino acid substitution is understood to relate to a substitution which due to similar size, charge, polarity and/or conformation does not significantly affect the structure and function of the protein. Groups of conservative amino acids in that sense represent, e.g., the non-polar amino acids Gly, Ala, Val, Ile and Leu; the aromatic amino acids Phe, Trp and Tyr; the positively charged amino acids Lys, Arg and His; and the negatively charged amino acids Asp and Glu.

Importantly, Fc region of the antibody ADC of the invention is genetically engineered such as to comprise at least one cysteine amino acid substitution at a site within the Fc region such that this engineered cysteine is available for conjugation but does not perturb immunoglobulin folding and assembly. Corresponding cysteine-substituted or cysteine-engineered antibodies have disclosed in WO2016040856A2, or Junutula, et al., 2008b Nature Biotech., 26(8):925-932; Dornan et al (2009) Blood 114(13):2721-2729; US 7521541; US 7723485; WO2009/052249 and WO2016/142049). The preferred cysteine substitution in the Fc region of the inventive antibodies as disclosed herein is D265C (according to EU numbering system) as disclosed in WO2016142049A1.

Preferably, the antibody comprises a heavy chain constant (Fc) region which comprises the amino acid substitution D265C. More specifically, the mAb1 antibody comprises the heavy chain constant (Fc) region of SEQ ID NO: 39, the mAb8 antibody comprises the heavy chain constant (Fc) region of SEQ ID NO: 40, and the mAb41 antibody comprises the heavy chain constant (Fc) region of SEQ ID NO: 41.More preferably, said heavy chain constant (Fc) region comprises the amino acid substitutions L234A, L235A and D265C according to EU numbering system (Edelman et al., 1969, Proc Natl Acad Sci USA 63:78-85). The EU numbering system may also be referred to as "EU index as in Kabat" and refers to the numbering of the human IgG1 EU antibody, which refers to the numbering of the EU antibody of Edelman et al., 1969, Proc Natl Acad Sci USA 63:78-85. Specifically, in embodiments where all three substitutions L234A, L235A and D265C are present in the Fc region of the antibody of the ADC of the invention, mAb1 antibody comprises the heavy chain constant (Fc) region of SEQ ID NO: 42, mAb8 antibody comprises the heavy chain constant (Fc) region of SEQ ID NO: 43, and mAb41 antibody comprises the heavy chain constant (Fc) region of SEQ ID NO: 44. More preferably, the antibody comprises (a) an mAb1-L234A-L235A-D265C heavy chain of SEQ ID NO: 42; or (b) an mAb8-L234A-L235A-D265C heavy chain of SEQ ID NO: 43; or (c) an mAb41-L234A-L235A-D265C of SEQ ID NO: 44.The use of said Fc regions comprising the substitutions L234A, L235A and D265C in the anti-GUCY2C antibodies of the ADC of the first aspect is particularly advantageous to reduce the interaction of the respective Fc regions (i.e. those comprising said mutations) with FcγRs by at least 95%, 97.5%, 99% compared to a wild-type Fc region while at the same time providing the attachment site for the drug-carrying linker to the antibody of the ADC via site-specific thiol-based conjugation. Corresponding use of such cysteine substituted Fc regions and antibodies comprising such mutations for linker-payload conjugation is, e.g., disclosed in WO2016142049A1.

In one specific embodiment of the first aspect, the antibody-drug conjugate is: 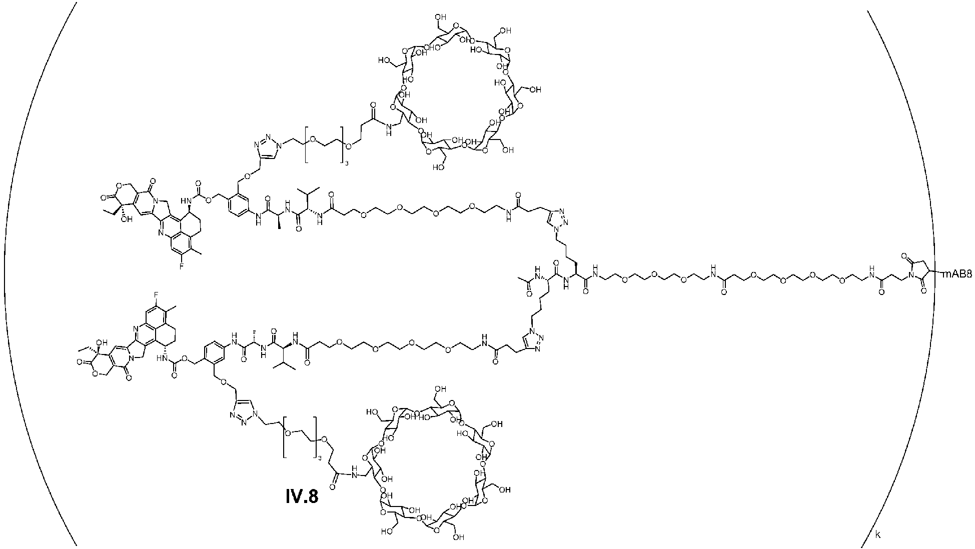 wherein k is 2.

In another specific embodiment of the first aspect, the antibody-drug conjugate is: 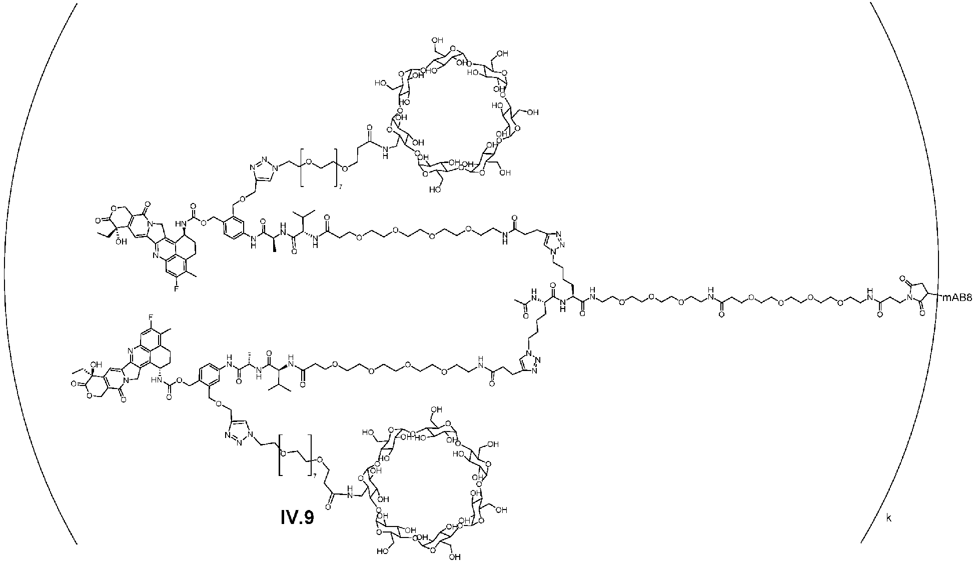 wherein k is 2.

In another specific embodiment of the first aspect, the antibody-drug conjugate is: 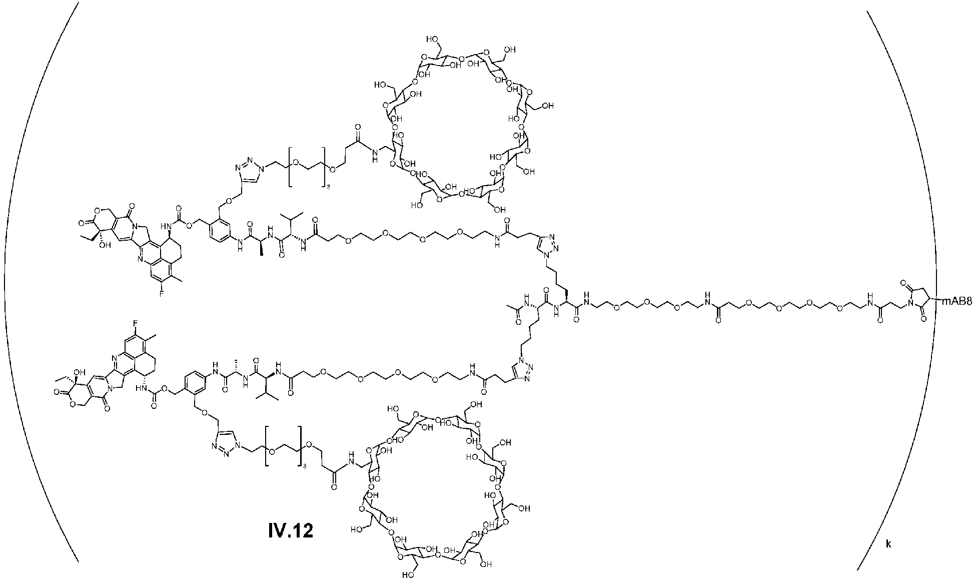 wherein k is 2.

In another specific embodiment of the first aspect, the antibody-drug conjugate is: 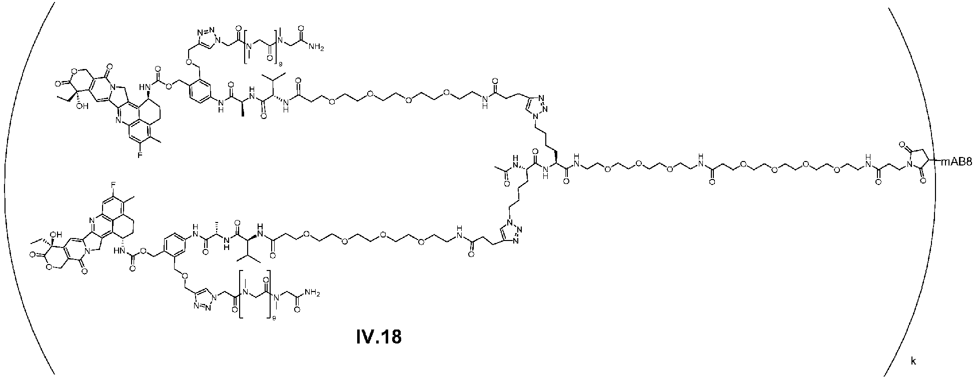 wherein k is 2.

In another specific embodiment of the first aspect, the antibody-drug conjugate is: 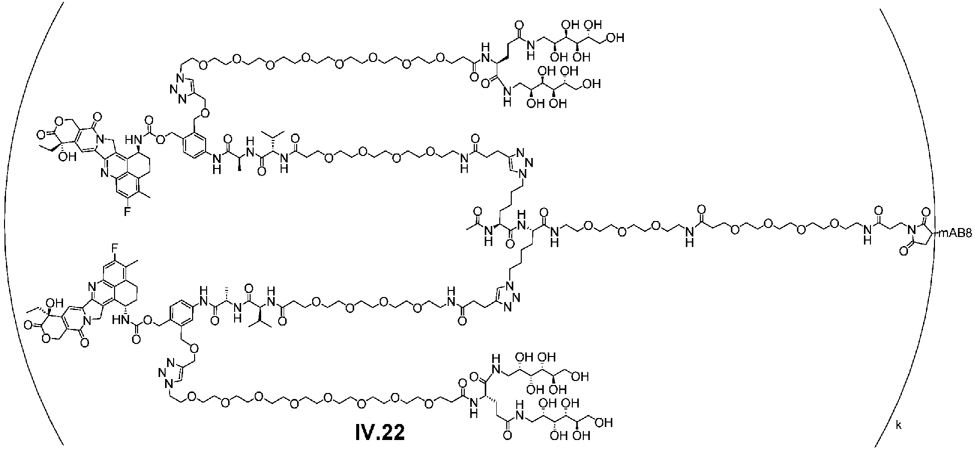 wherein k is 2.

As indicated above, in a second aspect, the present invention further relates to the ADC of the first aspect for use as a medicament.

As described above, the ADC is particularly suited for use in the treatment of cancer, more preferably for the treatment of gastrointestinal cancer, such as colorectal cancer (CRC), metastatic colorectal cancer (mCRC) or pancreatic cancer, where the gastrointestinal cancer can be characterised by the expression of a target sequence or epitope of a Guanylate Cyclase 2C (GUCY2C) antigen.

Furthermore, in a third aspect, the present invention relates to a pharmaceutical composition comprising the antibody-drug conjugate of the first aspect.

In particular embodiments, the pharmaceutical composition is used in the form of a systemically administered medicament. This includes parenterals, which comprise among others injectables and infusions. Injectables are formulated either in the form of ampoules or as so called ready-for-use injectables, e.g. ready-to-use syringes or single-use syringes and aside from this in puncturable flasks for multiple withdrawal. The administration of injectables can be in the form of subcutaneous (s.c), intramuscular (i.m.), intravenous (i.v.) or intracutaneous (i.e.) application. In particular, it is possible to produce the respectively suitable injection formulations as
a suspension of crystals, solutions, nanoparticular or a colloid dispersed systems like, e.g. hydrosols.

Injectable formulations can further be produced as concentrates, which can be dissolved or dispersed with aqueous isotonic diluents. The infusion can also be prepared in form of isotonic solutions, fatty emulsions, liposomal formulations and micro-emulsions. Similar to injectables, infusion formulations can also be prepared in the form of concentrates for dilution. Injectable formulations can also be applied in the form of permanent infusions both in in-patient and ambulant therapy, e.g. by way of mini-pumps.

It is possible to add to parenteral drug formulations, for example, albumin, plasma, expander, surface-active substances, organic diluents, pH-influencing substances, complexing substances or polymeric substances, in particular as substances to influence the adsorption of the TBDCs of the invention to proteins or polymers or they can also be added with the aim to reduce the adsorption of the TBDCs of the invention to materials like injection instruments or packaging-materials, for example, plastic or glass.

Adjuvants and carriers added during the production of the pharmaceutical compositions of the present invention formulated as parenterals are preferably aqua sterilisata (sterilized water), pH value influencing substances like, e.g. organic or inorganic acids or bases as well as salts thereof, buffering substances for adjusting pH values, substances for isotonization like e.g. sodium chloride, sodium hydrogen carbonate, glucose and fructose, tensides and surfactants, respectively, and emulsifiers like, e.g. partial esters of fatty acids of polyoxyethylene sorbitans (for example, Tween^{®}) or, e.g. fatty acid esters of polyoxyethylenes (for example, Cremophor^{®}), fatty oils like, e.g. peanut oil, soybean oil or castor oil, synthetic esters of fatty acids like, e.g. ethyl oleate, isopropyl myristate and neutral oil (for example, Miglyol^{®}) as well as polymeric adjuvants like, e.g. gelatine, dextran, polyvinylpyrrolidone, additives which increase the solubility of organic solvents like, e.g. propylene glycol, ethanol, N,N-dimethylacetamide, propylene glycol or complex forming substances like, e.g. citrate and urea, preservatives like, e.g. benzoic acid hydroxypropyl ester and methyl ester, benzyl alcohol, antioxidants like e.g. sodium sulfite and stabilizers like e.g. EDTA.

When formulating the pharmaceutical compositions of the present invention as suspensions in a preferred embodiment thickening agents to prevent the setting of the TBDCs of the invention or, tensides and polyelectrolytes to assure the resuspendability of sediments and/or complex forming agents like, for example, EDTA are added. It is also possible to achieve complexes of the active ingredient with various polymers. Examples of such polymers are polyethylene glycol, polystyrene, carboxymethyl cellulose, Pluronics^{®}or polyethylene glycol sorbitol fatty acid ester. The TBDCs of the invention can also be incorporated in liquid formulations in the form of inclusion compounds e.g. with cyclodextrins. In particular embodiments dispersing agents can be added as further adjuvants. For the production of lyophilisates scaffolding agents like mannite, dextran, saccharose, human albumin, lactose, PVP or varieties of gelatine can be used.

In light of the above, it will be appreciated that the second and third aspects of the present invention also encompass methods of treating cancer, preferably of treating gastrointestinal cancer, such as colorectal cancer (CRC), metastatic colorectal cancer (mCRC) or pancreatic cancer, wherein the method comprises administering a therapeutically effective amount of the antibody-drug conjugate of the first aspect or of the pharmaceutical composition of the third aspect to a patient in need thereof. Similarly, at the second aspect of the present invention also encompasses use of the antibody-drug conjugate of the first aspect in the manufacture of a medicament for the treatment of cancer, preferably for the treatment of gastrointestinal cancer, such as colorectal cancer (CRC), metastatic colorectal cancer (mCRC) or pancreatic cancer.

Again, in such embodiments, the gastrointestinal cancer is typically characterised by the expression of a target sequence or epitope of a Guanylate Cyclase 2C (GUCY2C) antigen.

### Sequences

**Table 1: Amino acid sequences of the invention**

| **SEQ ID NO** | **Sequence** | **Description** |
|---|---|---|
| 1 | RASQSISSYLN | mAb1 CDRL1 |
| 2 | AASSLQS | mAb1 CDRL2 |
| 3 | QQSYSTPWT | mAb1 CDRL3 |
| 4 | SSAM H | mAb1 CDRH1 |
| 5 | LINPGNGNTKYSQKFQG | mAb1 CDRH2 |
| 6 | AYFRGLGFDI | mAb1 CDRH3 |
| 7 | QQSYSTPLT | mAb8 CDRL3 |
| 8 | TGTSSDVGSYNLVS | mAb41 CDRL1 |
| 9 | EGSKRPS | mAb41 CDRL2 |
| 10 | SYVPRSSLV | mAb41 CDRL3 |
| 11 | TYTIN | mAb41 CDRH1 |
| 12 | RIIPVLGIANYAQKFQG | mAb41 CDRH2 |
| 13 | DTPRLRSSYYM DV | mAb41 CDRH3 |
| 14 | DIQMTQSPSSLSASAGDRVTITC | mAb1 FRL1 |
| 15 | WYQQKPGKAPKLLIY | mAb1 FRL2 |
| 16 | GVPSRFSGSGSGTDFTLAISSLQPEDFATYYC | mAb1 FRL3 |
| 17 | FGQGTKVDIK | mAb1 FRL4 |
| 18 | QVQLVQSGAEVKKPGASVKVSCKASGYIFI | mAb1 FRH1 |
| 19 | WVRQAPGQRLEWMG | mAb1 FRH2 |
| 20 | RVTITRDTSASTAYMELSSLRSEDTAVYYCAR | mAb1 FRH3 |
| 21 | WGQGTLVTVSS | mAb1 FRH4 |
| 22 | DIRMTQSPSSLSASAGDRVTITC | mAb8 FRL1 |
| 23 | GVPSRFSGSGSGTDFTLTISSLQPEDFATYYC | mAb8 FRL3 |
| 24 | FGGGTKLEIK | mAb8 FRL4 |
| 25 | QSALTQPASVSGSPGQSITISC | mAb41 FRL1 |
| 26 | WYQQHPGKAPKLMIY | mAb41 FRL2 |
| 27 | GVSNRFSASKSGNTASLTISGLQTEDEADYYC | mAb41 FRL3 |
| 28 | FGGGTKLTVL | mAb41 FRL4 |
| 29 | QVQLVQSGAEVKKPGSSVKVSCKASGGTFS | mAb41 FRH1 |
| 30 | WVRQAPGQGLEWMG | mAb41 FRH2 |
| 31 | RVTITADKSTSTAYMELSSLRSEDTAVYYCAR | mAb41 FRH3 |
| 32 | DIQMTQSPSSLSASAGDRVTITCRASQSISSYL NWYQQKPGKAPKLLIYAASSLQSGVPSRFSGS GSGTD FTLAISSLQP EDFATYYCQQ SYSTPWTFGQ GTKVDIK | mAb1 VL |
| 33 | QVQLVQSGAEVKKPGASVKVSCKASGYIFISS AMHWVRQAPGQRLEWMGLINPGNGNTKYSQ KFQGRVTITRDTSASTAYMELSSLRSEDTAVYY CARAYFRGLGFDIWGQGTLVTVSS | mAb1 VH |
| 34 | DIRMTQSPSSLSASAGDRVTITCRASQSISSYL NWYQQKPGKAPKLLIYAASSLQSGVPSRFSGS GSGTDFTLTISSLQPEDFATYYCQQSYSTPLTF GGGTKLEIK | mAb8 VL |
| 35 | QVQLVQSGAEVKKPGASVKVSCKASGYIFISS AMHWVRQAPGQRLEWMGLINPGNGNTKYSQ KFQGRVTITRDTSASTAYMELSSLRSEDTAVYY CARAYFRGLGFDIWGQGTLVTVSS | mAb8 VH |
| 36 | QSALTQPASVSGSPGQSITISCTGTSSDVGSYN LVSWYQQHPGKAPKLMIYEGSKRPSGVSNRF SASKSGNTASLTISGLQTEDEADYYCSSYVPRS SLVFGGGTKLTVL | m41 VL |
| 37 | QVQLVQSGAEVKKPGSSVKVSCKASGGTFST YTINWVRQAPGQGLEWMGRIIPVLGIANYAQK FQGRVTITADKSTSTAYMELSSLRSEDTAVYYC ARDTPRLRSSYYMDVWGQGTLVTVSS | mAb41 VH |
| 38 | | HC (wild type) |
| 39 | QVQLVQSGAEVKKPGASVKVSCKASGYIFISS AMHWVRQAPGQRLEWMGLINPGNGNTKYSQ KFQGRVTITRDTSASTAYMELSSLRSEDTAVYY CARAYFRGLGFDIWGQGTLVTVSSASTKGPSV FPLAPSSKSTSGGTAALGCLVKDYFPEPVTVS WNSGALTSGVHTFPAVLQSSGLYSLSSVVTVP SSSLGTQTYICNVNHKPSNTKVDKKVEPKSCD | mAb1 heavy chain (HC) comprising the mutation D265C (according to EU numbering system) |
| | KTHTCPPCPAPELLGGPSVFLFPPKPKDTLMIS RTPEVTCVVVCVSHEDPEVKFNWYVDGVEVH NAKTKPREEQYNSTYRVVSVLTVLHQDWLNG KEYKCKVSNKALPAPIEKTISKAKGQPREPQVY TLPPSREEMTKNQVSLTCLVKGFYPSDIAVEW ESNGQPENNYKTTPPVLDSDGSFFLYSKLTVD KSRWQQGNVFSCSVMHEALHNHYTQKSLSLS PGK | |
| 40 | QVQLVQSGAEVKKPGASVKVSCKASGYIFISS AMHWVRQAPGQRLEWMGLINPGNGNTKYSQ KFQGRVTITRDTSASTAYMELSSLRSEDTAVYY CARAYFRGLGFDIWGQGTLVTVSSASTKGPSV FPLAPSSKSTSGGTAALGCLVKDYFPEPVTVS WNSGALTSGVHTFPAVLQSSGLYSLSSVVTVP SSSLGTQTYICNVNHKPSNTKVDKKVEPKSCD KTHTCPPCPAPELLGGPSVFLFPPKPKDTLMIS RTPEVTCVVVCVSHEDPEVKFNWYVDGVEVH NAKTKPREEQYNSTYRVVSVLTVLHQDWLNG KEYKCKVSNKALPAPIEKTISKAKGQPREPQVY TLPPSREEMTKNQVSLTCLVKGFYPSDIAVEW ESNGQPENNYKTTPPVLDSDGSFFLYSKLTVD KSRWQQGNVFSCSVMHEALHNHYTQKSLSLS PGK | mAb8 HC comprising the mutation D265C (according to EU numbering system) |
| 41 | QVQLVQSGAEVKKPGSSVKVSCKASGGTFST YTINWVRQAPGQGLEWMGRIIPVLGIANYAQK FQGRVTITADKSTSTAYMELSSLRSEDTAVYYC ARDTPRLRSSYYMDVWGQGTLVTVSSASTKG PSVFPLAPSSKSTSGGTAALGCLVKDYFPEPVT VSWNSGALTSGVHTFPAVLQSSGLYSLSSVVT VPSSSLGTQTYICNVNHKPSNTKVDKKVEPKS CDKTHTCPPCPAPELLGGPSVFLFPPKPKDTL MISRTPEVTCVVVCVSHEDPEVKFNWYVDGV EVHNAKTKPREEQYNSTYRVVSVLTVLHQDWL NGKEYKCKVSNKALPAPIEKTISKAKGQPREPQ VYTLPPSREEMTKNQVSLTCLVKGFYPSDIAVE WESNGQPENNYKTTPPVLDSDGSFFLYSKLTV DKSRWQQGNVFSCSVMHEALHNHYTQKSLSL SPGK | mAb41 HC comprising the mutation D265C (numbering according to EU numbering system) |
| 42 | QVQLVQSGAEVKKPGASVKVSCKASGYIFISS AMHWVRQAPGQRLEWMGLINPGNGNTKYSQ KFQGRVTITRDTSASTAYMELSSLRSEDTAVYY CARAYFRGLGFDIWGQGTLVTVSSASTKGPSV FPLAPSSKSTSGGTAALGCLVKDYFPEPVTVS WNSGALTSGVHTFPAVLQSSGLYSLSSVVTVP SSSLGTQTYICNVNHKPSNTKVDKKVEPKSCD KTHTCPPCPAPEAAGGPSVFLFPPKPKDTLMIS RTPEVTCVVVCVSHEDPEVKFNWYVDGVEVH NAKTKPREEQYNSTYRVVSVLTVLHQDWLNG KEYKCKVSNKALPAPIEKTISKAKGQPREPQVY TLPPSREEMTKNQVSLTCLVKGFYPSDIAVEW | mAb1 HC comprising the mutations L234A, L235A and D265C (numbering according to EU numbering system) |
| | ESNGQPENNYKTTPPVLDSDGSFFLYSKLTVD KSRWQQGNVFSCSVMHEALHNHYTQKSLSLS PGK | |
| 43 | QVQLVQSGAEVKKPGASVKVSCKASGYIFISS AMHWVRQAPGQRLEWMGLINPGNGNTKYSQ KFQGRVTITRDTSASTAYMELSSLRSEDTAVYY CARAYFRGLGFDIWGQGTLVTVSSASTKGPSV FPLAPSSKSTSGGTAALGCLVKDYFPEPVTVS WNSGALTSGVHTFPAVLQSSGLYSLSSVVTV PSSSLGTQTYICNVNHKPSNTKVDKKVEPKSC DKTHTCPPCPAPEAAGGPSVFLFPPKPKDTLM ISRTPEVTCVVVCVSHEDPEVKFNWYVDGVEV HNAKTKPREEQYNSTYRVVSVLTVLHQDWLN GKEYKCKVSNKALPAPIEKTISKAKGQPREPQV YTLPPSREEMTKNQVSLTCLVKGFYPSDIAVE WESNGQPENNYKTTPPVLDSDGSFFLYSKLTV DKSRWQQGNVFSCSVMHEALHNHYTQKSLSL SPGK | mAb8 HC comprising the mutations L234A, L235A and D265C (numbering according to EU numbering system) |
| 44 | QVQLVQSGAEVKKPGSSVKVSCKASGGTFST YTINWVRQAPGQGLEWMGRIIPVLGIANYAQK FQGRVTITADKSTSTAYMELSSLRSEDTAVYYC ARDTPRLRSSYYMDVWGQGTLVTVSSASTKG PSVFPLAPSSKSTSGGTAALGCLVKDYFPEPVT VSWNSGALTSGVHTFPAVLQSSGLYSLSSVVT VPSSSLGTQTYICNVNHKPSNTKVDKKVEPKS CDKTHTCPPCPAPEAAGGPSVFLFPPKPKDTL MISRTPEVTCVVVCVSHEDPEVKFNWYVDGV EVHNAKTKPREEQYNSTYRVVSVLTVLHQDWL NGKEYKCKVSNKALPAPIEKTISKAKGQPREPQ VYTLPPSREEMTKNQVSLTCLVKGFYPSDIAVE WESNGQPENNYKTTPPVLDSDGSFFLYSKLTV DKSRWQQGNVFSCSVMHEALHNHYTQKSLSL SPGKMABLALADC | mAb41 HC comprising the mutations L234A, L235A and D265C (numbering according to EU numbering system) |

### EXAMPLES

The invention is further described by the following non-limiting Examples.

### General information

While the invention has been illustrated and described in detail in the drawings and foregoing description, such illustration and description are to be considered illustrative or exemplary and not restrictive; the invention is not limited to the disclosed embodiments.

All amino acid sequences disclosed herein are shown from N-terminus to C-terminus; all nucleic acid sequences disclosed herein are shown 5'->3'.

Chemicals and solvents were obtained from commercial suppliers such ABCR, Fisher Scientific, Merck Chemicals, Carl Roth, Sigma-Aldrich, VWR International, Biozol Diagnostika, TCI Deutschland, IRIS Biotech, AnalytiChem, Hycultec, Hölzel Diagnostika, Bachem, Thermo Fisher, Activate Scientific, AxisPharm, Biosynth or BLD Pharmatech and were used without further purification. The water used for reactions, workups and purifications was purified by a ELGA Purelab flex 2 pure water system. TLC was performed with silica gel 60-coated polyester sheets (Macherey-Nagel) and spots visualised by irradiation (λ = 254 nm), or ninhydrin, molybdate or KMNO₄ staining solutions.

### General experimental methods

### Analytical HPLC

Analytical RP-HPLC was carried out on a VWR-Hitachi Chromaster system equipped with a diode array detector 5430, autosampler 5260, quaternary pump 5160, column oven 6310 and a Luna^{®} C18 (2) 5 µm 100 Å 250 × 4.6 mm, Kinetex^{®} EVO-C18 5 µm 100 Å 250 × 4.6 mm and ACE C18-PFP 5 µm 100 Å 250 × 4.6 mm at 25 °C with a flowrate between 1 - 1.4 ml/min using linear gradient elution of acetonitrile in water. The specified parameters are stated below.

### Method A

| | | | |
|---|---|---|---|
| Column | Phenomenex Luna C18(2), 100 Å, 250 × 4.6 mm, 5 or 10 µm | | |
| Mobile Phase | A: 0.05 % TFA in water | | |
| | B: Acetonitrile | | |

| Gradient | Time [min] | A [%] | B [%] |
|---|---|---|---|
| | 0.0 | 95 | 5 |
| | 15.0 | 0 | 100 |
| | 18.0 | 0 | 100 |
| | 18.5 | 95 | 5 |
| | 22.0 | 95 | 5 |
| Wavelengths | 210, 260 nm | | |
| Flow Rate | 1.4 mL/min | | |
| Column Temperature | 22 °C | | |
| Run Time | 22 min | | |

### Method B

| | | | |
|---|---|---|---|
| Column | Phenomenex Luna C18(2), 100 Å, 250 × 4.6 mm, 5 or 10 µm | | |
| Mobile Phase | A: 0.05 % TFA in water | | |
| | B: Acetonitrile | | |

| Gradient | Time [min] | A [%] | B [%] |
|---|---|---|---|
| | 0.0 | 95 | 5 |
| | 0.5 | 70 | 30 |
| | 15.5 | 50 | 50 |
| | 16.0 | 0 | 100 |
| | 19.0 | 0 | 100 |
| | 19.5 | 95 | 5 |
| | 22.0 | 95 | 5 |
| Wavelengths | 210, 260 nm | | |
| Flow Rate | 1.4 mL/min | | |
| Column Temperature | 22 °C | | |
| Run Time | 22 min | | |

### Method C

| | | | |
|---|---|---|---|
| Column | Phenomenex Kinetex EVO-18, 100 Å, 250 × 4.6 mm, 5 µm | | |
| Mobile Phase | A: 0.05 % TFA in water | | |
| | B: Acetonitrile | | |

| Gradient | Time [min] | A [%] | B [%] |
|---|---|---|---|
| | 0.0 | 95 | 5 |
| | 15.0 | 0 | 100 |
| | 18.0 | 0 | 100 |
| | 18.5 | 95 | 5 |
| | 22.0 | 95 | 5 |
| Wavelengths | 246, 210 nm | | |
| Flow Rate | 1.4 mL/min | | |
| Column Temperature | 25 °C | | |
| Run Time | 22 min | | |

### Method D

| | | | |
|---|---|---|---|
| Column | Phenomenex Kinetex EVO-18, 100 Å, 250 x 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 15.0 | 0 | 100 |
| | 18.0 | 0 | 100 |
| | 18.5 | 95 | 5 |
| | 22.0 | 95 | 5 |
| Wavelengths | 210, 260 nm | | |
| Flow Rate | 1.4 mL/min | | |
| Column Temperature | 25 °C | | |
| Run Time | 22 min | | |

### Method E

| | | | |
|---|---|---|---|
| Column | Avantor ACE C18-PFP, 100 Å, 250 x 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 2.0 | 70 | 30 |
| | 26.0 | 40 | 60 |
| | 26.5 | 0 | 100 |
| | 28.0 | 0 | 100 |
| | 28.5 | 95 | 5 |
| | 30.0 | 95 | 5 |
| Wavelengths | 210, 380 nm | | |
| Flow Rate | 1 mL/min | | |
| Column Temperature | 25 °C | | |
| Run Time | 30 min | | |

### Method F

| | | | |
|---|---|---|---|
| Column | Phenomenex Kinetex EVO-18, 100 Å, 250 x 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 2.0 | 70 | 30 |
| | 26.0 | 40 | 60 |
| | 26.5 | 0 | 100 |
| | 28.0 | 0 | 100 |
| | 28.5 | 95 | 5 |
| | 30.0 | 95 | 5 |
| Wavelengths | 210, 380 nm | | |
| Flow Rate | 1.4 mL/min | | |
| Column Temperature | 25 °C | | |
| Run Time | 30 min | | |

### Method G

| | | | |
|---|---|---|---|
| Column | Phenomenex Kinetex EVO-18, 100 Å, 250 x 4.6 mm, 5 µm | | |
| Mobile Phase | A: Water | | |
| | B: Acetonitrile | | |
| Gradient | Time [min] | A [%] | B [%] |
| | 0.0 | 95 | 5 |
| | 14.8 | 50 | 50 |
| | 15.0 | 0 | 100 |
| | 18.0 | 0 | 100 |
| | 18.5 | 95 | 5 |
| | 22.0 | 95 | 5 |
| Wavelengths | 190, 210 nm | | |
| Flow Rate | 1.4 mL/min | | |
| Column Temperature | 25 °C | | |
| Run Time | 22 min | | |

### Method H

| | | | |
|---|---|---|---|
| Column | Phenomenex Luna C18(2), 100 Å, 250 x 4.6 mm, 5 or 10 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 14.8 | 50 | 50 |
| | 15.0 | 0 | 100 |
| | 18.0 | 0 | 100 |
| | 18.5 | 95 | 5 |
| | 22.00 | 95 | 5 |
| Wavelengths | 210, 246 nm | | |
| Flow Rate | 1.4 mL/min | | |
| Column Temperature | 25 °C | | |
| Run Time | 22 min | | |

### Method I

| | | | |
|---|---|---|---|
| Column | Phenomenex Luna C18(2), 100 Å, 250 x 4.6 mm, 5 or 10 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 15.0 | 0 | 100 |
| | 18.0 | 0 | 100 |
| | 18.5 | 95 | 5 |
| | 22.0 | 95 | 5 |
| Wavelengths | 210, 305 nm | | |
| Flow Rate | 1.4 mL/min | | |
| Column Temperature | 25 °C | | |
| Run Time | 22 min | | |

### Method J

| | | | |
|---|---|---|---|
| Column | Phenomenex Kinetex EVO-18, 100 Å, 250 x 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 26.0 | 15 | 85 |
| | 26.5 | 0 | 100 |
| | 28.0 | 0 | 100 |
| | 28.5 | 95 | 5 |
| | 30 | 95 | 5 |
| Wavelengths | 210, 380 nm | | |
| Flow Rate | 1.4 mL/min | | |
| Column Temperature | 25 °C | | |
| Run Time | 30 min | | |

### Method K

| | | | |
|---|---|---|---|
| Column | Phenomenex Kinetex EVO-18, 100 Å, 250 x 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 14.8 | 70 | 30 |
| | 15.0 | 0 | 100 |
| | 18.0 | 0 | 100 |
| | 18.5 | 95 | 5 |
| | 22.0 | 95 | 5 |
| Wavelengths | 210, 230 nm | | |
| Flow Rate | 1.4 mL/min | | |
| Column Temperature | 25 °C | | |
| Run Time | 22 min | | |

### Method L

| | | | |
|---|---|---|---|
| Column | Phenomenex Kinetex EVO-18, 100 Å, 250 × 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 25.0 | 40 | 60 |
| | 25.2 | 0 | 100 |
| | 27.8 | 0 | 100 |
| | 28.0 | 95 | 5 |
| | 30.0 | 95 | 5 |
| Wavelengths | 210, 260 nm | | |
| Flow Rate | 1.4 mL/min | | |
| Column Temperature | 25 °C | | |
| Run Time | 30 min | | |

### Method M

| | | | |
|---|---|---|---|
| Column | Phenomenex Luna C18(2), 100 Å, 250 × 4.6 mm, 5 or 10 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 15.0 | 80 | 20 |
| | 15.2 | 0 | 100 |
| | 18.0 | 0 | 100 |
| | 18.5 | 95 | 5 |
| | 22.0 | 95 | 5 |
| Wavelengths | 205, 220 nm | | |
| Flow Rate | 1.4 mL/min | | |
| Column Temperature | 25 °C | | |
| Run Time | 22 min | | |

### Method N

| | | | |
|---|---|---|---|
| Column | Phenomenex Luna C18(2), 100 Å, 250 × 4.6 mm, 5 or 10 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 0.5 | 80 | 20 |
| | 15.5 | 60 | 40 |
| | 16.0 | 0 | 100 |
| | 19.0 | 0 | 100 |
| | 19.5 | 95 | 5 |
| | 22.0 | 95 | 5 |
| Wavelengths | 210, 260 nm | | |
| Flow Rate | 1.4 mL/min | | |
| Column Temperature | 22 °C | | |
| Run Time | 22 min | | |

### Method O

| | | | |
|---|---|---|---|
| Column | Phenomenex Kinetex EVO-18, 100 Å, 250 × 4.6 mm, 5 µm | | |
| Mobile Phase | A: 0.05 % TFA in water | | |
| | B: Acetonitrile | | |
| Gradient | Time [min] | A [%] | B [%] |
| | 0.0 | 95 | 5 |
| | 0.5 | 80 | 20 |
| | 15.5 | 60 | 40 |
| | 16.0 | 0 | 100 |
| | 19.0 | 0 | 100 |
| | 19.5 | 95 | 5 |
| | 22.0 | 95 | 5 |
| Wavelengths | 210, 260 nm | | |
| Flow Rate | 1.4 mL/min | | |
| Column Temperature | 22 °C | | |
| Run Time | 22 min | | |

### NMR spectroscopy

¹H and spectra were recorded at 500 MHz at room temperature and were referenced to trimethylsilane (TMS). Chemical shifts (δ) are reported in parts per million (ppm) from low field to high field. Coupling constants (*J*) are reported in Hertz (Hz) and abbreviations indicating multiplicity are used as follows: s = singlet, d = doublet, t = triplet, dd = doublet of doublet, m = multiplet.

### Mass spectrometry (MS)

MS measurements were carried out on an Advion expression^{®} CMS. Samples were submitted by direct injection with a syringe. For ionization, an electrospray ionization source (ESI) or atmospheric pressure ionization source (APCI) was used. Analyses were performed in positive ion mode. For ESI, the capillary temperature was set at 250 °C. The source gas temperatures were set at 250 and 300 °C, respectively with a flow of 4 and 5 L/min, respectively. Nebulizing gas flow was set to 0.5 L/min. The electrospray voltage was set at 3.5 kV. For APCI, the capillary temperature was set at 250 °C, source gas temperature at 350 °C and APCI corona discharge at 5 µA was used.

### Preparative RP-HPLC purification

Preparative RP-HPLC purification was carried out on an Agilent 1260 Infinity II system equipped with a binary pump, multisampler, VWD, fraction collector and column organizer. As columns, Phenomenx Luna^{®} C18 (2) 10 µm 100 Å 250 × 21.2 mm, Kinetex^{®} EVO-C18 5 µm 100 Å 250 × 21.2 mm and ACE C18-PFP 5 µm 100 Å 250 × 21.2 mm with flow rates between 24 - 30 ml/min using linear gradient elution of acetonitrile in water (+0.05 % TFA).

### Flash chromatography

Purification by flash chromatography on silica was performed using a Teledyne ISCO CombiFlash NextGen 300+ equipped with UV and ELSD detection and RediSep^{®} single-use normal-phase silica columns with mesh between 230 and 400.

### General Procedure 1 (GP1): CuAAC click reaction for attachment of solubility enhancer

To a stirred solution of azide (0.9-1.5 equiv.) and alkyne (1 equiv.) in DMF was added DMF:water (5:1 or 10:1) containing tris[(1-benzyl-1H-1,2,3-triazol-4-yl)methyl]amin) (TBTA, 0.125-0.625 equiv.) or tris(3-hydroxypropyltriazolylmethyl)amine (THPTA, 0.125-2 equiv.), copper(II) sulfate pentahydrate (0.1-1 equiv.) and Na-ascorbate (0.25-3.0 equiv.). The resulting reaction mixture was allowed to stir at room temperature until completion. Upon completion, the reaction mixture was purified directly without any pretreatment by prep HPLC to yield the product after lyophilization.

### General Procedure 2 (GP2): CuAAC click reaction on polyazide scaffold

To a stirred solution of polyazide (1 equiv.) and alkyne (1.05-1.25 equiv. per azide moiety) in DMF was added water containing Tris(3-hydroxypropyltriazolylmethyl)amine (THPTA, 0.63-1.35 equiv. per azide moiety), copper(II) sulfate pentahydrate (0.525-0.65 equiv. per azide moiety) and Na-ascorbate (1-2.15 equiv. per azide moiety). The resulting ratio of DMF/water was 5:1 or 10:1. The resulting reaction mixture was allowed to stir at room temperature until completion. Upon completion, the reaction mixture was purified directly without any pretreatment by prep HPLC to yield the multimeric linker-payload after lyophilization.

### General Procedure 3 (GP3): Attachment of intermediate 3.1 or 3.2

Fmoc-protected linker-payload was treated with diethylamine (Et₂N, 20-24 equiv.) in DMF at room temperature and the reaction progress followed by RP-HPLC. The solvent was removed *in vacuo* and the residue co-evaporated once with DMF. Then, the residue was dissolved in DMF under argon and a solution of **intermediate 3.1 or 3.2** (1.1-1.5 equiv.) in DMF was added followed by *N*-ethyldiisopropylamine (DIPEA, 1.4-3 equiv.). The solution was stirred at room temperature and the progress followed by RP-HPLC. Upon completion, the solvent was evaporated, and the crude product purified by RP-HPLC.

### General Procedure 4 (GP4): Attachment of maleimide

To a stirred solution of multimeric linker-payload in dry DMF was added Mal-PEG(4)-NHS ester (1-4 equiv.) and DIPEA (1-4 equiv.). The reaction mixture was stirred at room temperature for 30 min. Upon completion, the reaction mixture was purified directly without any pretreatment by prep HPLC to yield the final compound after lyophilization.

### General Procedure 5 (GP5): Attachment of PEG-azide to Mono-6-amino-6-deoxycyclodextrin

Azido-PEG-NHS ester (1 equiv.) was dissolved in water or DMF and a solution of mono-6-amino-6-deoxy-cyclodextrin (1 equiv.) in water or DMF was added. In case DMF was used as solvent, DIPEA (1.5-2 equiv.) was added if necessary. The resulting reaction mixture was allowed to stir at room temperature until completion and monitored by RP-HPLC. If necessary, another portion of mono-6-amino-6-deoxycyclodextrin (0.2 equiv.) was added. Upon completion, the reaction mixture was freeze dried and used as crude without further purification in case water was used as solvent. In case DMF was used as solvent, the product was isolated by precipitation from MTBE. In case of mono-6-amino-6-deoxy-beta-cyclodextrin use, commercially available mono-6-amino-6-deoxy-beta-cyclodextrin was employed. In case of mono-6-amino-6-deoxy-gamma-cyclodextrin use, the material was synthesized from gamma-cyclodextrin according to Tang, W., Ng, S.-C. Nat. Protoc. 2008, 3, 691-697.

### ADC generation - conjugation of linker payloads to antibodies

Antibodies were conjugated to the drug-carrying linker conjugates by means of the so-called Thiomab technology. In this approach, the conjugation takes place by coupling a terminal thiol-reactive moiety such as a maleimide residue of the drug-carrying linker to the free SH group of a genetically-engineered cysteine residue in the Fc region of the antibody, as shown in the following reaction scheme:

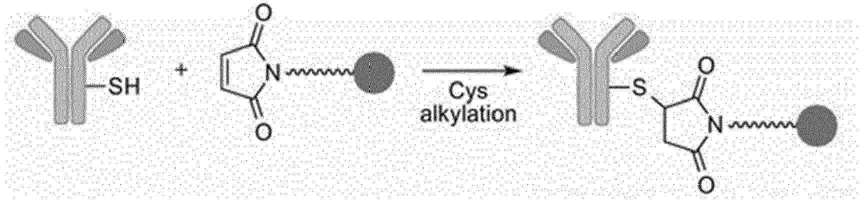

The principles of this conjugation method are disclosed in Junutula *et al.* (2008), the content of which is incorporated herein by reference.

The anti-GUCY2C antibodies used in the present experiments comprise a D265C substitution in both Fc domains, in order to provide a cysteine residue that has a free SH group. The respective technology is disclosed in WO2016/142049 A1, the content of which is incorporated herein by reference.

### EXAMPLE 1 - Synthesis of modular linker compounds

### Example 1.1- Syntheses of Linker Intermediates

### Step 1: Synthesis of 4-nitro-2-((prop-2-yn-1-yloxy)methyl)benzoic acid

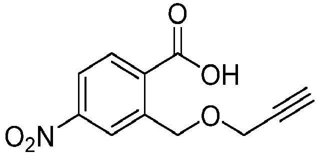

To a solution of Methyl-2-(bromomethyl)-4-nitrobenzoate (CAS-Nr. 133446-99-8, 2 g, 7.30 mmol, 1 equiv.) in 10 ml acetonitrile were added prop-2-yn-ol (2.16 ml, 36.5 mmol, 5 equiv.) and Cs₂CO₃ (5.96 g, 18.3 mmol, 2.5 equiv.) at room temperature. The mixture was stirred at 80 °C for 3 h and the progress monitored by RP-HPLC. The reaction mixture was filtered. The filtrate was diluted with ethyl acetate (20 ml) and washed with water (10 ml) and extracted with NaHCOs (2x 10 ml). For precipitation, the combined aqueous layers were acidified to pH 2 by using 2 M HCl (50 ml). After filtration, the solid obtained was taken up in 1,4-dioxane and lyophilized overnight to yield 4-nitro-2-((prop-2-yn-1-yloxy)methyl)benzoic acid as pale red solid (1.44 g, 83 %). ¹H NMR (500 MHz, DMSO) δ 8.36 (d, *J* = 2.4 Hz, 1H), 8.22 (dd, *J* = 8.6, 2.5 Hz, 1H), 8.09 (d, *J* = 8.5 Hz, 1H), 4.94 (s, 2H), 4.36 (d, *J* = 2.4 Hz, 2H), 3.53 (t, *J* = 2.4 Hz, 1H). HPLC (method **A**): *t*_{R} = 11.3 min.

### Step 2: Synthesis of Methyl- 4-nitro-2-((prop-2-yn-1-yloxy)methyl)benzoate

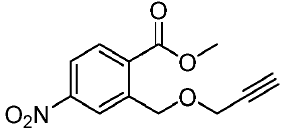

To a solution of 4-nitro-2-((prop-2-yn-1-yloxy)methyl)benzoic acid (1.44 g, 6.12 mmol, 1 equiv.) in 30 ml dry methanol was added SOCl₂ ( 3.56 ml, 49 mmol, 8 equiv.) at 0 °C. The mixture was stirred at 70 °C for 3.5 h and the progress monitored by RP-HPLC. Upon completion, the solvent and the volatiles were removed under reduced pressure. The obtained residue was taken up in 1,4-dioxane and lyophilized overnight to yield methyl- 4-nitro-2-((prop-2-yn-1-yloxy)methyl)benzoate (1.52 g, 99%) as pale brown solid. ¹H NMR (500 MHz, DMSO) δ 8.37 (dd, *J* = 2.3, 1.1 Hz, 1H), 8.28 - 8.22 (m, 1H), 8.08 (d, *J* = 8.5 Hz, 1H), 4.92 (d, *J* = 0.8 Hz, 2H), 4.34 (d, *J* = 2.4 Hz, 2H), 3.90 (s, 3H), 3.49 (t, *J* = 2.4 Hz, 1H). HPLC (method **A**): *t*_{R} = 13.1 min.

### Step 3: Synthesis of Synthesis of methyl 4-amino-2-((prop-2-yn-1-yloxy)methyl)benzoate

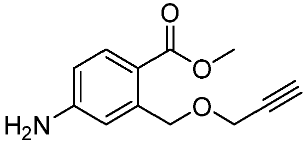

To a stirred suspension of methyl- 4-nitro-2-((prop-2-yn-1-yloxy)methyl)benzoate (1.52 g, 6.1 mmol, 1 equiv.) in a mixture of EtOH (15 ml) and water (7.5 ml) was added iron powder (2.73 g, 48.8 mmol, 8 equiv.) and NH₄Cl (2.61 g, 48.8 mmol, 8 equiv.) at room temperature. The resulting mixture was allowed to stir at 80 °C for 4 h and the progress monitored by RP-HPLC. After total consumption of the starting material, the reaction was cooled to rt and filtered through celite and washed with ethyl acetate (15 ml). The filtrate was washed with sat. sodium bicarbonate solution, water and brine (each 15 ml). The aqueous layers were extracted with ethyl acetate (15) ml. The combined organic layers were dried over MgSO₄. After filtration the solvent was removed under reduced pressure to obtain 4-amino-2-((prop-2-yn-1-yloxy)methyl)benzoate as red solid (1.29 g, 96 %) after drying *in vacuo.* ¹H NMR (500 MHz, CDCl₃) δ 7.87 (d, *J* = 8.5 Hz, 1H), 6.99 (dt, *J* = 2.2, 1.0 Hz, 1H), 6.59 (dd, *J* = 8.5, 2.5 Hz, 1H), 5.00 (s, 2H), 4.31 (d, *J* = 2.4 Hz, 2H), 3.84 (s, 3H), 2.48 (t, *J* = 2.4 Hz, 1H). HPLC (method **A**): *t*_{R} = 10.5 min.

### Step 4: Synthesis of (4-amino-2-((prop-2-yn-1-yloxy)methyl)phenyl)methanol

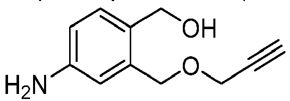

To a cooled solution (0 °C) of dry THF (13 ml) was added LiAlH₄ (1 M in THF, 12.01 mmol, 12.01 ml, 2.5 equiv.) slowly. A solution of 4-amino-2-((prop-2-yn-1-yloxy)methyl)benzoate (1.06 g, 4.8 mmol, 1 equiv.) in dry THF (10 ml) was added dropwise at 0 °C. After complete addition, the resulting mixture was allowed to stir overnight at rt. The reaction progress was monitored by RP-HPLC. The reaction mixture was poured into a vigorous stirred 1 M potassium sodium tartrate solution (120 ml) and stirred for another 30 min at rt. The reaction mixture was extracted with ethyl acetate (3x 30 ml). The combined organic layers were washed with water and brine (each 30 ml). The organic layer was dried over MgSO₄. The solvent was removed under reduced pressure to obtain crude product. Purification by ISCO SiO₂ flash chromatography (0-100 % ethyl acetate in hexane) yielded (4-amino-2-((prop-2-yn-1-yloxy)methyl)phenyl)methanol (0.65 g, 71 %). ¹H NMR (500 MHz, DMSO) δ 6.99 (d, *J* = 8.1 Hz, 1H), 6.58 (d, *J* = 2.4 Hz, 1H), 6.45 (dd, *J* = 8.0, 2.4 Hz, 1H), 4.93 (s, 2H), 4.62 - 4.53 (m, 1H), 4.47 (s, 2H), 4.35 (d, *J* = 5.4 Hz, 2H), 4.15 (d, *J* = 2.4 Hz, 2H), 3.42 (t, *J* = 2.4 Hz, 1H). HPLC (method A): *t*_{R} = 4.8 min. MS (ESI): found *m*/*z =* 174.2 [M-H₂O+H]⁺, calcd. *m*/*z* = 174.1

### Step 5: Synthesis Fmoc-Val-Ala-OH

The synthesis of Fmoc-Val-Ala-OH was performed as disclosed in WO 2017/149077.

### Example 1.2 - Syntheses of Linker-Payload Intermediates

### Step 6: Synthesis of intermediate 1.1

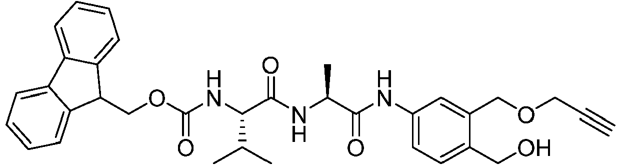

To a stirred solution of Fmoc-Val-Ala-OH (1.43g, 3.41 mmol, 1 equiv.) and (4-amino-2-((prop-2-yn-1-yloxy)methyl)phenyl)methanol (0.65 g, 3.41 mmol, 1 equiv.) in dry THF (20 ml) was added N-Ethoxycarbonyl-2-ethoxy-1,2-dihydroquinoline (EEDQ, 1.05 g, 4.1 mmol, 1.2 equiv.). at room temperature. The resulting mixture was stirred at room temperature in the absence of light for 5 d. The solvent was removed under reduced pressure. The resulting crude intermediate was purified by flash chromatography on silica (gradient from 0-5 % MeOH in DCM) to yield **intermediate 1.1** as colorless solid (1.25 g, 63 %). HPLC (method **A**): *t*_{R}= 12.9 min. MS (ESI): found *m*/*z =* 601.2 [M+NH_{4]}⁺, calcd. *m*/*z* = 601.3

### Step 7: Synthesis of intermediate 1.2

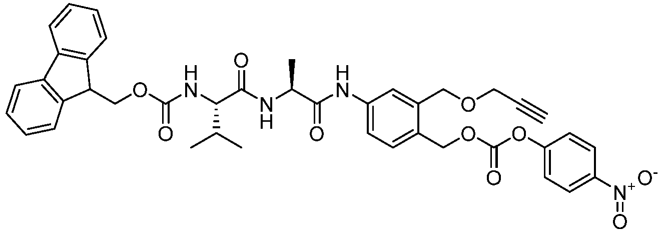

To a stirred solution of **intermediate 1.1** (0.72 g, 1.24 mmol) in dry DMF (15 ml) were added bis-4-nitrophenyl-carbonate (0.75 g, 2.48 mmol, 2 equiv.) and *N-*ethyldiisopropylamine (DIPEA, 3.1 mmol, 0.53 ml, 2.5 equiv.) under an argon atmosphere. The reaction was allowed to stir at room temperature for 4 h. After completion, the solvent was removed under reduced pressure. The crude intermediate was purified by flash chromatography on silica (gradient from 0-100 % ethylacetate in n-hexane) to yield **intermediate 1.2** (0.75 g, 81 %) as pale yellow solid after lyophilization from 1,4-dioxane:water (4:1). HPLC (method **C**): *t*_{R} = 13.4 min. MS (ESI): found *m*/*z =* 771.2 [M+Na]⁺, calcd. *m*/*z* = 771.3

### Step 8: Synthesis of Intermediate 1.3

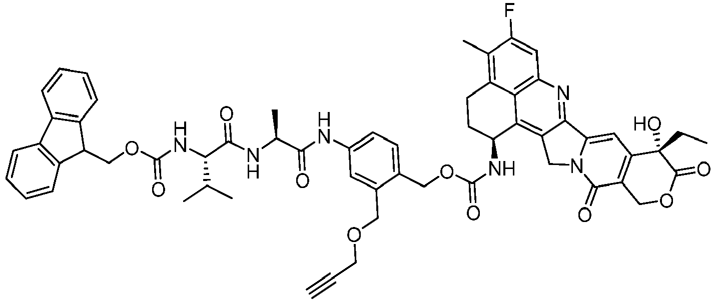

To a stirring suspension of exatecan mesylate (0.58g, 1.1 mmol, 1 equiv.) in dry DMF (20 ml) was added a freshly prepared solution of **intermediate 1.2** (0.82 g; 1.1 mmol, 1 equiv.) and 1-hydroxybenzotriazole (HOBt, 0.042 g, 0.275 mmol, 0.25 equiv.) in dry DMF (20 ml). DIPEA was added to the resulting mixture (0.28 g, 2.16 mmol, 0.37 ml, 2 equiv.) and 2,6-lutidine (141 mmol, 16.3 ml, 129 equiv.). The reaction mixture was stirred at room temperature overnight and the progress monitored by RP-HPLC. The reaction mixture was concentrated under reduced pressure. Purification by flash chromatography on silica (gradient from 0-5 % MeOH in DCM) and lyophilization from 1,4-dioxane:water (4:1) yielded **intermediate 1.3** (1.08 g, 95 %) as gray solid. HPLC (method **D**): *t*_{R} = 13.0 min. MS (ESI): found *mlz* = 1045.3 [M+H]⁺, calcd. *m*/*z* = 1045.4

### Example 1.3 - Synthesis of intermediate 2.1

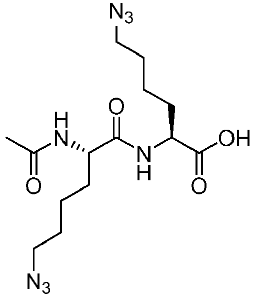

**Ac-Lys(N₃)-Lys(N₃)-OH**

2-chlorotrityl resin (4 g) were swollen for 10 min in abs. DCM (20 mL). The suspension was filtered, a solution of Fmoc-Lys(N₃)-OH (1.578 g, 4 mmol, 1 equiv.), DIPEA (3.48 mL, 20 mmol, 5 equiv.) in abs. DCM (20 mL) added and shaken for 2 h at room temperature. The suspension was filtered, the resin washed with DMF (3 × 1 min) and the capping solution (20 mL, MeOH/DIPEA/DCM 1/1/8) was added and shaken for 10 min at room temperature. The suspension was filtered and the capping procedure repeated once. The resin was washed with DMF (3 × 1 min) and DCM (3 × 1 min) and the resin dried *in vacuo.* The loading of the resin was determined as described in J. Pept. Sci. 2017, 23, 757-762.

For the next step, the resin was swollen in DMF for 10 min. The suspension was filtered and a Fmoc-deprotection solution of 20 % piperidine in DMF added to the resin. The suspension was incubated for 20 min, filtered and the deprotection procedure repeated once. Then, the resin was washed with DMF (3 × 1 min) and DCM (3 × 1 min). The resin was swollen in DMF for 10 min, filtered and a solution of Fmoc-Lys(N₃)-OH (3 equiv.), TBTU (3 equiv.) and DIPEA (6 equiv.) in DMF added. The suspension was shaken for 2 h at room temperature. The resin was washed with DMF (3 × 1 min) and DCM (3 × 1 min) and the Fmoc-group removed with 20 % piperidine as described above. For acetylation, the resin was swollen in DMF for 10 min, the suspension filtered and a solution of acetic anhydride (3 equiv.) and pyridine (3 equiv.) in DMF added. The suspension was incubated for 2 h at room temperature, filtered, the resin washed with DMF (3 × 1 min) and DCM (3 × 1 min) and dried *in vacuo.*

For cleavage, the resin was treated with a solution of hexafluoroisopropanol in DCM (1:4, 20 mL) for 15 min. The solution is collected and the resin treated again with hexafluoroisopropanol in DCM (1:4, 20 mL). The resin is washed with hexafluoroisopropanol in DCM (1:4) and the combined filtrates concentrated *in vacuo.* The resulting crude peptide was purified by RP-HPLC and **Ac-Lys(N₃)-Lys(N₃)-OH** (731 mg, 50 %) obtained as colorless lyophilizate.

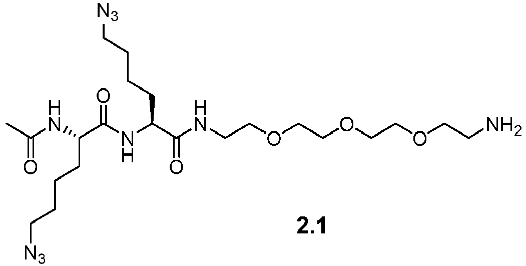

**Ac-Lys(N₃)-Lys(N₃)-OH** (731 mg, 1.98 mmol, 1 equiv.) and HOBt (910 mg, 5.94 mmol, 3 equiv.) were dissolved in DMF (8 mL). DIC (920 µL, 5.9 mmol, 3 equiv.) was added, the solution stirred for 5 min at room temperature after which a solution of Boc-Amino-PEG(3)-Amine (CAS-Nr. 101187-40-0, 868 mg, 2.97 mmol, 1.5 equiv.) in DMF (4 mL) was added followed by DIPEA (1.04 mL, 5.98 mmol, 3 equiv.). The reaction was stirred at room temperature and followed by RP-HPLC. After 20 h, the solution was concentrated and the residue dissolved in ethyl acetate. The organic layer was washed with 0.2 M citric acid (1 × 50 mL), sodium bicarbonate solution (3 × 50 mL) and brine. The organic layer was dried over magnesium sulfate and concentrated in *vacuo* giving 1.428 g of crude product. Purification by RP-HPLC followed by freeze drying in the presence of HCl (1 equiv.) for two times afforded **intermediate 2.1** (705 mg, 66 %) as HCl-salt. HPLC (method H): *t*_{R} = 11.2 min. MS (ESI): found *m*/*z* = 543.3 [M+H]⁺, calcd. *m*/*z* = 543.3 [M+H]⁺.

### Example 1.4- Synthesis of intermediate 3.1

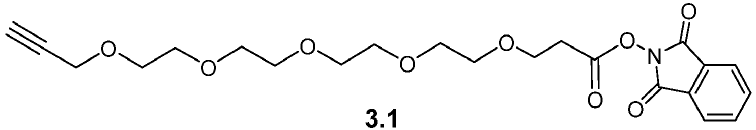

EDC x HCl (485.8 mg, 2.53 mmol, 1.3 equiv.) and DIPEA (1.09 mL, 6.24 mmol, 3.2 equiv.) were added to a solution of Propargyl-PEG(5)-acid (CAS-Nr. 1245823-51-1, 593.1 mg, 1.94 mmol, 1 equiv.) in abs. THF (8.9 mL). A solution of *N-*hydroxyphthalimide (413.4 mg, 2.53 mmol, 1.3 equiv.) in abs. THF (8.9 mL) was added dropwise for 10 minutes. The reaction was stirred at room temperature and monitored by TLC and RP-HPLC. After 21 h, the solution was diluted with ethyl acetate (60 mL) and the organic layer washed with 1N HCl (30 mL), sodium bicarbonate solution (30 mL) and brine (30 mL). The organic layer was dried over magnesium sulfate and concentrated *in vacuo.* The resulting crude product (724.7 mg) was purified by flash chromatography on silica using a gradient from n-hexane to ethyl acetate. **Intermediate 3.1** (457.3 mg, 52 %) was obtained as yellowish oil. C₂₂H₂₇NO₉. ¹H NMR (500 MHz, CDCl₃): δ (ppm) = 7.89 (dd, J = 5.5, 3.1 Hz, 2H), 7.80 (dd, J = 5.5, 3.1 Hz, 2H), 4.21 (d, J = 2.3 Hz, 2H), 3.89 (t, J = 6.4 Hz, 2H), 3.73 - 3.63 (m, 17H), 2.97 (t, J = 6.4 Hz, 2H), 2.44 (t, J = 2.4 Hz, 1H). ¹³C NMR (126 MHz, CDCl₃): δ (ppm) = 167.65, 161.73, 134.73, 128.82, 123.92, 79.61, 74.48, 70.69, 70.58, 70.54, 70.53, 70.49, 70.46, 70.33, 69.04, 65.76, 58.33, 32.14. HPLC (method **I**): *t*_{R} = 11.7 min. MS (ESI): found *m*/*z* = 472.2 [M+Na]⁺, calcd. *m*/*z* = 472.2 [M+Na]⁺.

### Example 1.5 - Synthesis of intermediate 3.2

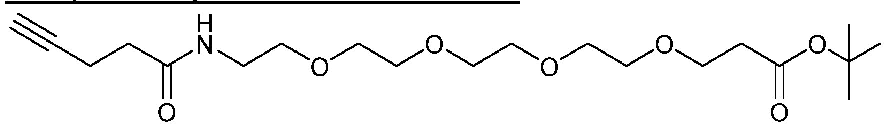

To a solution of pent-4-ynoic acid (2 g, 20.4 mmol, 1.05 equiv.) in DMF (40 mL) were added HATU (11.64 g, 30.6 mmol, 1.6 equiv.) and DIPEA (10.7 mL, 61.2 mmol) at 5 °C and the resulting solution stirred for 10 min at 5 °C. Then, a solution of tert-butyl 1-amino-3,6,9,12-tetraoxapentadecan-15-oate (6.24 g, 19.4 mmol, 1 equiv.) in DMF (10 mL) was added dropwise and the resulting solution allowed to warm to room temperature. After 2 h, the solution was concentrated *in vacuo* and the residue taken up in ethyl acetate (300 mL). The organic layer was washed with 0.2 M citric acid (150 mL), saturated sodium bicarbonate solution (150 mL) and brine (150 mL). The organic layer was dried over magnesium sulfate and concentrated *in vacuo.* Purification by flash chromatography on silica using a gradient from hexane to acetone afforded tert-butyl 1-(pent-4-ynamido)-3,6,9,12-tetraoxapentadecan-15-oate (8.20 g, quant.). C₂₀H₃₅NO₇. HPLC (method **A**): *t*_{R} = 10.6 min. MS (ESI): found *m*/*z* = 424.4 [M+Na]⁺, calcd. *m*/*z* = 424.2 [M+Na]⁺.

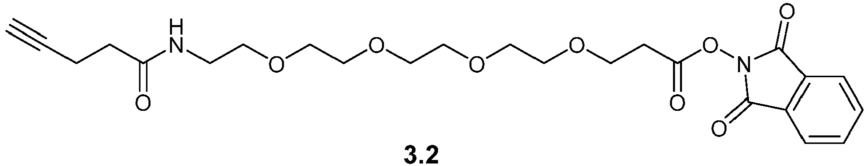

tert-Butyl 1-(pent-4-ynamido)-3,6,9,12-tetraoxapentadecan-15-oate (8.20 g, max. 19.4 mmol, 1 equiv.) was dissolved in TFA (15.6 mL) and stirred for 1 h at 300 mbar. TFA was removed *in vacuo* and the residue co-evaporated twice with toluene. The residue obtained was dissolved in THF (abs., 50 mL) and *N*-Ethyl-*N*'-carbodiimide hydrochloride (4.66 g, 24.3 mmol, 1.25 equiv.) as well as DIPEA (11 mL, 63 mmol, 3.25 mmol) were added. Then, a solution of *N*-hydroxyphtalimide (3.96 g, 24.3 mmol, 1.25 equiv.) in THF (abs., 50 mL) was added dropwise and the resulting reddish solution stirred overnight at room temperature. Ethyl acetate (400 mL) was added and the organic layer washed with 2N HCl (100 mL), saturated sodium bicarbonate solution (100 mL) and brine (100 mL). The organic layer was dried over magnesium sulfate and concentrated *in vacuo.* Purification by flash chromatography on silica using a gradient from dichloromethane to acetone afforded **intermediate 3.2** (4.96 g, 52 %) as yellowish oil. C₂₄H₃₀N₂O₉. ¹H NMR (500 MHz, DMSO-d6): δ (ppm) = 8.01 - 7.89 (m, 4H), 7.87 (s, 1H), 3.77 (t, *J* = 6.0 Hz, 2H), 3.63 - 3.46 (m, 12H), 3.40 (t, *J* = 5.9 Hz, 2H), 3.20 (q, *J* = 5.8 Hz, 2H), 3.01 (t, *J* = 6.0 Hz, 2H), 2.70 (t, *J* = 2.6 Hz, 1H), 2.39 - 2.31 (m, 2H), 2.31 - 2.23 (m, 2H). ¹³C NMR (126 MHz, DMSO-d6): δ (ppm) = 170.18, 168.21, 161.61, 135.39, 128.10, 123.85, 83.65, 71.00, 69.73, 69.68, 69.62, 69.60, 69.50, 69.01, 65.18, 38.50, 34.02, 31.55, 14.09. HPLC (method **A**): *t*_{R} = 10.5 min. MS (ESI): found *m*/*z* = 491.4 [M+H]⁺, calcd. *m*/*z* = 491.2 [M+H]⁺.

### Example 1.6 - Synthesis of intermediates 4.x (N₃-PEGₙ-beta/gamma-CD)

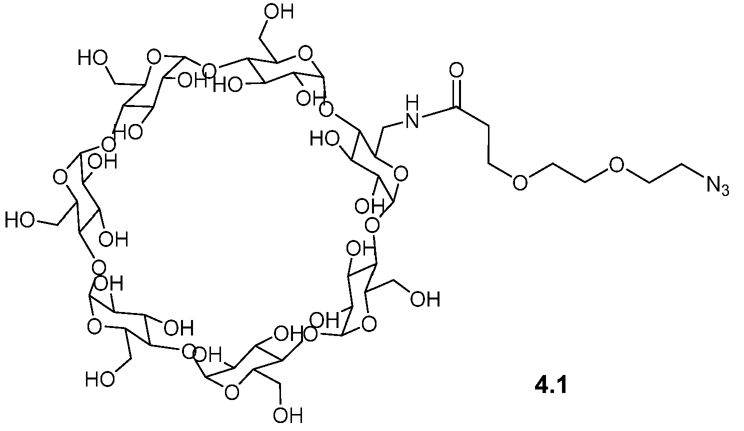

**Intermediate 4.1** was synthesized according to GP5 using azido-PEG(2)-NHS ester and 6-amino-6-deoxy-beta-cyclodextrin. The obtained crude product was used without further purification.

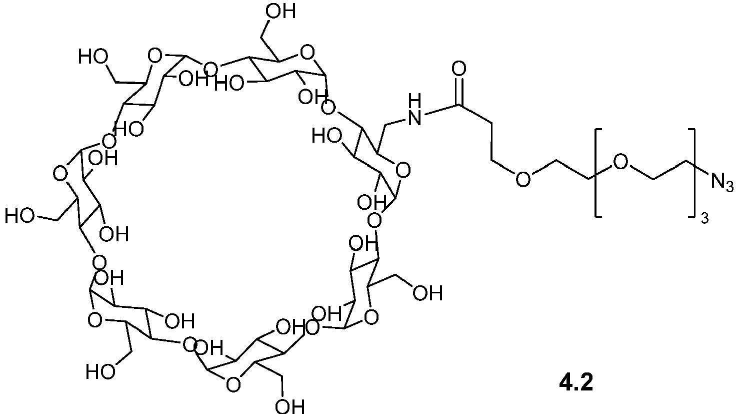

**Intermediate 4.2** was synthesized according to GP5 using azido-PEG(4)-NHS ester and 6-amino-6-deoxy-beta-cyclodextrin. The obtained crude product was used without further purification.

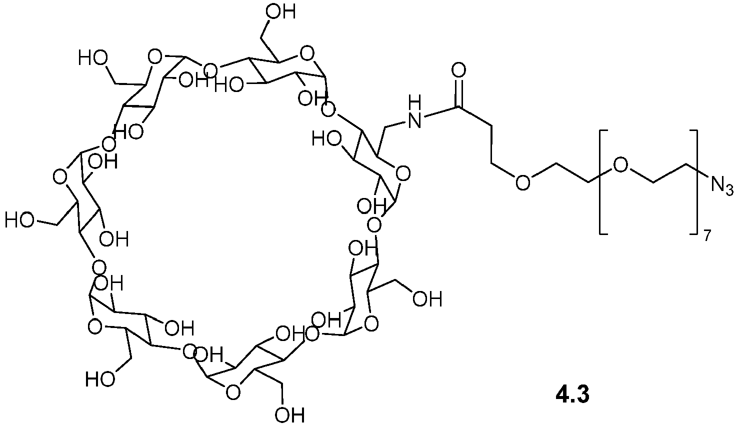

**Intermediate 4.3** was synthesized according to GP5 using azido-PEG(8)-NHS ester and 6-amino-6-deoxy-beta-cyclodextrin. The obtained crude product was used without further purification.

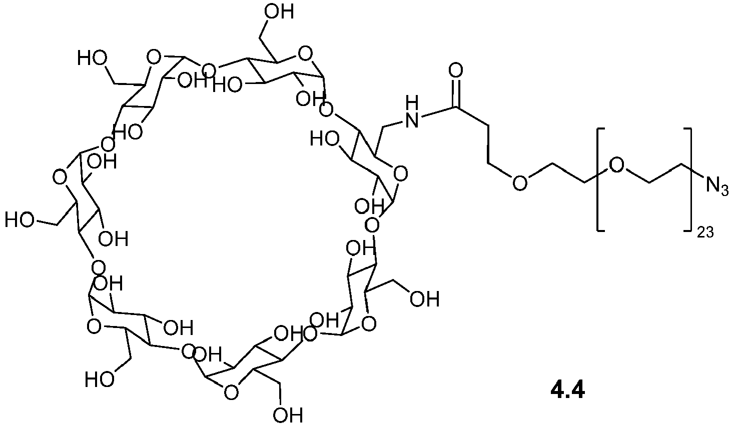

**Intermediate 4.4** was synthesized according to GP5 using azido-PEG(24)-NHS ester and 6-amino-6-deoxy-beta-cyclodextrin. The obtained crude product was used without further purification.

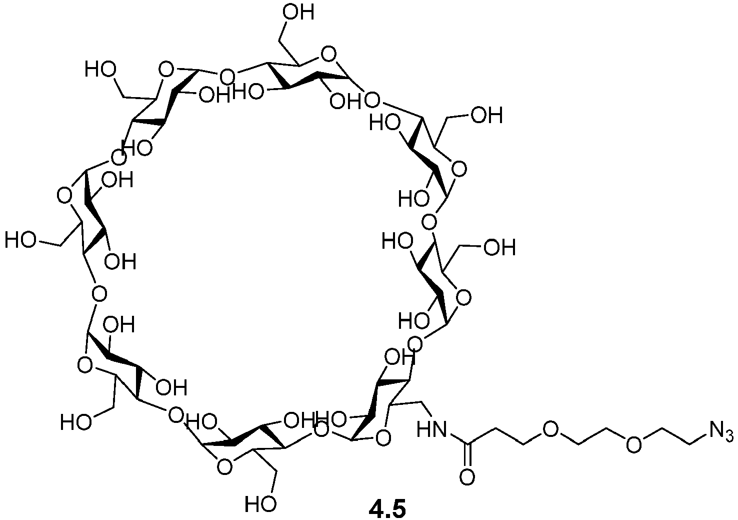

**Intermediate 4.5** was synthesized according to GP5 using azido-PEG(2)-NHS ester and 6-amino-6-deoxy-gamma-cyclodextrin. The obtained crude product was used without further purification.

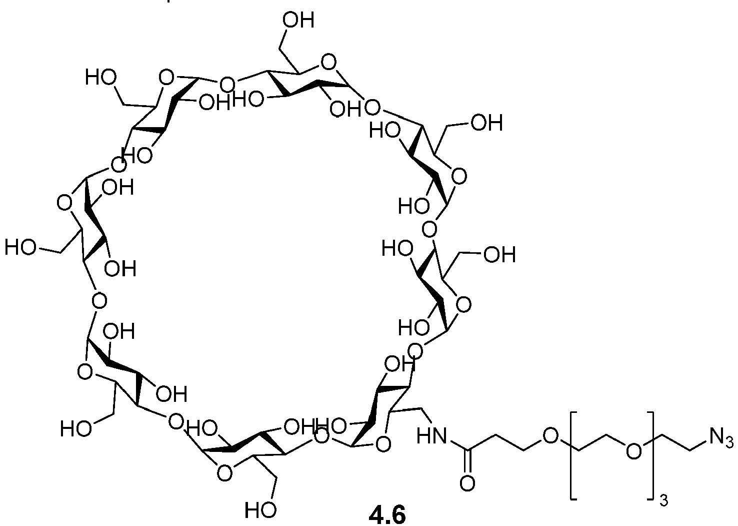

**Intermediate 4.6** was synthesized according to GP5 using azido-PEG(4)-NHS ester and 6-amino-6-deoxy-gamma-cyclodextrin. The obtained crude product was used without further purification.

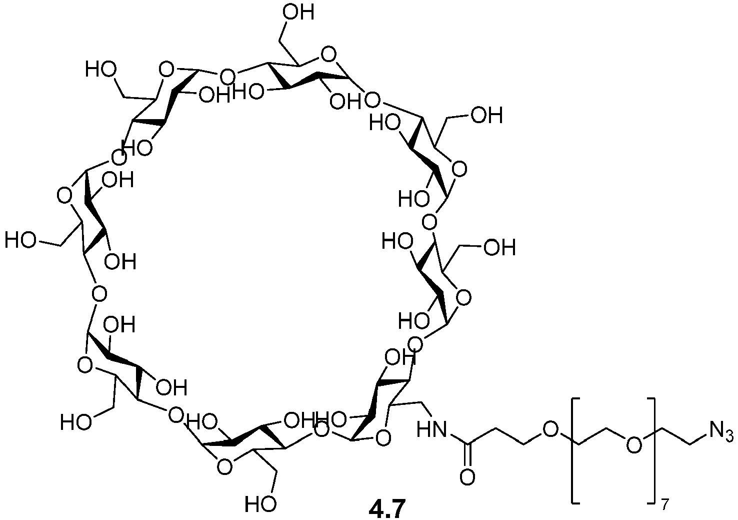

**Intermediate 4.7** was synthesized according to GP5 using azido-PEG(8)-NHS ester and 6-amino-6-deoxy-gamma-cyclodextrin. The obtained crude product was used without further purification.

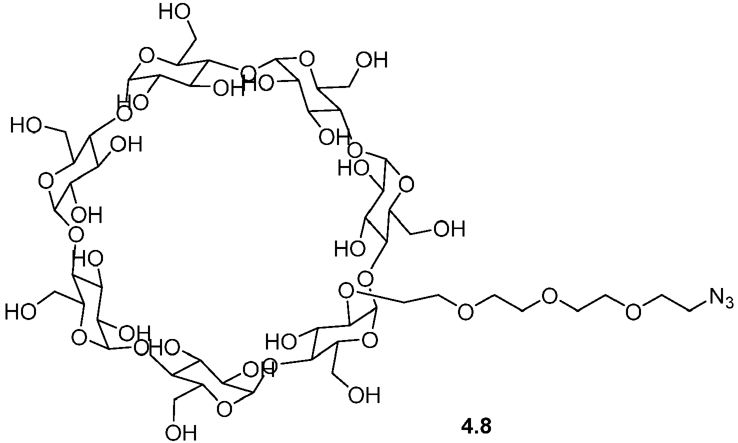

Beta-cyclodextrin (2.000 g. 1.762 mmol) was co-evaporated twice with 30 ml dry DMSO (abs.) and re-dissolved in 30 ml DMSO. Lithium hydride (21 mg, 2.643 mmol, 1.5 equiv.) was added and the suspension was stirred under Argon atmosphere for 15 h. Subsequently, 1-azido-2-{2-[2-(2-bromoethoxy)ethoxy]ethoxy}ethane (497 mg, 1.762 mmol, 1.0 equiv.) dissolved in 2 ml DMSO (abs.) was added, followed by Lithium iodide (24 mg 176 µmol, 0.1 equiv.) and the mixture was stirred at 55 °C for 7.5 h and then at room temperature. After TLC (acetonitrile/water/conc. ammonia 6:3:1) indicated no further conversion, the mixture was concentrated to approximately 6 ml by *in vacuo* and the remaining was added dropwise to 160 ml ice cooled acetone. The resulted precipitate was isolated by centrifugation, resuspended in 120 ml cold acetone by sonification and centrifuged again. After drying *in vacuo,* the solid was taken up in 5 ml water, insoluble beta-cyclodextrin removed by filtration and the filtrate was freeze-dried. The crude product (2.263 g) was acidified to pH 2 with trifluoro acetic acid and purified by prep. RP-HPLC to result in 532 mg (23 %) pure **intermediate 4.8** as colorless lyophilisate. MS (ESI): found *m*/*z* = 1336.8 [M+H]⁺, calcd. *m*/*z* = : 1336.5 [M+H]⁺.

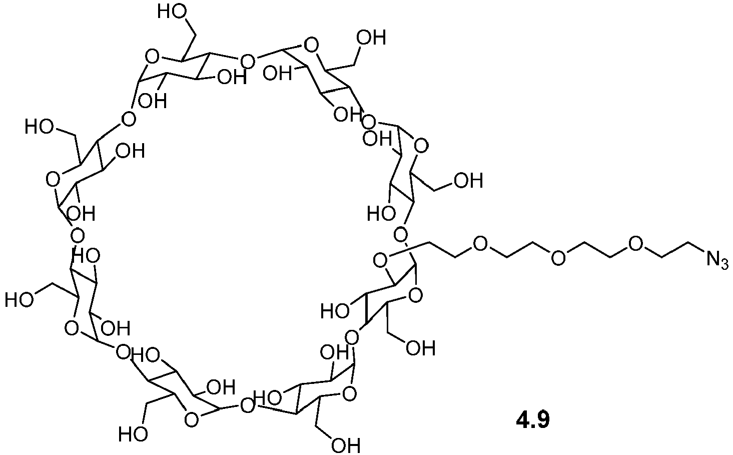

**Intermediate 4.9** was synthesized according to **intermediate 4.8** using gamma-cyclodextrin. The target product was obtained in 22% yield as a colorless lyophilisate. MS (ESI): found *m*/*z* = 1498.9 [M+H]⁺, calcd. *m*/*z* = 1498.5 [M+H]⁺.

### Example 1.7 - Synthesis of intermediates 5.x

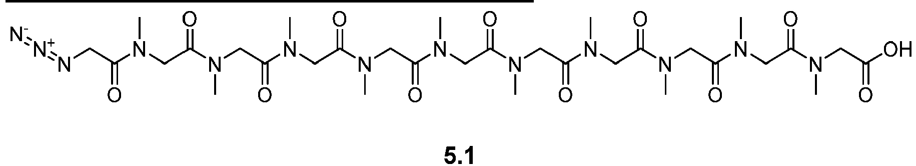

**Intermediate 5.1** was synthesized according to WO2019081455 and WO2022207699 using Wang resin loaded with Fmoc-Sar, Fmoc-Sar-Sar-OH, Fmoc-Sar-OH and azidoacetic acid for *N*-terminal capping. Fmoc-deprotection was performed with 20 % piperidine in DMF for 15 min (twice). Amino acid coupling was performed using amino acid (3 equiv.), HATU (2.9 equiv.) and DIPEA (6 equiv.) in DMF. Cleavage from resin was performed using neat TFA (10 mL/g resin) followed by precipitation from ice-cold methyl tert-butyl ether and purification by RP-HPLC. **Intermediate 5.1** (166.7 mg, 41 %) was obtained as colourless solid. C₃₂H₅₃N₁₃O₁₂. HPLC (method H): *t*_{R} = 7.4 min. MS (ESI): found *m*/*z* = 834.2 [M+Na]⁺, calcd. *m*/*z* = 834.4 [M+Na]⁺.

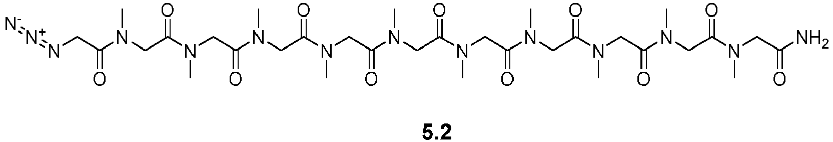

**Intermediate 5.2** was synthesized according to WO2019081455 and WO2022207699 using NovaGel Rink-Amide resin loaded with Fmoc-Sar, Fmoc-Sar-Sar-OH, Fmoc-Sar-OH and azidoacetic acid for *N*-terminal capping. Fmoc-deprotection was performed with 20 % piperidine in DMF for 15 min (twice). Amino acid coupling was performed using amino acid (3 equiv.), HATU (2.9 equiv.) and DIPEA (6 equiv.) in DMF. Cleavage from resin was performed using TFA/TIS 95/5 (10 mL/g resin) followed by precipitation from ice-cold methyl tert-butyl ether and purification by RP-HPLC. **Intermediate 5.2** (83.6 mg, 54 %) was obtained as colourless solid. C₃₂H₅₄N₁₄O₁₁. HPLC (method K): *t*_{R} = 7.1 min. MS (ESI): found *m*/*z* = 811.5 [M+H]⁺, calcd. *m*/*z* = 811.4 [M+H]⁺.

### Example 1.8 - Synthesis of intermediates 6.x

### Synthesis of intermediate 6.1

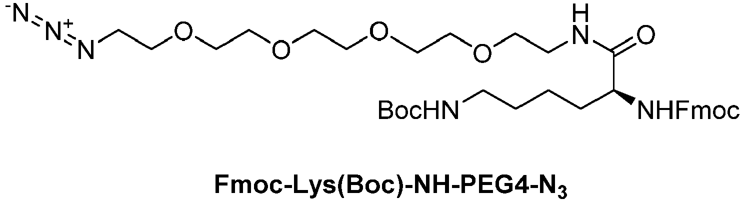

To a solution of Fmoc-Lys(Boc)-OH (3.056 g, 6.5 mmol, 1 equiv.) in DMF (30 mL) were added TBTU (2.467 g, 7.8 mmol, 1.2 equiv) and DIPEA (1.33 mL, 7.8 mmol, 1.2 equiv.) and the resulting solution mixed for 1 min. Then, the pre-activated amino acid solution was added to a solution of 14-azido-3,6,9,12-tetraoxatetradecan-1-amine (1.680 g, 6.5 mmol, 1 equiv.) in DMF (10 mL) and the resulting solution stirred for 2 h at room temperature. Water (5 mL) was added and the solution evaporated in *vacuo.* The residue was dissolved in ethyl acetate (100 mL) and the organic layer washed with aqueous 0.2 M citric acid solution (50 mL), aqueous saturated sodium bicarbonate solution (50 mL) and brine (50 mL). The organic layer was dried over magnesium sulfate and evaporated *in vacuo.* Purification by flash chromatography on silica using a gradient from MTBE to acetone afforded **Fmoc-Lys(Boc)-NH-PEG4-N₃** (3.545 g, 76 %) as colourless, sticky solid. C₃₆H₅₂N₆O₉. ¹H NMR (500 MHz, DMSO-d6): δ (ppm) = 7.88 (dt, J = 7.7, 0.9 Hz, 2H), 7.82 (t, J = 5.7 Hz, 1H), 7.76 - 7.68 (m, 2H), 7.42 (td, J = 7.5, 1.0 Hz, 2H), 7.33 (tt, J = 7.4, 1.2 Hz, 3H), 6.68 (s, 1H), 4.30 - 4.18 (m, 3H), 3.93 (td, J = 8.6, 5.2 Hz, 1H), 3.62 - 3.56 (m, 2H), 3.55 - 3.48 (m, 12H), 3.41 (t, J = 6.0 Hz, 2H), 3.37 (dd, J = 5.6, 4.4 Hz, 2H), 3.21 (ddt, J = 19.6, 13.6, 7.6 Hz, 2H), 2.89 (q, J = 10.3, 8.3 Hz, 2H), 1.69 - 1.43 (m, 2H), 1.37 (s, 9H), 1.24 (s, 4H). ¹³C NMR (126 MHz, DMSO-d6): δ (ppm) = 171.86, 155.78, 155.45, 143.79, 143.70, 140.60, 127.49, 126.92, 125.17, 119.95, 77.21, 69.71, 69.69, 69.67, 69.63, 69.60, 69.48, 69.11, 68.88, 65.50, 54.56, 49.93, 46.62, 38.47, 31.63, 29.08, 28.16, 22.69. HPLC (method A): *t*_{R} = 14.3 min. MS (ESI): found *m*/*z =* 735.4 [M+Na]⁺, calcd. *m*/*z* = 735.4 [M+Na]⁺.

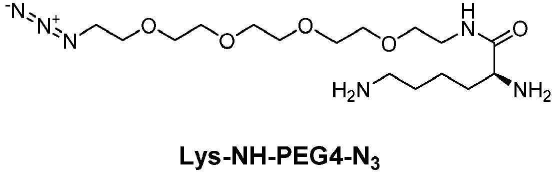

To a solution Fmoc-Lys(Boc)-NH-PEG4-N₃ (2.00 g, 2.8 mmol, 1 equiv.) in DMF (20 mL) was added diethylamine (5.00 mL, 56.1 mmol, 17 equiv.) and the resulting solution stirred for 30 min at 600 mbar at room temperature. The solvent was removed *in vacuo* and purification by flash chromatography on silica using a gradient from dichloromethane to dichloromethane/MeOH/conc. ammonia (9:1:0.1) afforded the Fmoc-deprotected intermediate H-Lys(Boc)-NH-PEG4-N₃ (1.182 g, 86 %) as colourless oil. Next, to a solution of H-Lys(Boc)-NH-PEG4-N₃ (1.163 g, 2.4 mmol, 1 equiv.) in 1,4-dioxane (20 mL) was added 1M HCl (11.85 mL, 11.8 mmol, 5 equiv.) and stirred for 10 min at 600 mbar at room temperature. The solvent was removed in *vacuo,* the residue dissolved in 1M HCl (11.85 mL, 11.8 mmol, 5 equiv.) and stirred for 10 min at 600 mbar at room temperature. The solvent was removed *in vacuo* and additionally two times co-evaporated with 1,4-dioxane. **Lys-NH-PEG4-N₃** (1.119 g, quant.) was obtained as highly viscous, colorless oil. C₁₆H₃₄N₆O₅. ¹H NMR (500 MHz, DMSO-d6): δ (ppm) = 8.67 (t, J = 5.6 Hz, 1H), 8.32 (s, 3H), 8.11 (s, 3H), 3.78 - 3.72 (m, 1H), 3.61 - 3.58 (m, 2H), 3.59 - 3.47 (m, 12H), 3.45 (t, J = 5.9 Hz, 2H), 3.38 (dd, J = 5.5, 4.4 Hz, 2H), 3.28 (d, J = 5.9 Hz, 1H), 3.23 (dq, J = 13.7, 5.8 Hz, 1H), 2.73 (d, J = 8.7 Hz, 2H), 1.73 (q, J = 7.3 Hz, 2H), 1.59 (qd, J = 8.5, 7.8, 6.0 Hz, 2H), 1.42 - 1.33 (m, 2H). ¹³C NMR (126 MHz, DMSO-d6): δ (ppm) = 168.43, 69.74, 69.70, 69.66, 69.62, 69.49, 69.13, 68.63, 51.78, 49.97, 38.66, 38.11, 30.18, 26.08, 20.97. HPLC (method H): *t*_{R} = 6.9 min. MS (ESI): found *m*/*z* = 391.3 [M+H]⁺, calcd. *m*/*z* = 391.3 [M+H]⁺.

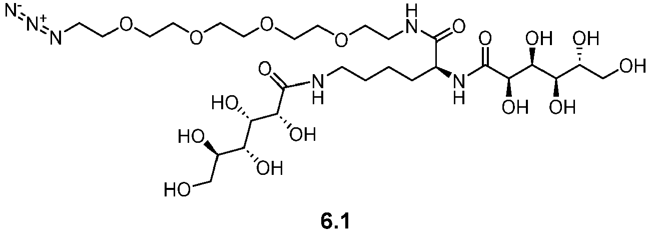

To a solution of Lys-NH-PEG4-N₃ (500 mg, 1.1 mmol, 1 equiv.) in DMF (10 mL) were added D-Glucono-1,5-lactone (481 mg, 2.7 mmol, 2.5 equiv.) and DIPEA (917 µL, 5.4 mmol, 5.0 equiv.) and the resulting solution stirred for 15 h at room temperature followed by 1 h at 80 °C. After 16.5 h reaction time in total, D-Glucono-1,5-lactone (481 mg, 2.7 mmol, 2.5 equiv.) and DIPEA (917 µL, 5.4 mmol, 5.0 equiv.). After 21 h reaction time in total, the solvent was removed *in vacuo* and the residue co-evaporated with a mixture of methanol (10 mL) and DIPEA (917 µL) followed by coevaporation with methanol (10 mL). The crude product was precipitated from ice-cold MTBE. Purification by preparative HPLC afforded **intermediate 6.1** (294 mg, 36 %) as colourless, glass-like solid. C₂₈H₅₄N₆O₁₇. ¹H NMR (500 MHz, DMSO-d6): δ (ppm) = 7.90 (t, J = 5.7 Hz, 1H), 7.61 (d, J = 8.4 Hz, 1H), 7.53 (t, J = 5.9 Hz, 1H), 4.41 (s, 10H), 4.22 (td, J = 8.4, 5.0 Hz, 2H), 4.06 (d, J = 3.5 Hz, 1H), 3.97 (d, J = 3.7 Hz, 1H), 3.93 - 3.86 (m, 3H), 3.62 - 3.56 (m, 4H), 3.56 - 3.43 (m, 14H), 3.43 - 3.35 (m, 4H), 3.20 (qd, J = 6.0, 3.5 Hz, 4H), 3.05 (dp, J = 13.9, 6.9 Hz, 2H), 1.73 - 1.64 (m, 1H), 1.56 (tdd, J = 13.5, 9.4, 5.3 Hz, 1H), 1.45 - 1.33 (m, 2H), 1.23 (s, 2H). ¹³C NMR (126 MHz, DMSO-d6): δ (ppm) = 172.28, 172.17, 171.28, 73.50, 73.32, 72.36, 72.16, 71.46, 70.42, 70.10, 69.72, 69.64, 69.50, 69.14, 68.75, 63.29, 63.24, 51.99, 49.97, 38.52, 38.08, 31.75, 28.77, 22.46. HPLC (method M): *t*_{R} = 13.5 min. MS (ESI): found *m*/*z* = 747.4 [M+H]⁺, calcd. *m*/*z* = 747.4 [M+H]⁺.

### Synthesis of intermediate 6.2

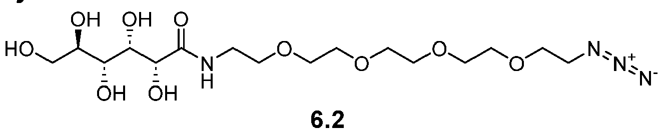

14-Azido-3,6,9,12-tetraoxatetradecan-1-amine (262 mg, 1.0 mmol, 1 equiv.) was dissolved in DMF (2 mL). D-Glucono-1,5-lactone (214 mg, 1.2 mmol, 1.2 equiv.) was added as solid followed by the addition of DIPEA (348 µL, 2.0 mmol, 2 equiv.) and the resulting solution stirred for 2 h at 110 °C. After cooling to room temperature, the solvent was evaporated *in vacuo.* Purification by flash chromatography on silica using a gradient from dichloromethane to dichloromethane/methanol/water (5:1:0.1) afforded intermediate **6.1** (318 mg, 72 %). C₁₆H₃₂N₄O₁₀. HPLC (method M): *t*_{R} = 13.7 min. MS (ESI): found *m*/*z* = 441.3 [M+H]⁺, calcd. *m*/*z* = 441.2 [M+H]⁺.

### Synthesis of intermediate 6.3

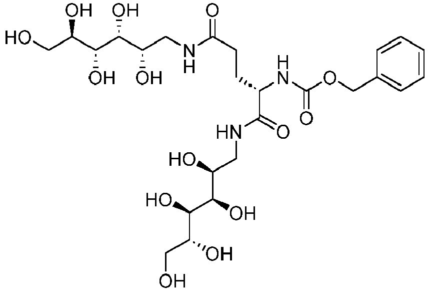

### Z-L-Glu(NHDG)-NHDG

To a solution of Z-L-Glu-OH (2.50 g, 8.9 mmol, 1 equiv.) in THF (abs., 50 mL) was added *N*-hydroxy-succinimid (2.32 g, 20.2 mmol, 2.3 equiv.) and the solution cooled to 0 °C. Then, *N,N*'-dicyclohexylcarbodiimide (3.85 g, 18.7 mmol, 2.1 equiv.) was added and the resulting suspension was stirred 2 h at 0 °C followed by stirring overnight at room temperature. For work-up, the formed urea was removed by filtration and the oily residue was dissolved in ethyl acetate (75 mL). The organic layer was washed with saturated aqueous sodium bicarbonate solution, brine and water, dried over magnesium sulfate and the solvent removed *in vacuo.* After treatment with diethyl ether, Z-L-Glu(Osu)-Osu (3.68 g, 87 %) was obtained as intermediate, which was used without further purification.

Next, to a solution of Z-L-Glu(Osu)-Osu (1.00 g, 2.1 mmol, 1 equiv.) in DMF (abs., 40 mL) was added D-glucamine (762.23 mg, 4.2 mmol, 2 equiv.) and the resulting suspension stirred for 3 h at 65 °C. As the reaction progressed, the initial suspension became a solution. The solution was removed *in vacuo* and purification by RP-HPLC afforded **Z-L-Glu(NHDG)-NHDG** (933.9 mg, 73 %). C₂₅H₄₁N₃O₁₄. HPLC (method H): *t*_{R} = 8.0 min. MS (ESI): found *m*/*z* = 630.3 [M+Na]⁺, calcd. *m*/*z* = 630.2 [M+Na]⁺.

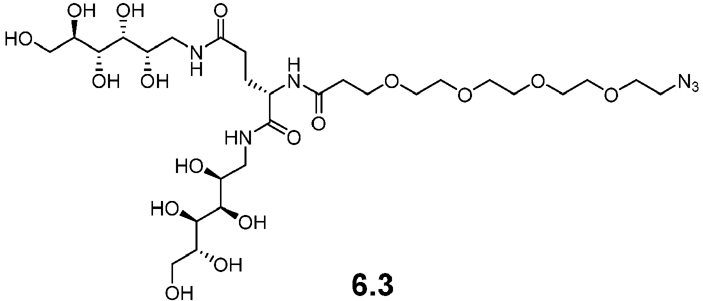

To a solution of **Z-L-Glu(NHDG)-NHDG** (924.9 mg, 1.5 mmol) in methanol (180 mL) was added Pd/C (10%, 161 mg) under Argon. The atmosphere was changed from argon to hydrogen, and the resulting suspension was stirred for 3.5 h at room temperature. The catalyst was removed by filtration through Celite and the solvent of the resulting filtrate was removed *in vacuo* affording H-L-Glu(NHDG)-NHDG (728.7 mg, 1.5 mmol, quant.). Next, H-L-Glu(NHDG)-NHDG (100.0 mg, 211.2 µmol, 1.5 equiv.) was dissolved in DMF (abs., 2 mL) and added to a solution of azido-PEG4-NHS ester (CAS-Nr. 944251-24-5, 54.68 mg, 140.8 µmol, 1 equiv.) under an atmosphere of argon. DIPEA (73.57 µL, 422.4 µmol, 3 equiv) was added and the resulting solution stirred for 1.5 h at room temperature. The solvent was removed in *vacuo* and purification by RP-HPLC afforded **intermediate 6.3** (82.74 mg, 79 %). C₂₈H₅₄N₆O₁₇. HPLC (method A): *t*_{R} = 5.9 min. MS (ESI): found *m*/*z* = 747.2 [M+H]⁺, calcd. *m*/*z* = 747.4 [M+H]⁺.

### Synthesis of intermediate 6.4

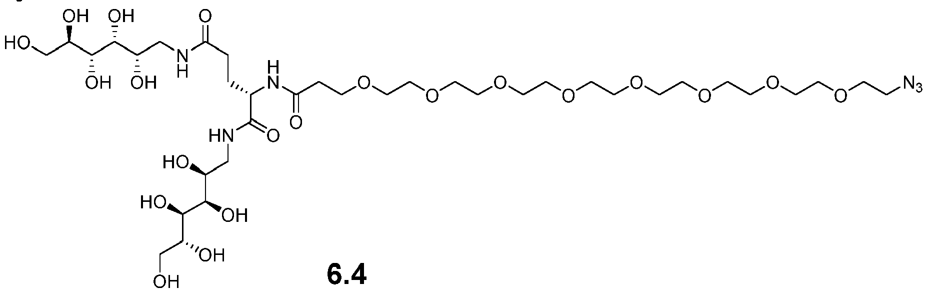

Intermediate 6.4 (91.9 mg, 71 %) was synthesized as described for intermediate 6.3 but using azido-PEG8-NHS ester (CAS-Nr. 1204834-00-3) instead of azido-PEG4-NHS ester. C₃₆H₇₀N₆O₂₁. HPLC (method A): *t*_{R} = 6.7 min. MS (ESI): found *m*/*z* = 923.6 [M+H]⁺, calcd. *m*/*z* = 923.5 [M+H]⁺.

### Example 2 - Synthesis of multimeric linker exatecans

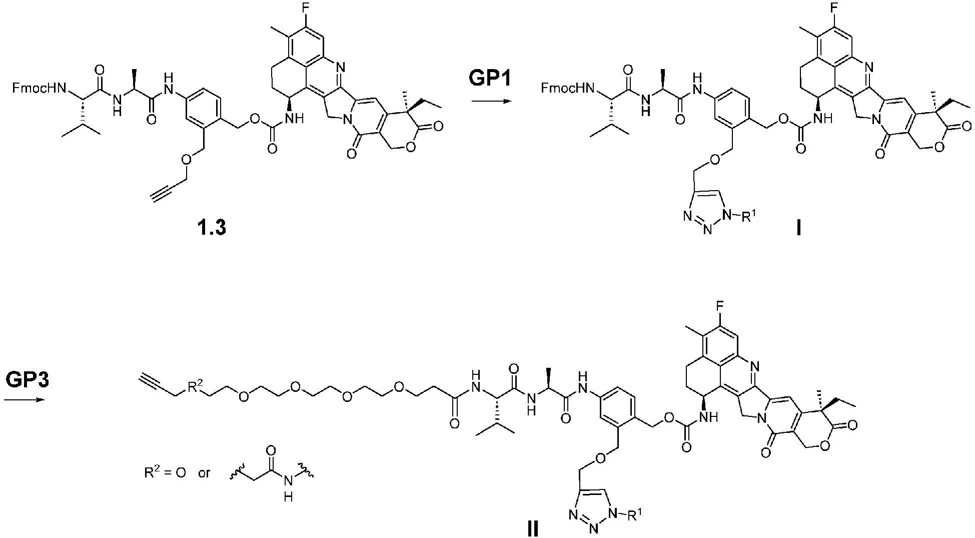

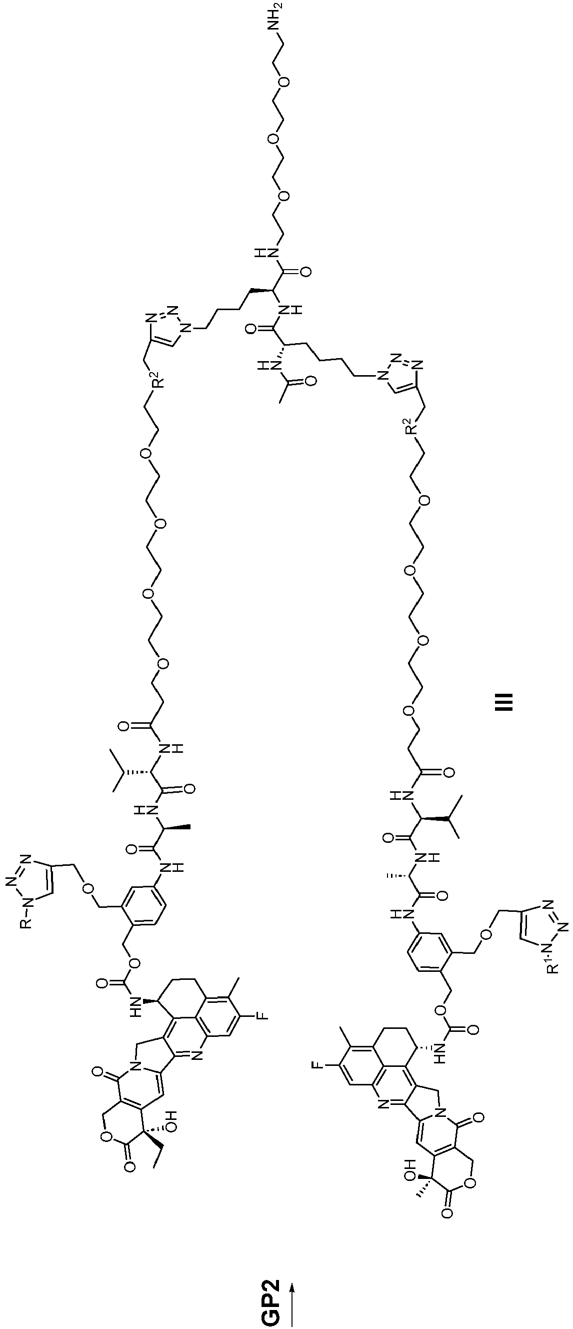

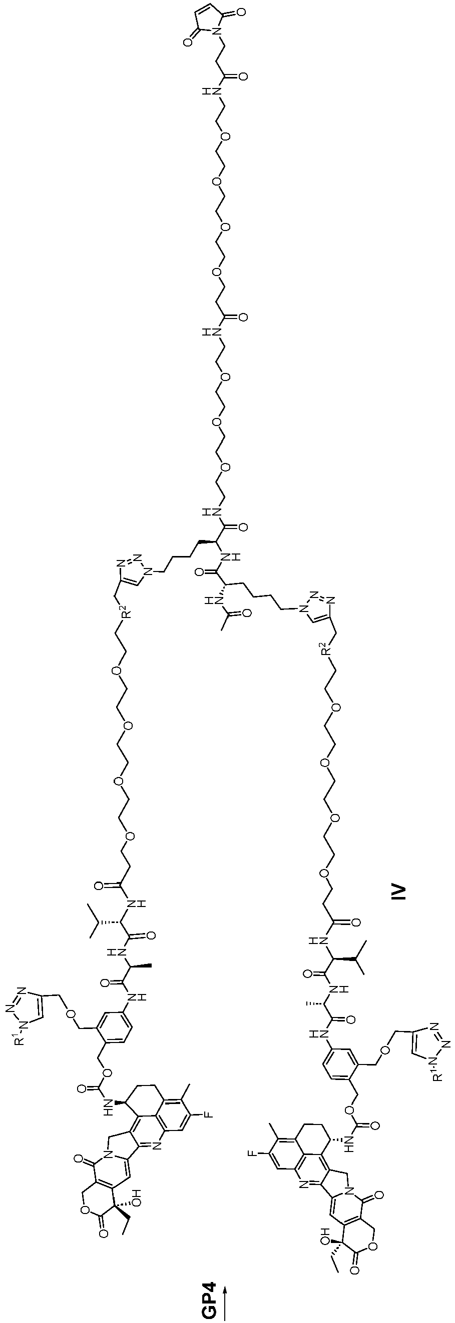

### Example 3 - Synthesis of Compound IV.1

### Synthesis of I.1

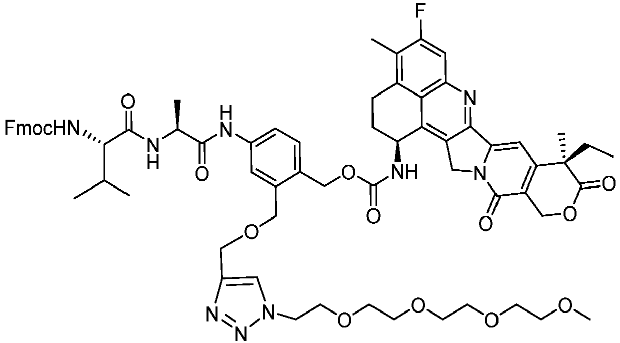

Intermediate **I.1** was synthesized according to GP1 using m-PEG(4)-azide (40.4 mg, 173 µmol, 1.2 equiv.), intermediate **1.3** (150 mg, 144 µmol, 1.0 equiv.), copper(II) sulfate pentahydrate (3.6 mg, 14.4 µmol, 0.1 equiv.), tris(benzyltriazolylmethyl)amine (9.6 mg, 18 µmol, 0.125 equiv.) and sodium ascorbate (7.1 mg, 36 µmol, 0.25 equiv.) in DMF/water (1.65 mL, 10:1). Purification by RP-HPLC afforded intermediate **I.1** (137 mg, 74 %) as yellowish solid. HPLC (method D): *t*_{R} = 11.8 min. MS (ESI): found *mlz* = 1278.5 [M+H]⁺, calcd. *mlz* = 1278.5 [M+H]⁺.

### Synthesis of II.1

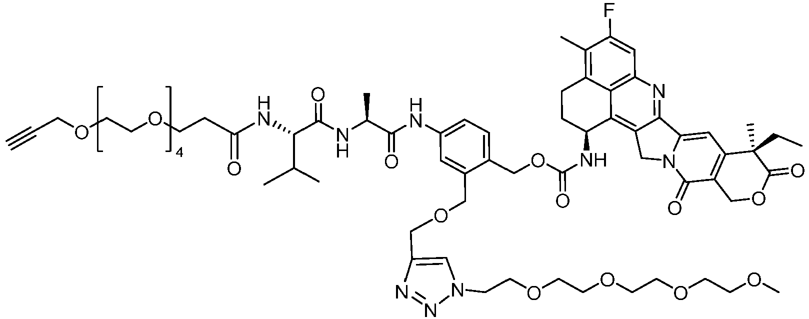

Intermediate **II.1** was synthesized according to GP3 using 222 µL diethylamine (2.14 mmol, 20 equiv.) in 1.37 mL DMF for Fmoc-deprotection and intermediate **I.1** (137 mg, 107 µmol, 1 equiv.), **intermediate 3.1** (53.0 mg, 118 µmol, 1.1 equiv.), diisopropylethylamine (56 µL, 321 µmol, 3 equiv.) in DMF (1 mL) for the coupling reaction. Purification by RP-HPLC afforded intermediate **II.1** (101 mg, 70 %) as yellowish solid. HPLC (method A): *t*_{R} = 11.1 min. MS (ESI): found *m*/*z =* 1342.5 [M+H]⁺, calcd. *m*/*z* = 1342.6 [M+H]⁺.

### Synthesis of III.1

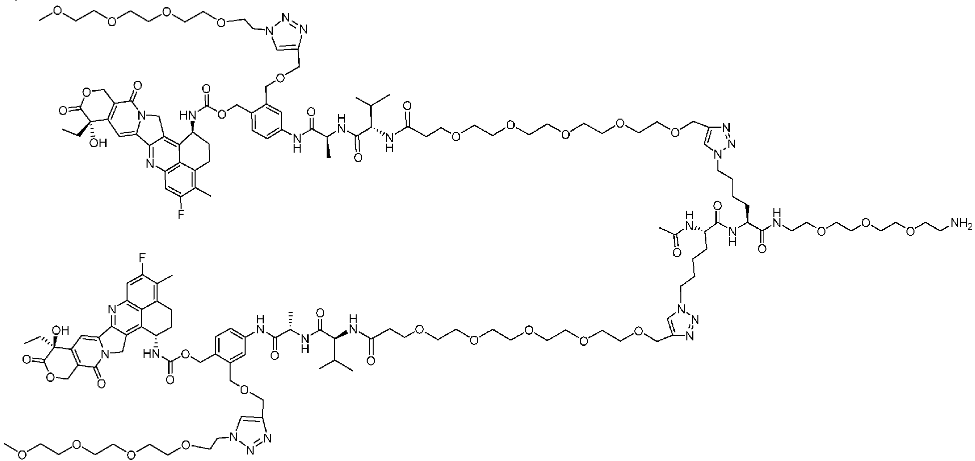

Intermediate **III.1** was synthesized according to GP2 using **intermediate 2.1** (3.3 mg, 5.0 µmol, 1 equiv.), intermediate **II.1** (17.3 mg, 12.9 µmol, 2.5 equiv.), copper(II) sulfate pentahydrate (1.6 mg, 6.5 µmol, 1.25 equiv.), Tris(3-hydroxypropyltriazolylmethyl)amine (5.6 mg, 12.9 µmol, 2.5 equiv.) and sodium ascorbate (4.2 mg, 21.4 µmol, 4.3 equiv.) in DMF/water (0.55 mL, 10:1). Purification by RP-HPLC afforded intermediate **III.1** (22.4 mg, quant.) as yellowish solid. HPLC (method J): *t*_{R} = 15.0 min. MS (ESI): found *m*/*z* = 1614.17 [M+2H]²⁺, calcd. *m*/*z* = 1613.8 [M+2H]²⁺.

### Synthesis of IV.1

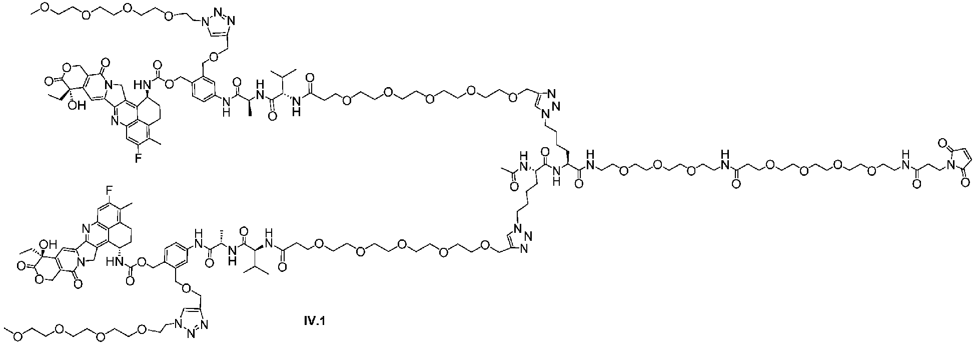

Compound **IV.1** was synthesized according to GP4 using the TFA-salt of intermediate **III.1** (16.3 mg, 4.9 µmol, 1 equiv.), *N*-[15-[(2,5-Dioxo-1-pyrrolidinyl)oxy]-15-oxo-3,6,9,12-tetraoxapentadec-1-yl]-2,5-dihydro-2,5-dioxo-1H-pyrrole-1-propanamide = Mal-PEG4-NHS-ester (10.8 mg, 21.0 µmol, 4.3 equiv.) and DIPEA (3.3 µL, 19.0 µmol, 3.9 equiv.) in DMF (652 µL). Purification by RP-HPLC afforded compound **IV.1** (7.4 mg, 42 %) as yellowish solid. C₁₇₄H₂₄₄F₂N₃₀O₅₂. HPLC (method J): *t*_{R} = 16.7 min. MS (ESI): found *m*/*z =* 1209.6 [M+3H]³⁺, calcd. *m*/*z* = 1208.9 [M+3H]³⁺.

### Example 4 - Synthesis of Compound IV.2

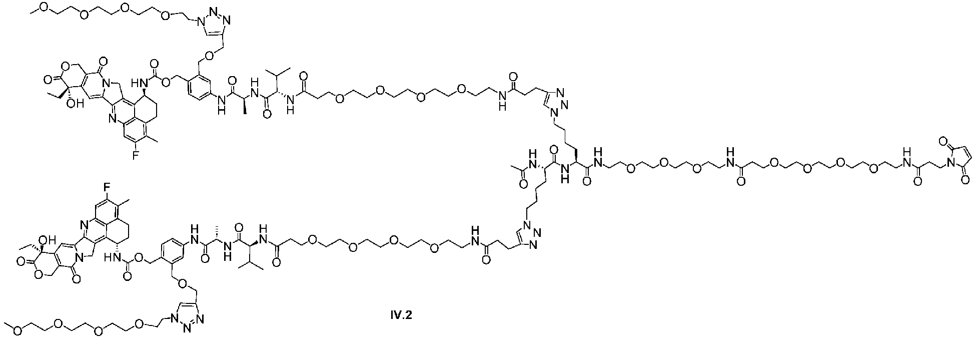

Compound **IV.2** was synthesized as described for compound IV.1 but using **intermediate 3.2.** Compound **IV.2** (30.76 mg) was obtained as yellowish solid. C₁₇₈H₂₅₀F₂N₃₂O₅₂. HPLC (method J): *t*_{R} = 16.4 min. MS (ESI): found *m*/*z* = 1236.8 [M+3H]³⁺, calcd. *m*/*z* = 1236.3 [M+3H]³⁺.

### Example 5 - Synthesis of Compound IV.3

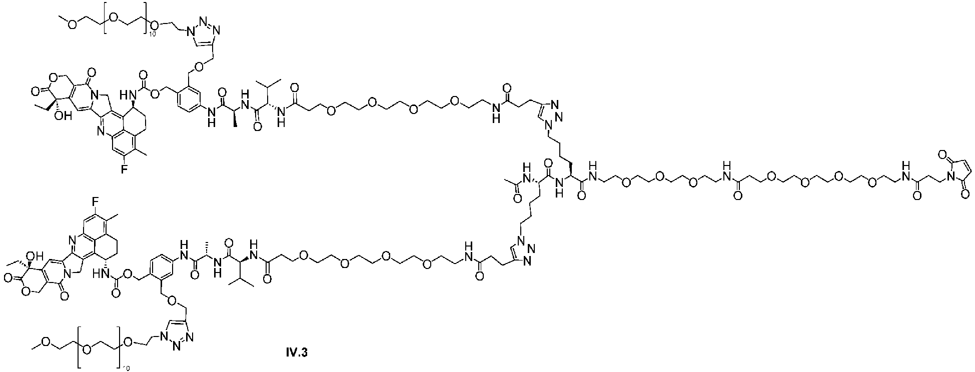

Compound **IV.3** was synthesized as described for compound **IV.1** but using m-PEG(12)-azide instead of m-PEG(4)-azide and **intermediate 3.2** instead of intermediate **3.1.** Compound **IV.3** (5.07 mg) was obtained as yellowish solid. C₂₁₀H₃₁₄F₂N₃₂O₆₈. HPLC (method J): *t*_{R} = 16.4 min. MS (ESI): found *m*/*z =* 1471.7 [M+3H]³⁺, calcd. *m*/*z* = 1471.1 [M+3H]³⁺.

### Example 6 - Synthesis of Compound IV.4

### Synthesis of I.4

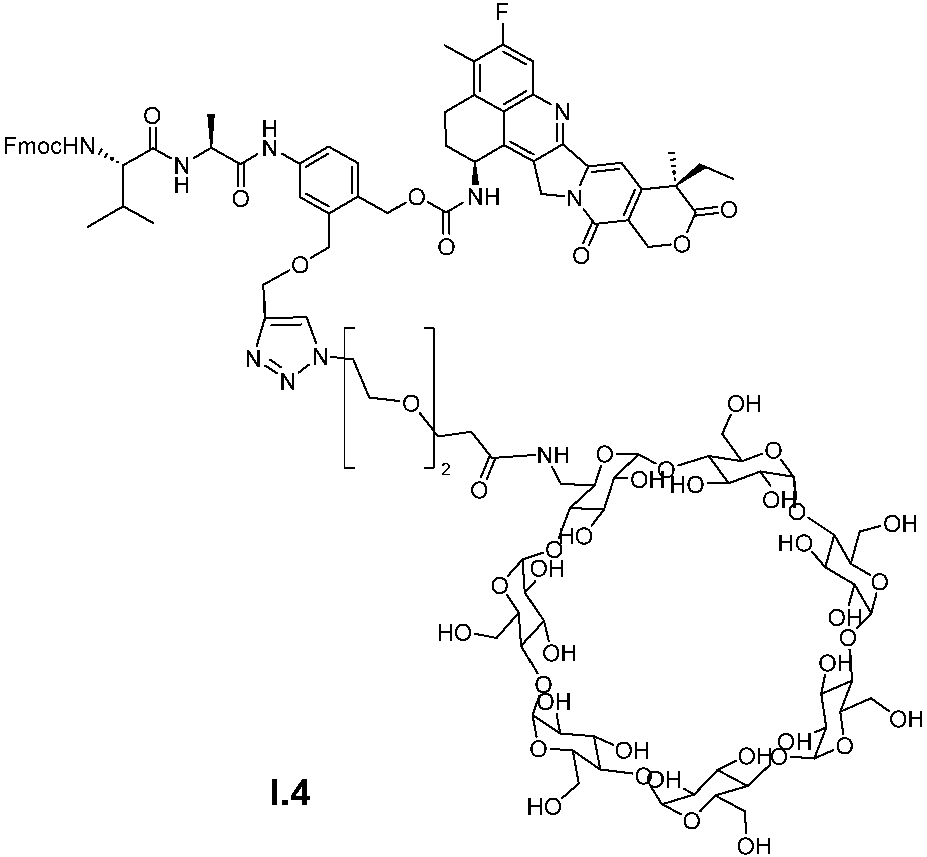

Intermediate **I.4** was synthesized according to GP1 using intermediate **4.1** (22.4 µmol, 1 equiv.), intermediate **1.3** (26.16 mg, 25.0 µmol, 1.1 equiv.), copper(II) sulfate pentahydrate (1.23 mg, 4.9 µmol, 0.2 equiv.), tris(benzyltriazolylmethyl)amine (3.17 mg, 6.0 µmol, 0.26 equiv.) and sodium ascorbate (4.33 mg, 11.9 µmol, 0.5 equiv.) in DMF/water (0.28 mL, 10:1). Purification by RP-HPLC afforded intermediate **I.4** (31.51 mg, 60%) as yellowish solid. C₁₀₈H₁₃₉FN₁₀O₄₈. HPLC (method D): *t*_{R} = 10.7 min. MS (ESI): found *mlz =* 1182.5 [M+2H]²⁺, calcd. *mlz* = 1182.4 [M+2H]²⁺.

### Synthesis of II.4

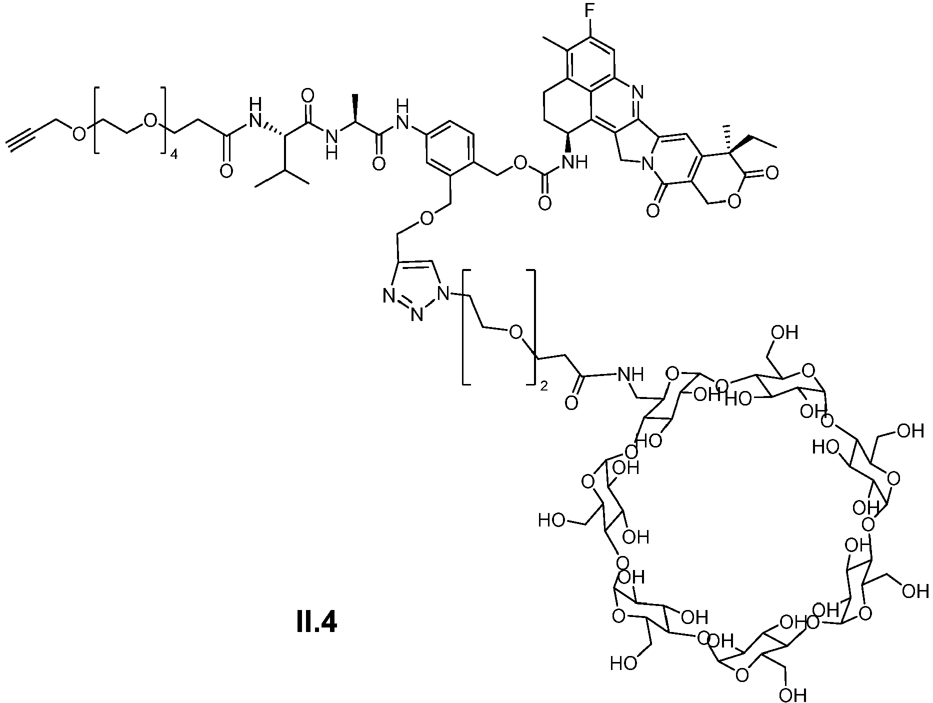

Intermediate **II.4** was synthesized according to GP3 using intermediate **I.4** (31.51 mg, 13.3 µmol, 1 equiv.), 33 µL diethylamine (319.9 µmol, 24 equiv.) in 1.6 mL DMF for Fmoc-deprotection and **intermediate 3.1** (8.98 mg, 20.0 µmol, 1.5 equiv.), diisopropylethylamine (6.8 µL, 40.0 µmol, 3 equiv.) in DMF (2.1 mL) for the coupling reaction. Purification by RP-HPLC afforded intermediate **II.4** (15.51 mg, 50%) as yellowish solid. C₁₀₇H₁₅₁FN₁₀O₅₂. HPLC (method D): *t*_{R} = 9.1 min. MS (ESI): found *m*/*z =* 1214.5 [M+2H]²⁺, calcd. *m*/*z* = 1214.5 [M+2H]²⁺.

### Synthesis of III.4

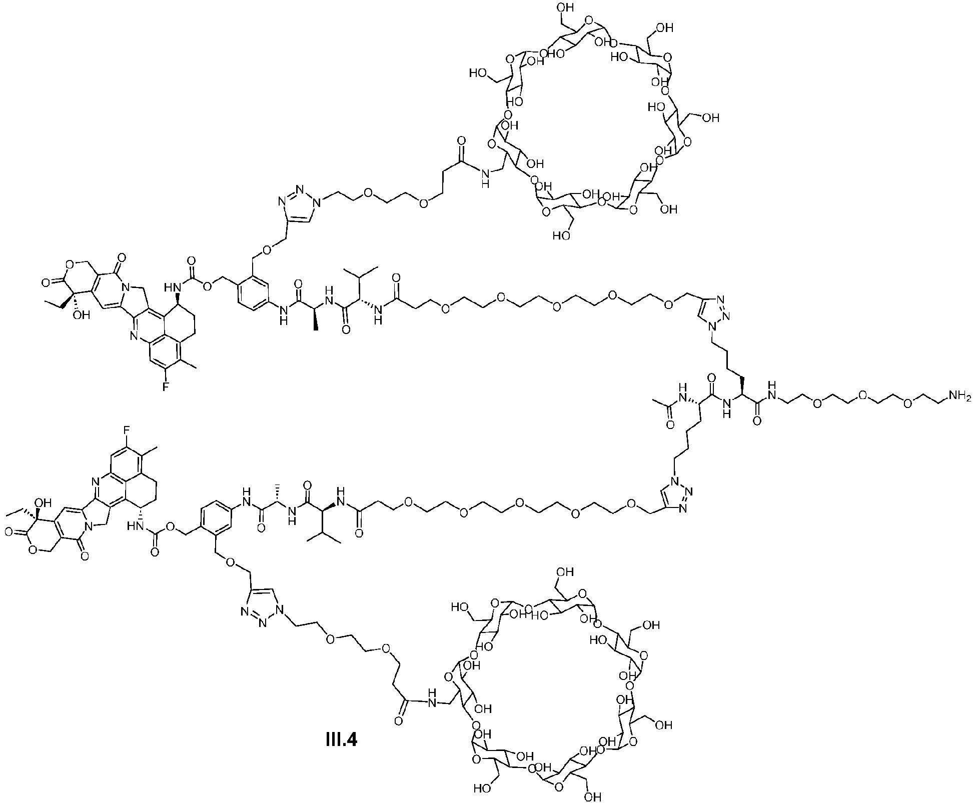

Intermediate **III.4** was synthesized according to GP2 using **intermediate 2.1** (1.54 mg, 2.6 µmol, 1 equiv.), intermediate **II.4** (15.51 mg, 6.4 µmol, 2.5 equiv), copper(II) sulfate pentahydrate (0.87 mg, 3.5 µmol, 1.3 equiv.), Tris(3-hydroxypropyltriazolylmethyl)amine (2.83 mg, 6.5 µmol, 2.5 equiv.) and sodium ascorbate (1.97 mg, 9.9 µmol, 3.8 equiv.) in DMF/water (0.12 mL, 5:1). Purification by RP-HPLC afforded intermediate **III.4** (9.61 mg, 70%) as yellowish solid. C₂₃₆H₃₄₄F₂N₃₀O₁₁₀. HPLC (method E): *t*_{R} = 9.2 min. MS (ESI): found *m*/*z =* 1800.6 [M+3H]³⁺, calcd. *m*/*z* = 1799.7 [M+3H]³⁺.

### Synthesis of IV.4

Compound **IV.4** was synthesized according to GP4 using the TFA-salt of intermediate **III.4** (9.61 mg, 1.74 µmol, 1 equiv.), *N*-[15-[(2,5-Dioxo-1-pyrrolidinyl)oxy]-15-oxo-3,6,9,12-tetraoxapentadec-1-yl]-2,5-dihydro-2,5-dioxo-1*H*-pyrrole-1-propanamide = Mal-PEG4-NHS-ester (1.89 mg, 3.68 µmol, 2.1 equiv.) and DIPEA (0.59 µL, 3.48 µmol, 2 equiv.) in DMF (0.4 mL). Purification by RP-HPLC afforded compound **IV.4** (6.95 mg, 69 %) as yellowish solid. C₂₅₄H₃₇₀F₂N₃₂O₁₁₈. HPLC (method J): *t*_{R} = 12.9 min. MS (ESI): found *m*/*z =* 1455.1 [M+3H+Na]⁴⁺, calcd. *m*/*z* = 1455.7 [M+3H+Na]⁴⁺.

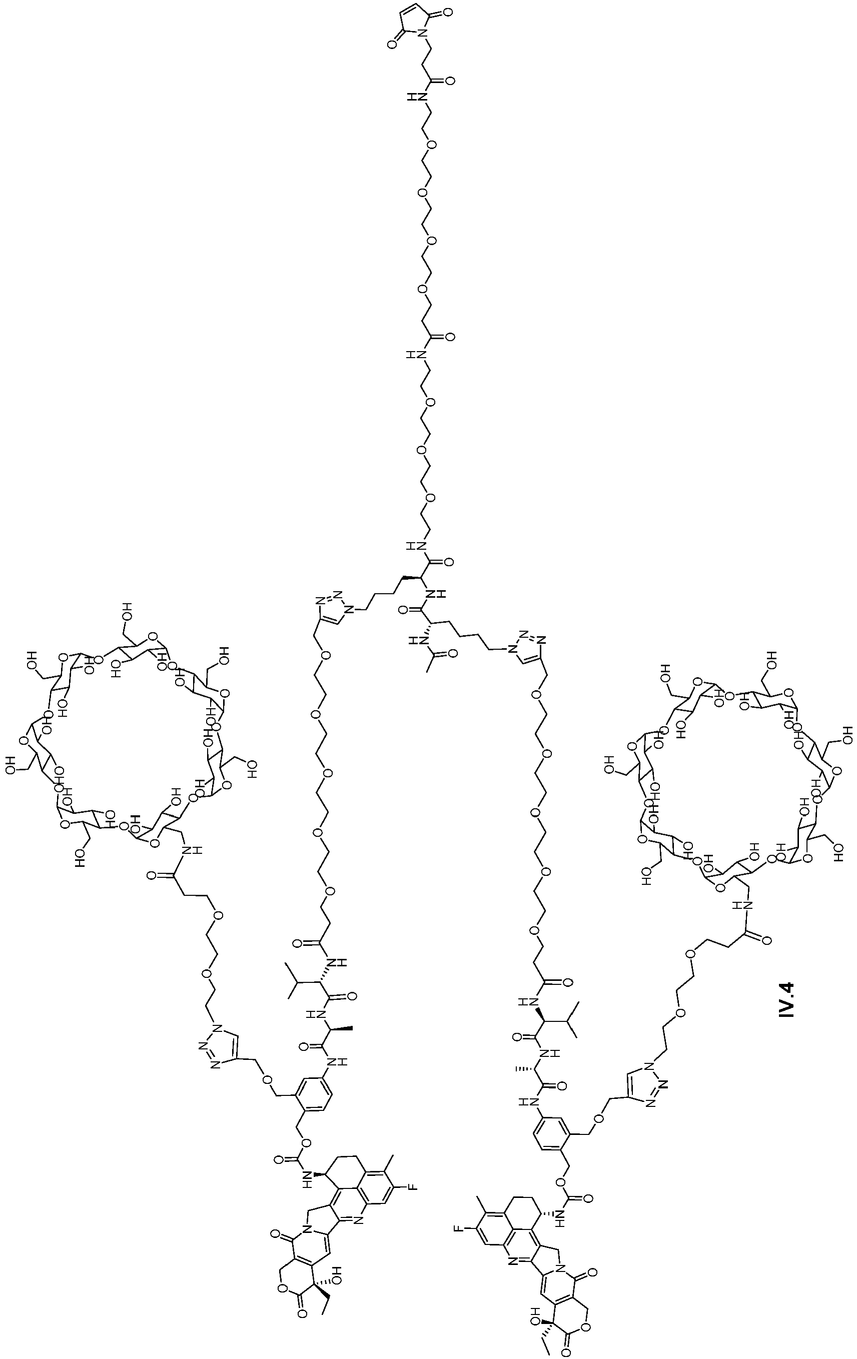

### Example 7 - Synthesis of Compound IV.5

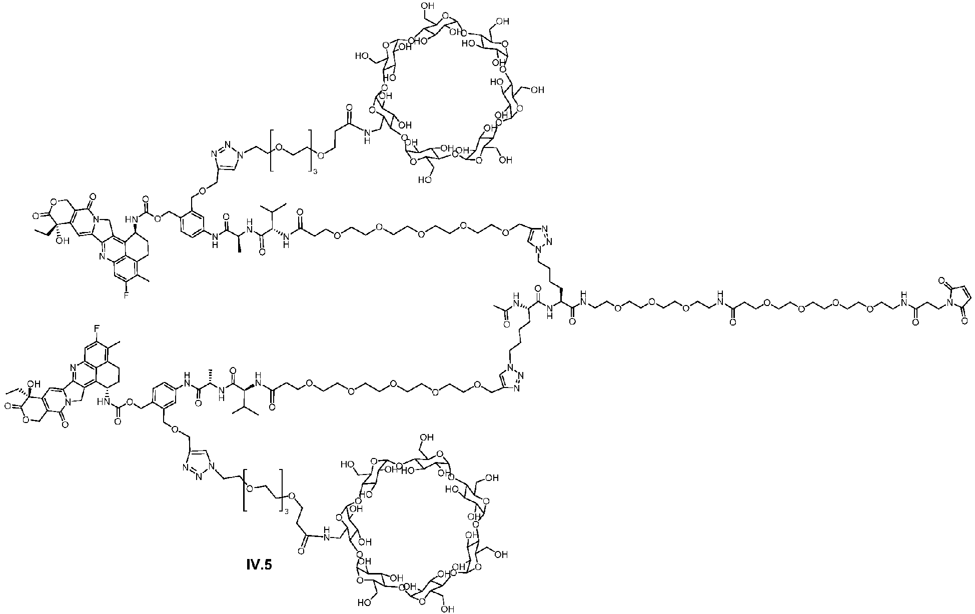

Compound **IV.5** was synthesized as described for compound **IV.4** but using **intermediate** 4.2. Compound **IV.5** (25.55 mg, 68 %) was obtained as yellowish solid. C₂₆₂H₃₈₆F₂N₃₂O₁₂₂. HPLC (method J): *t*_{R} = 13.1 min. MS (ESI): found *m*/*z* = 1494.3 [M+4H]⁴⁺, calcd. *m*/*z* = 1493.6 [M+xH]^{x+}.

### Example 8 - Synthesis of Compound IV.6

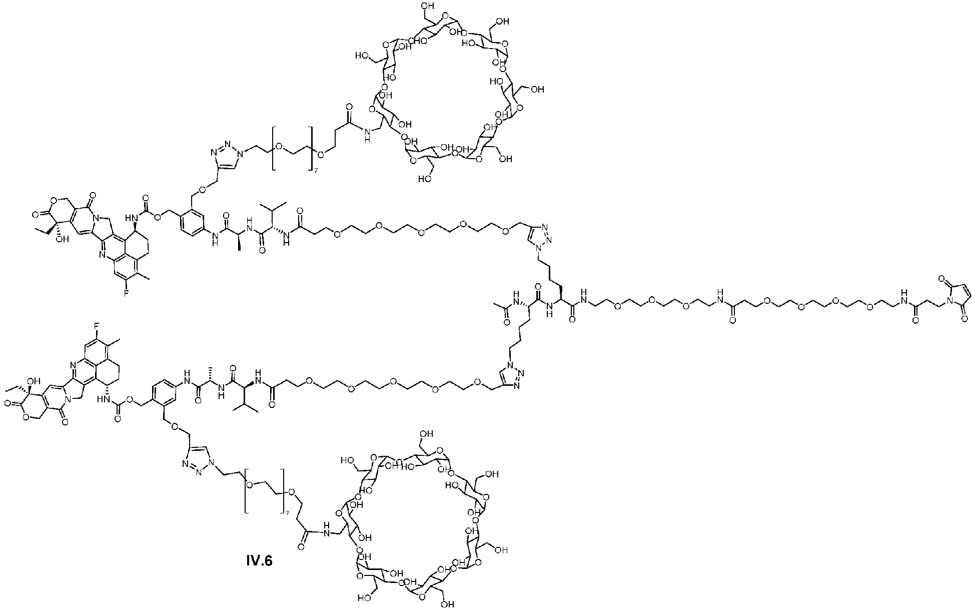

Compound **IV.6** was synthesized as described for compound **IV.4** but using **intermediate** 4.3. Compound **IV.6** (6.70 mg, 83 %) was obtained as yellowish solid. C₂₇₈H₄₁₈F₂N₃₂O₁₃₀. HPLC (method J): *t*_{R} = 13.3 min. MS (ESI): found *m*/*z* = 1582.3 [M+4H]⁴⁺, calcd. *m*/*z* = 1581.7 [M+4H]⁴⁺.

### Example 9 - Synthesis of Compound IV.7

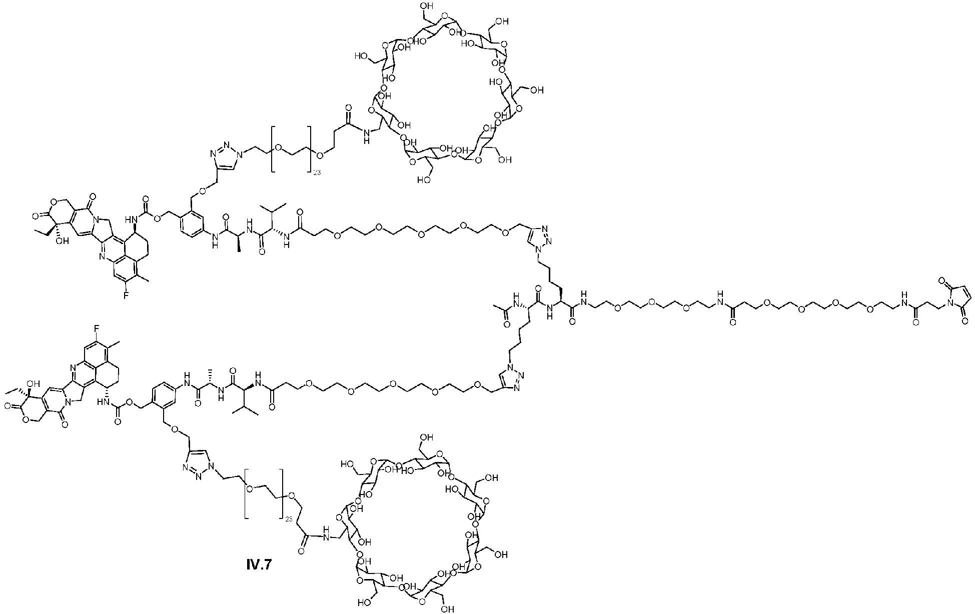

Compound **IV.7** was synthesized as described for compound **IV.4** but using **intermediate 4.4.** Compound **IV.7** (10.41 mg, 51 %) was obtained as yellowish solid. C₃₄₂H₅₄₆F₂N₃₂O₁₆₂. HPLC (method J): *t*_{R} = 14.0 min. MS (ESI): found *m*/*z* = 1934.3 [M+4H]⁴⁺, calcd. *m*/*z* = 1933.9 [M+4H]⁴⁺.

### Example 10 - Synthesis of Compound IV.8

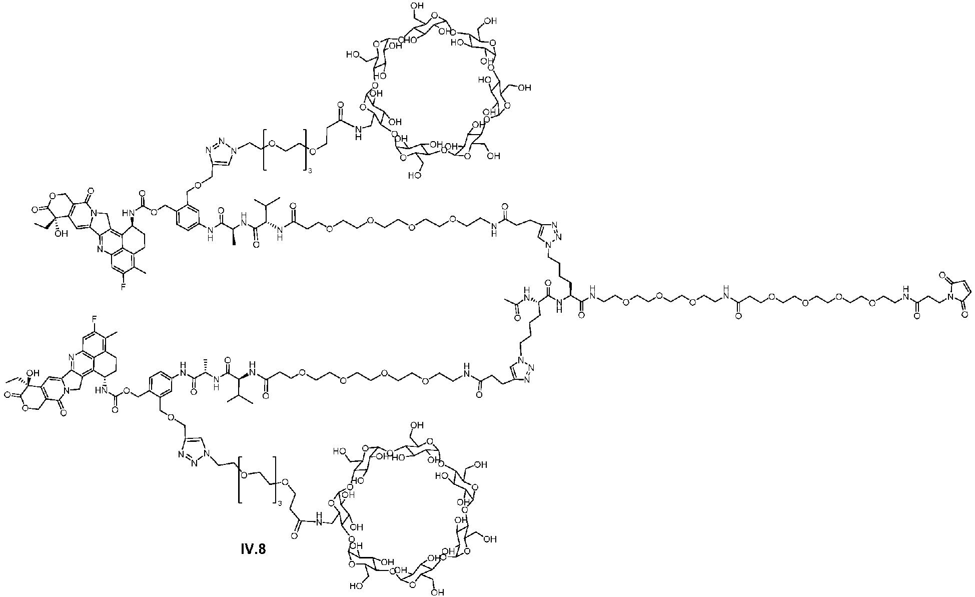

Compound **IV.8** was synthesized as described for compound **IV.4** but using **intermediates 3.2** and **4.2.** Compound **IV.8** (19.3 mg, 35 %) was obtained as yellowish solid. C₂₆₆H₃₉₂F₂N₃₄O₁₂₂. HPLC (method J): *t*_{R} = 12.9 min. MS (ESI): found *m*/*z* = 1514.6 [M+4H]⁴⁺, calcd. *m*/*z* = 1514.1 [M+4H]⁴⁺.

### Example 11 - Synthesis of Compound IV.9

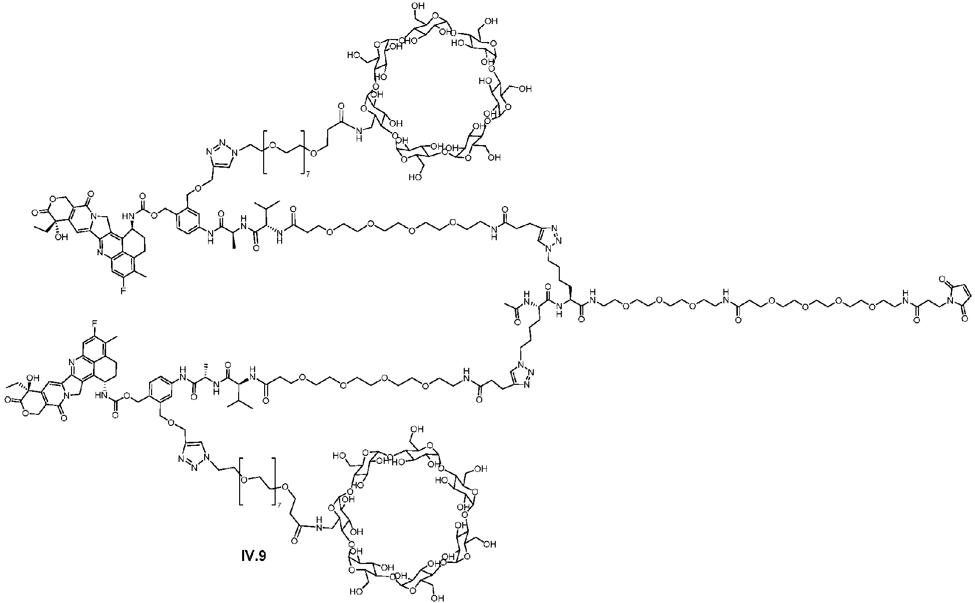

Compound **IV.9** was synthesized as described for compound **IV.4** but using **intermediates 3.2** and **4.3.** Compound **IV.9** (8.87 mg, 49 %) was obtained as yellowish solid. C₂₈₂H₄₂₄F₂N₃₄O₁₃₀. HPLC (method J): *t*_{R} = 13.2 min. MS (ESI): found *m*/*z* = 1602.7 [M+4H]⁴⁺, calcd. *m*/*z* = 1602.2 [M+4H]⁴⁺.

### Example 12 - Synthesis of Compound IV.10

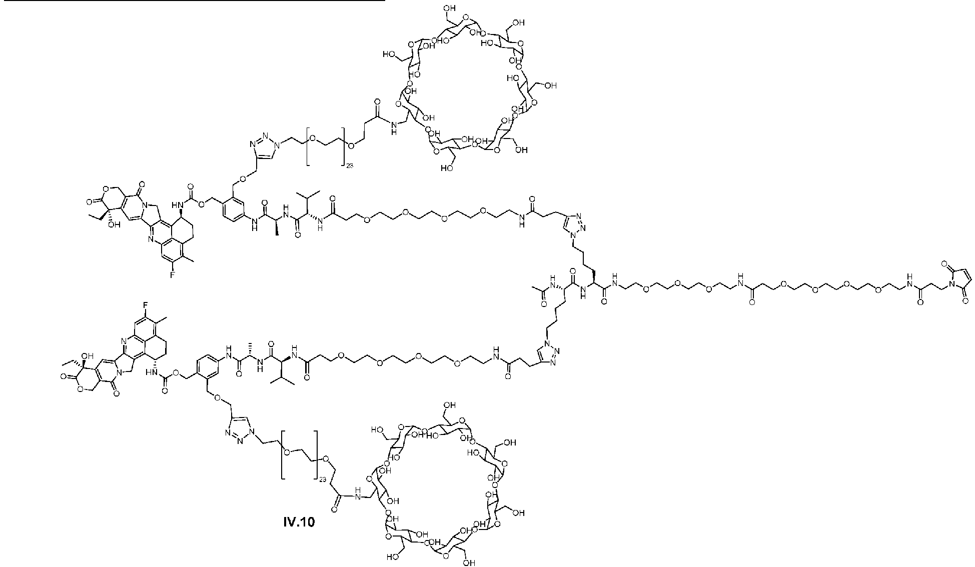

Compound **IV.10** was synthesized as described for compound **IV.4** but using **intermediates 3.2** and **4.4.** Compound **IV.10** (12.26 mg, 49 %) was obtained as yellowish solid. C₃₄₆H₅₅₂F₂N₃₄O₁₆₂. HPLC (method J): *t*_{R} = 13.9 min. MS (ESI): found *m*/*z =* 1304.3 [M+6H]⁶⁺, calcd. *m*/*z =* 1303.3 [M+6H]⁶⁺.

### Example 13 - Synthesis of Compound IV.11

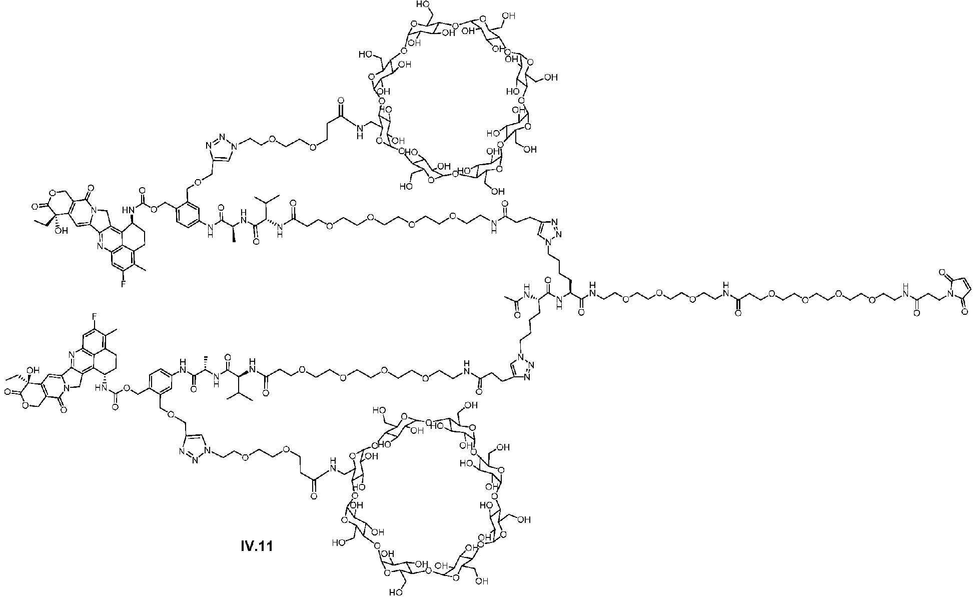

Compound **IV.11** was synthesized as described for compound **IV.4** but using **intermediates 3.2** and **4.5.** Compound **IV.11** (5.69 mg, 56 %) was obtained as yellowish solid. C₂₇₀H₃₉₆F₂N₃₄O₁₂₈. HPLC (method J): *t*_{R} = 12.6 min. MS (ESI): found *m*/*z* = 1551.8 [M+4H]⁴⁺, calcd. *m*/*z* = 1551.1 [M+4H]⁴⁺.

### Example 14 - Synthesis of Compound IV.12

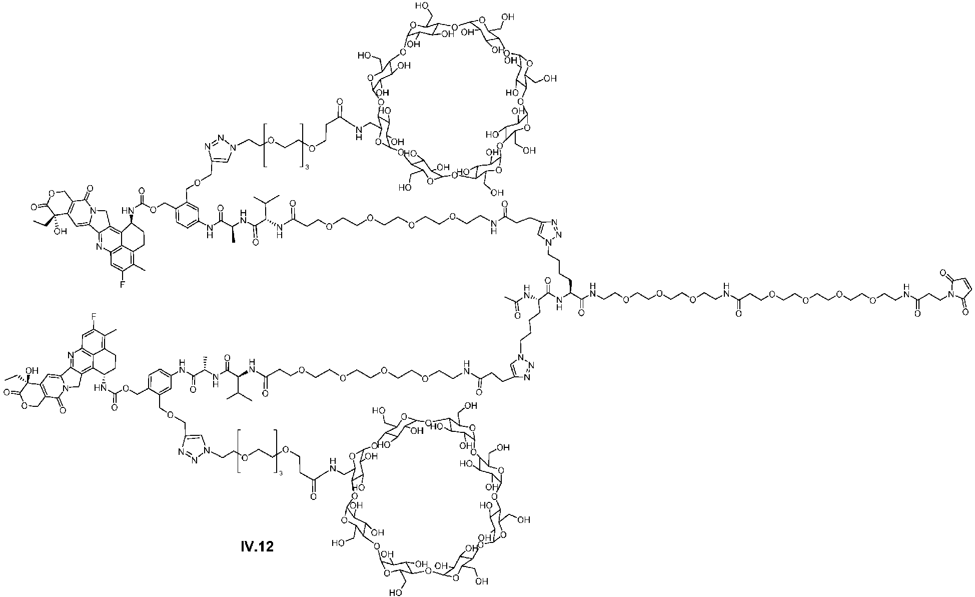

Compound **IV.12** was synthesized as described for compound **IV.4** but using **intermediates 3.2** and **4.6.** Compound **IV.12** (43.17 mg, 59 %) was obtained as yellowish solid. C₂₇₈H₄₁₂F₂N₃₄O₁₃₂. HPLC (method J): *t*_{R} = 12.4 min. MS (ESI): found *m*/*z* = 1596.8 [M+4H]⁴⁺, calcd. *m*/*z* = 1595.67 [M+4H]⁴⁺.

### Example 15 - Synthesis of Compound IV.13

Compound **IV.13** was synthesized as described for compound **IV.4** but using **intermediates 3.2** and **4.7.** Compound **IV.13** (29.51 mg, 59 %) was obtained as yellowish solid. C₂₉₄H₄₄₄F₂N₃₄O₁₄₀. HPLC (method J): *t*_{R} = 12.7 min. MS (ESI): found *m*/*z =* 1684.5 [M+4H]⁴⁺, calcd. *m*/*z =* 1683.2 [M+4H]⁴⁺.

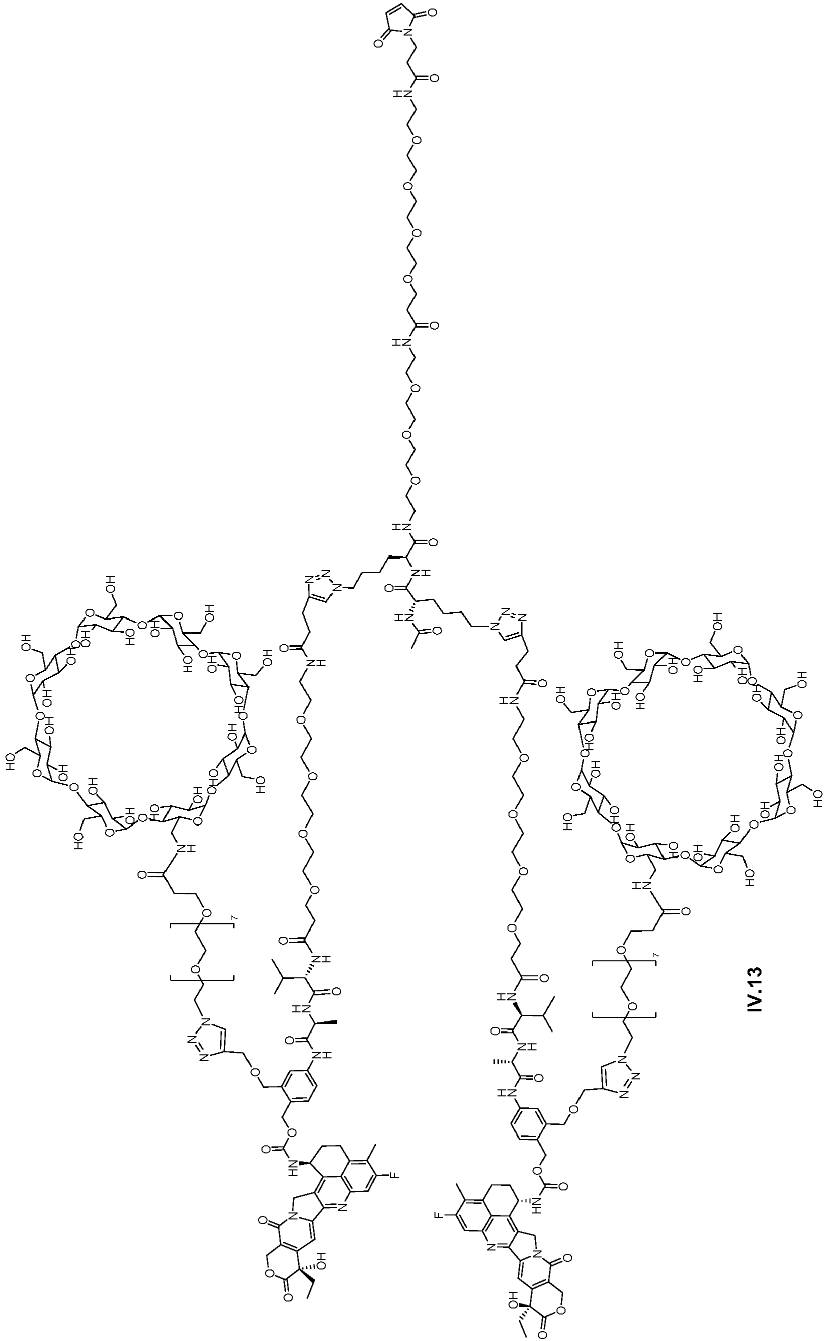

### Example 16 - Synthesis of Compound IV.14

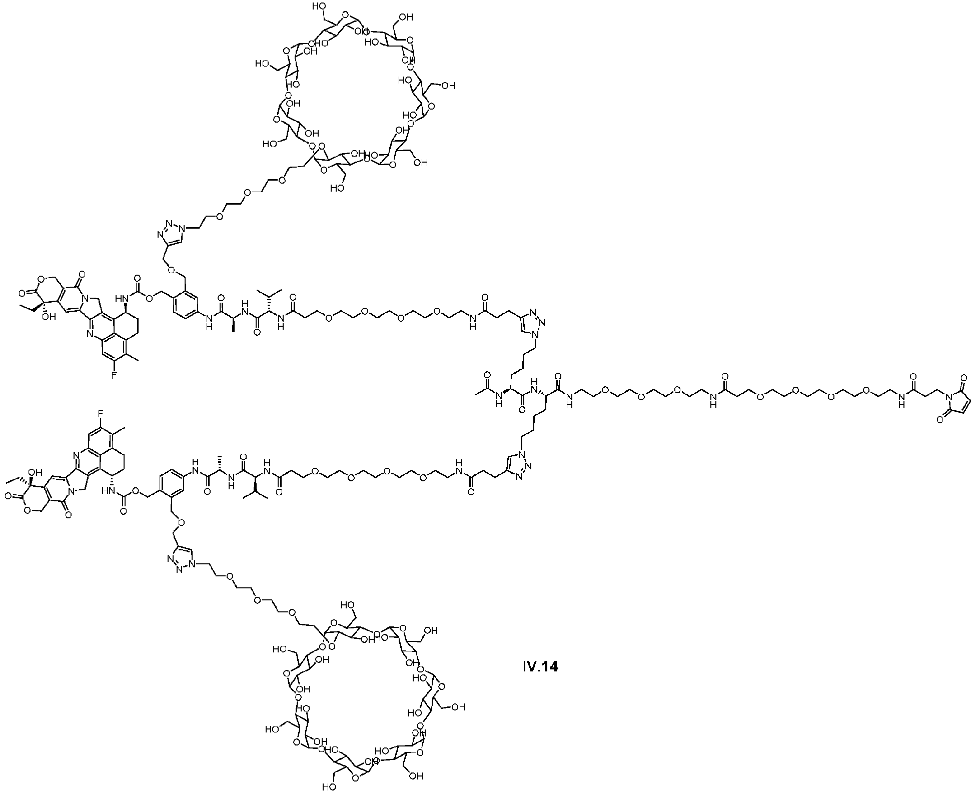

Compound **IV.14** was synthesized as described for compound **IV.4** but using **intermediates 3.2** and **4.8.** Compound **IV.14** (61.21 mg, 81 %) was obtained as yellowish solid. C₂₆₀H₃₈₂F₂N₃₂O₁₂₀. HPLC (method J): *t*_{R} = 12.7 min. MS (ESI): found *m*/*z =* 1479.8 [M+4H]⁴⁺, calcd. *m*/*z =* 1478.6 [M+4H]⁴⁺.

### Example 17 - Synthesis of Compound IV.15

Compound **IV.15** was synthesized as described for compound **IV.4** but using **intermediates 3.2** and **4.9.** Compound **IV.15** (29.68 mg, 68 %) was obtained as yellowish solid. C₂₇₂H₄₀₂F₂N₃₂O₁₃₀. HPLC (method J): *t*_{R} = 12.5 min. MS (ESI): found *m*/*z =* 1560.1 [M+4H]⁴⁺, calcd. *m*/*z* = 1559.7 [M+4H]⁴⁺.

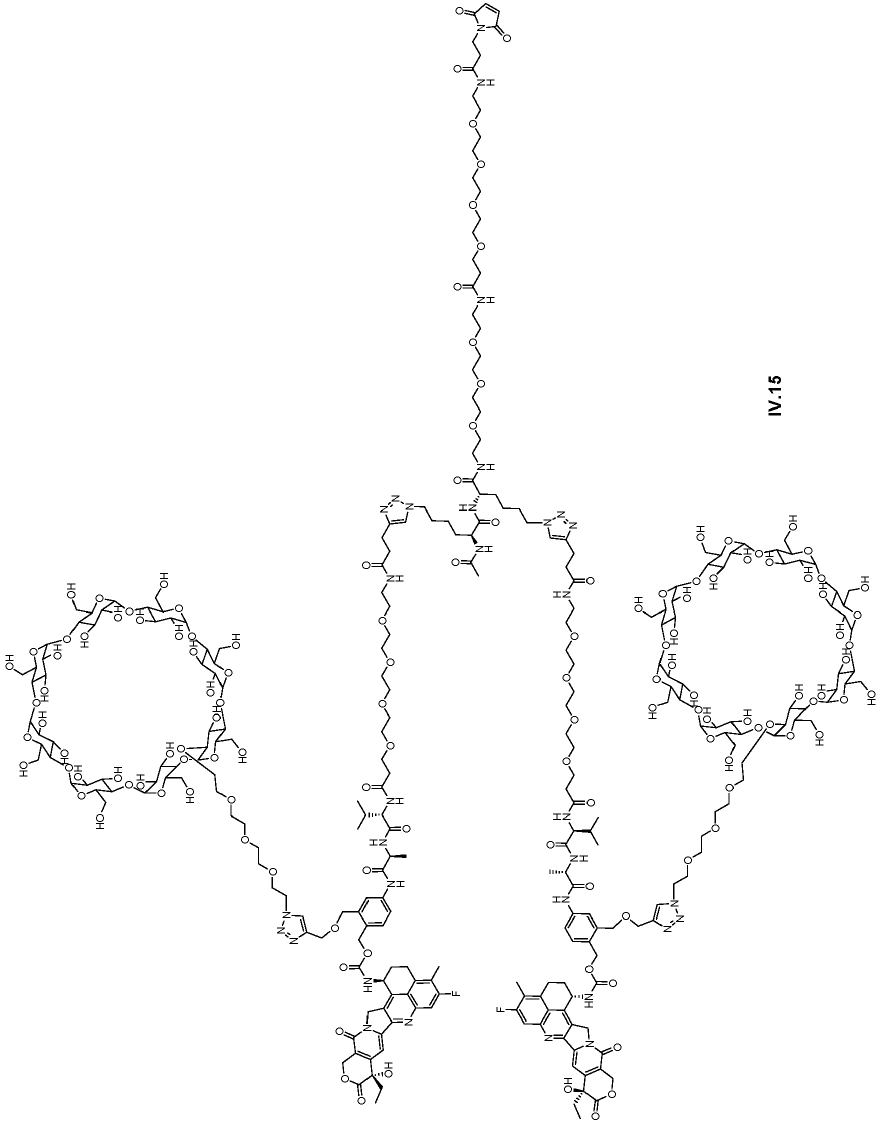

### Example 18 - Synthesis of Compound IV.16

### Synthesis of I.16

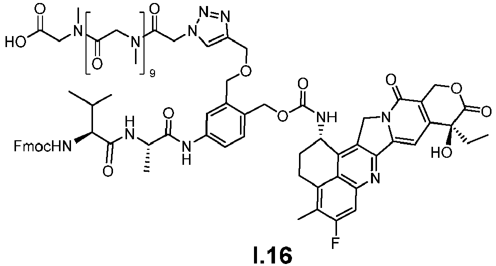

Intermediate **I.16** was synthesized according to GP1 using intermediate **5.1** (30 mg, 37.0 µmol, 1 equiv.), intermediate **1.3** (42.5 mg, 40.7 µmol, 1.1 equiv.), copper(II) sulfate pentahydrate (1.85 mg, 7.4 µmol, 0.2 equiv.), tris(benzyltriazolylmethyl)amine (4.91 mg, 9.25 µmol, 0.25 equiv.) and sodium ascorbate (3.66 mg, 18.5 µmol, 0.5 equiv.) in DMF/water (0.33 mL, 10:1). Purification by RP-HPLC afforded intermediate **I.16** (51.7 mg, 74 %) as yellowish solid. C₉₁H₁₁₀FN₁₉O₂₃. HPLC (method A): *t*_{R} = 11.1 min. MS (ESI): found *mlz* = 1855.1 [M-H]⁻, calcd. *mlz* = 1854.8 [M-H]⁻.

### Synthesis of II.16

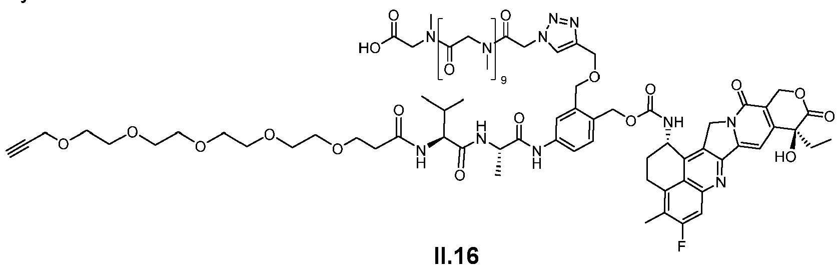

Intermediate **II.16** was synthesized according to GP3 using intermediate **I.16** (51.7 mg, 27.8 µmol, 1 equiv.), diethylamine (68.7 µL, 667.0 µmol, 24 equiv.) in 2.5 mL DMF for Fmoc-deprotection and **intermediate 3.1** (18.7 mg, 41.7 µmol, 1.5 equiv.), diisopropylethylamine (14.2 µL, 83.4 µmol, 3 equiv.) in DMF (3.44 mL) for the coupling reaction. Purification by RP-HPLC afforded intermediate **II.16** (43.8 mg, 82 %) as yellowish solid. C₉₀H₁₂₂FN₁₉O₂₇. HPLC (method A): *t*_{R} = 9.4 min. MS (ESI): found *m*/*z* = 961.6 [M+2H]²⁺, calcd. *m*/*z* = 960.9 [M+2H]²⁺.

### Synthesis of III.16

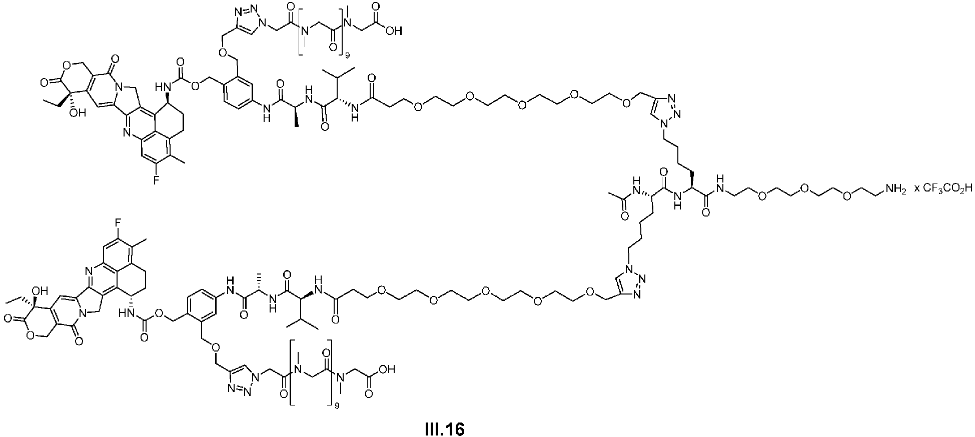

Intermediate **III.16** was synthesized according to GP2 using **intermediate 2.1** (2.67 mg, 4.6 µmol, 1 equiv.), intermediate **II.16** (20.4 mg, 10.6 µmol, 2.3 equiv), copper(II) sulfate pentahydrate (1.44 mg, 5.8 µmol, 1.25 equiv.), Tris(3-hydroxypropyltriazolylmethyl)amine (5.02 mg, 11.6 µmol, 2.5 equiv.) and sodium ascorbate (3.43 mg, 17.3 µmol, 3.8 equiv.) in DMF/water (0.44 mL, 10:1). Purification by RP-HPLC afforded intermediate **III.16** (9.2 mg, 46 %) as yellowish solid. C₂₀₂H₂₈₆F₂N₄₈O₆₀. HPLC (method L): *t*_{R} = 16.8 min. MS (ESI): found *m*/*z =* 1462.5 [M+3H]³⁺, calcd. *m*/*z* = 1461.7 [M+3H]³⁺.

### Synthesis of IV.16

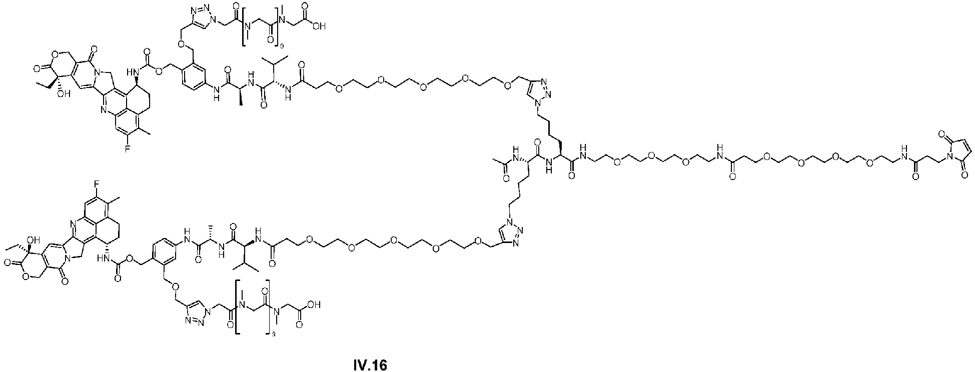

Compound **IV.16** was synthesized according to GP4 using the TFA-salt of intermediate **III.16** (7.4 mg, 1.26 µmol, 1 equiv.), *N*-[15-[(2,5-Dioxo-1-pyrrolidinyl)oxy]-15-oxo-3,6,9,12-tetraoxapentadec-1-yl]-2,5-dihydro-2,5-dioxo-1*H*-pyrrole-1-propanamide = Mal-PEG4-NHS-ester (0.72 mg, 1.39 µmol, 1.1 equiv.) and DIPEA (0.22 µL, 1.26 µmol, 1 equiv.) in DMF (0.3 mL). Purification by RP-HPLC afforded compound **IV.16** (3.85 mg, 61 %) as yellowish solid. C₂₂₀H₃₁₂F₂N₅₀O₆₈. HPLC (method J): *t*_{R} = 14.1 min. MS (ESI): found *m*/*z =* 1595.3 [M+3H]³⁺, calcd. *m*/*z* = 1594.4 [M+3H]³⁺.

### Example 19 - Synthesis of Compound IV.17

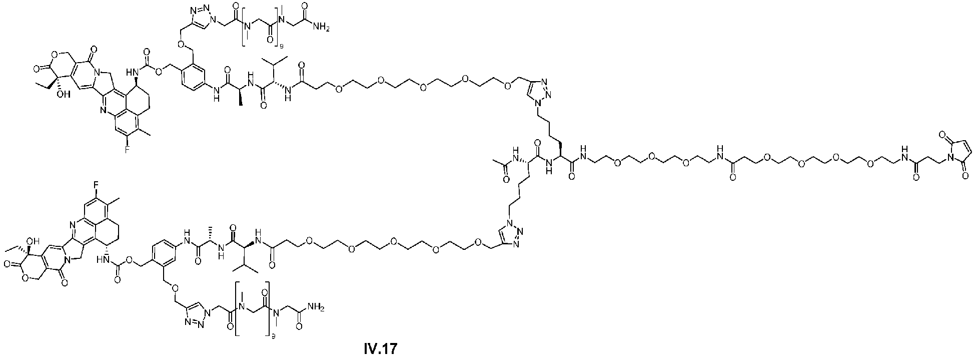

Compound **IV.17** was synthesized as described for compound **IV.16** but using intermediate **5.2.** Compound **IV.17** (23.5 mg, 75 %) was obtained as yellowish solid. C₂₂₀H₃₁₄F₂N₅₂O₆₆. HPLC (method J): *t*_{R} = 13.8 min. MS (ESI): found *m*/*z* = 1594.2 [M+3H]³⁺, calcd. *m*/*z* = 1593.8 [M+3H]³⁺.

### Example 20 - Synthesis of Compound IV.18

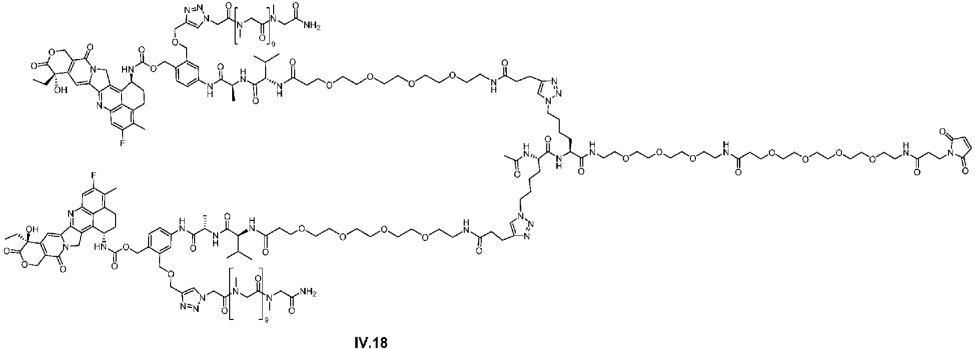

Compound **IV.18** was synthesized as described for compound **IV.16** but using **intermediates 3.2** and **5.2.** Compound **IV.18** (15.9 mg, 57 %) was obtained as yellowish solid. C₂₂₄H₃₂₀F₂N₅₄O₆₆. HPLC (method J): *t*_{R} = 12.9 min. MS (ESI): found *m*/*z* = 1621.8 [M+3H]³⁺, calcd. *m*/*z* = 1621.1 [M+3H]³⁺.

### Example 21 - Synthesis of Compound IV.19

### Synthesis of I.19

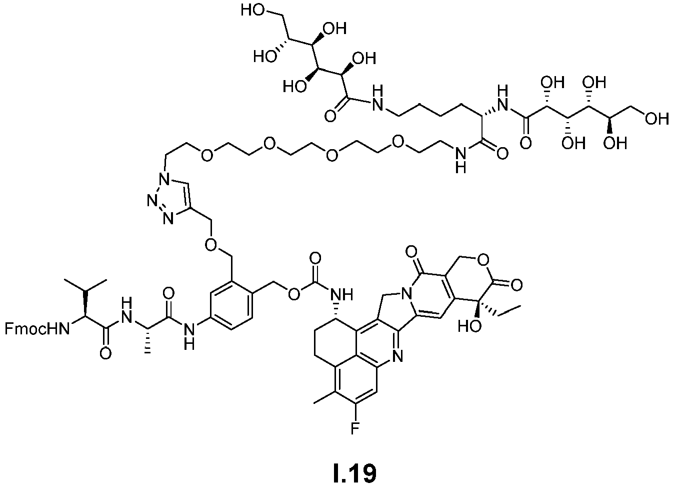

Intermediate **I.19** was synthesized according to GP1 using intermediate **6.1** (64.31 mg, 86.11 µmol, 1 equiv.), intermediate **1.3** (60.0 mg, 57.4 µmol, 1.0 equiv.), copper(II) sulfate pentahydrate (14.3 mg, 57.4 µmol, 1 equiv.), THPTA (50.08 mg, 115.2 µmol, 2 equiv.) and sodium ascorbate (34.38 mg, 173.5 µmol, 3 equiv.) in DMF/water (1.72 mL, 2:1). Purification by RP-HPLC afforded intermediate **I.17** (76.18 mg, 74 %) as yellowish solid. C₈₇H₁₁₁FN₁₂O₂₈. HPLC (method B): *t*_{R} = 16.8 min. MS (ESI): found *m*/*z* = 896.7 [M+2H]²⁺, calcd. *m*/*z* = 896.4 [M+2H]²⁺.

### Synthesis of II.19

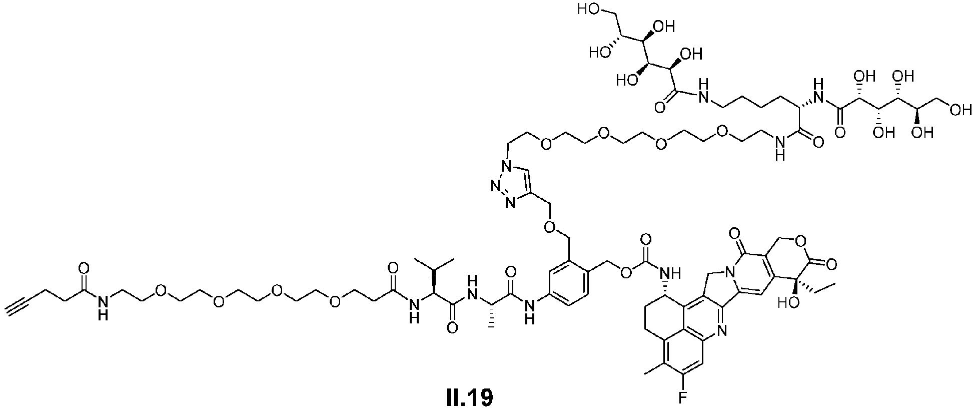

Intermediate **II.19** was synthesized according to GP3 using intermediate **I.19** (76.18 mg, 42.51 µmol, 1 equiv.), diethylamine (105 µL, 1020 µmol, 24 equiv.) in 5 mL DMF (abs.) for Fmoc-deprotection and **intermediate 3.2** (31.28 mg, 63.8 µmol, 1.5 equiv.), diisopropylethylamine (21.7 µL, 127.5 µmol, 3 equiv.) in DMF (1 mL) for the coupling reaction. Purification by RP-HPLC afforded intermediate **II.19** (38.97 mg, 48 %) as yellowish solid. C₈₈H₁₂₆FN₁₃O₃₂. HPLC (method N): *t*_{R} = 16.1 min. MS (ESI): found *m*/*z* = 949.6 [M+2H]²⁺, calcd. *m*/*z* = 948.9 [M+2H]²⁺.

### Synthesis of III.19

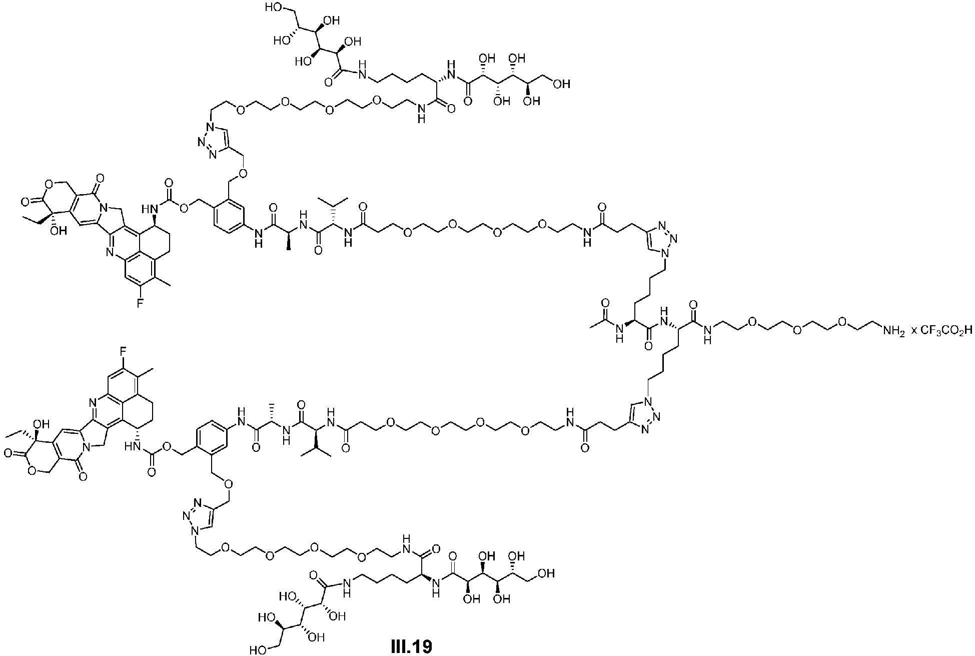

Intermediate **III.19** was synthesized according to GP2 using **intermediate 2.1** (4.96 mg, 8.6 µmol, 1 equiv.), intermediate **II.19** (38.97 mg, 20.5 µmol, 2.4 equiv), copper(II) sulfate pentahydrate (2.57 mg, 10.3 µmol, 1.2 equiv.), Tris(3-hydroxypropyltriazolylmethyl)amine (8.95 mg, 20.6 µmol, 2.4 equiv.) and sodium ascorbate (6.10 mg, 30.8 µmol, 3.6 equiv.) in DMF/water (1.13 mL, 10:1). After 3 h, the same catalyst amount was added again and the solution stirred for additional 40 min at room temperature. Purification by RP-HPLC afforded intermediate **III.19** (29.03 mg, 76 %) as yellowish solid. C₁₉₈H₂₉₄F₂N₃₆O₇₀. HPLC (method O): *t*_{R} = 12.3 min. MS (ESI): found *m*/*z* = 1446.6 [M+3H]³⁺, calcd. *m*/*z* = 1245.7 [M+3H]³⁺.

### Synthesis of IV.19

Compound **IV.19** was synthesized according to GP4 using the TFA-salt of intermediate **III.19** (29.03 mg, 6.5 µmol, 1 equiv.), *N*-[15-[(2,5-Dioxo-1-pyrrolidinyl)oxy]-15-oxo-3,6,9,12-tetraoxapentadec-1-yl]-2,5-dihydro-2,5-dioxo-1*H*-pyrrole-1-propanamide = Mal-PEG4-NHS-ester (13.40 mg, 26.1 µmol, 4 equiv.) and DIPEA (4.43 µL, 26.1 µmol, 4 equiv.) in DMF (0.78 mL). Upon completion after 1.5 h, the product was precipitated from ice-cold MTBE acidified with TFA (6 equiv.) and purification by RP-HPLC afforded compound **IV.17** (21.48 mg, 70 %) as yellowish solid. C₂₁₆H₃₂₀F₂N₃₈O₇₈. HPLC (method J): *t*_{R} = 13.3 min. MS (ESI): found *m*/*z* = 1578.7 [M+3H]³⁺, calcd. *m*/*z* = 1578.4 [M+3H]³⁺.

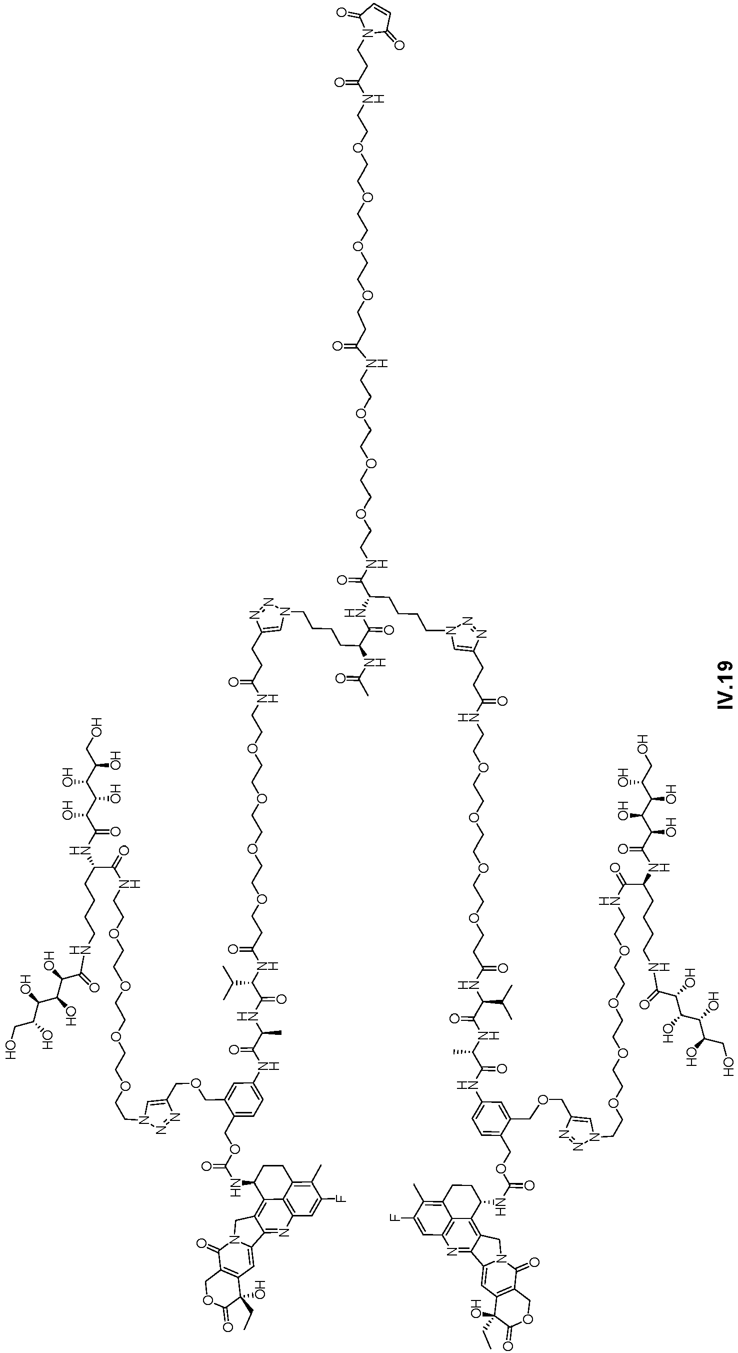

### Example 22 - Synthesis of IV.20

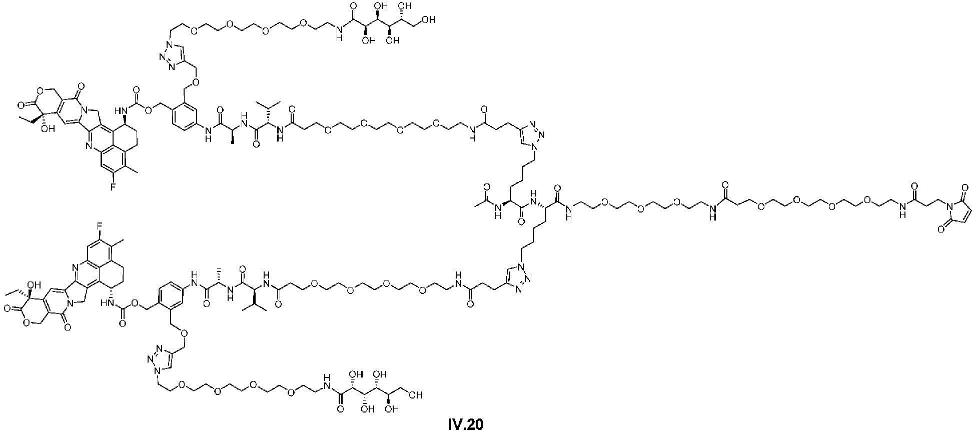

Compound **IV.20** was synthesized as described for compound **IV.19** but using **intermediate 6.2.** Compound **IV.20** (14.34 mg, 31 %) was obtained as yellowish solid. C₁₉₂H₂₇₆F₂N₃₄O₆₄. HPLC (method J): *t*_{R} = 13.5 min. MS (ESI): found *m*/*z* = 1374.9 [M+3H]³⁺, calcd. *m*/*z* = 1374.3 [M+3H]³⁺.

### Example 23 - Synthesis of IV.21

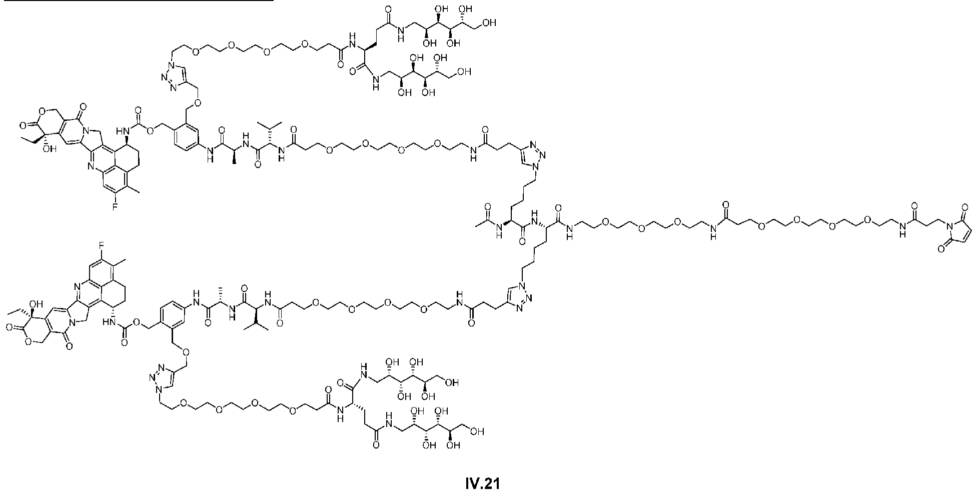

Compound **IV.21** was synthesized as described for compound **IV.19** but using **intermediate 6.3.** Compound **IV.21** (17.83 mg, 64 %) was obtained as yellowish solid. C₂₁₆H₃₂₀F₂N₃₈O₇₈. HPLC (method J): *t*_{R} = 13.1 min. MS (ESI): found *m*/*z* = 1579.1 [M+3H]³⁺, calcd. *m*/*z* = 1578.4 [M+3H]³⁺.

### Example 24 - Synthesis of IV.22

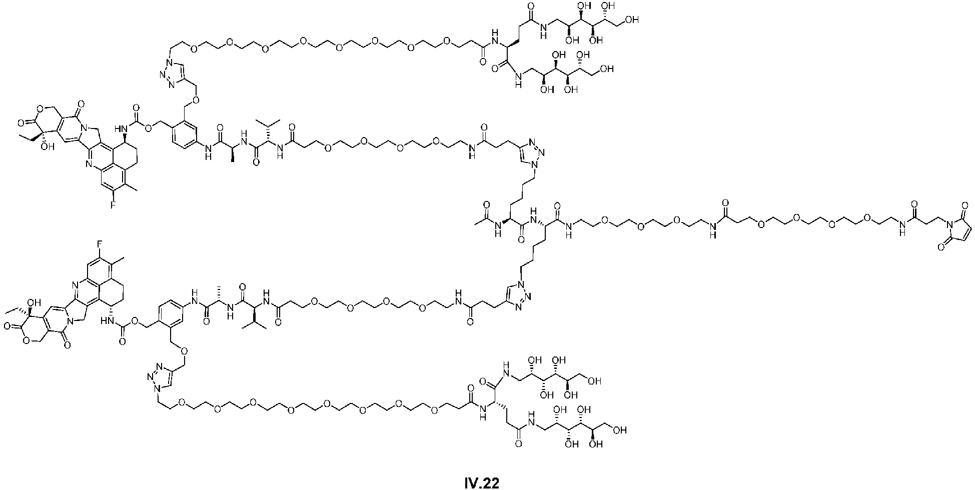

Compound **IV.22** was synthesized as described for compound **IV.19** but using **intermediate 6.4.** Compound **IV.22** (27.9 mg, 55 %) was obtained as yellowish solid. C₂₃₂H₃₅₂F₂N₃₈O₈₆. HPLC (method J): *t*_{R} = 13.4 min. MS (ESI): found *m*/*z* = 1697.0 [M+3H]³⁺, calcd. *m*/*z* = 1695.8 [M+3H]³⁺.

### Example 25 - In vitro cytotoxicity assays using 2D and 3D cell cultures

Cytotoxicity of anti-GUCY2C ADCs with modular linker compounds of the invention was assessed by using *in vitro* cytotoxicity assay with target-positive HEK293-GUCY2C and target-negative HEKwt cells based on 2D and 3D cell culture.

At the start of the 2D cytotoxicity assay, 2×10³ target-positive or target-negative cells were seeded per well in 96 Well plates and were incubated at 37°C and 5% CO2. Indicated ADCs were added 24h later at various concentrations ranging from 1×10⁻⁶ M to 1.28×10⁻¹² M to the adherent cells before the plates were incubated for further 4 days at 37°C and 5% CO₂. Cytotoxicity was assessed by using Cell Proliferation ELISA, BrdU (colorimetric) of Roche.

At the start of the 3D cytotoxicity assay, 2×10³ target-positive or target-negative cells were seeded per well in ultra-low adhesion (ULA) 96-Well plates and were incubated at 37°C and 5% CO₂. Spheroids were treated after 48h with the indicated ADCs at various concentrations ranging from 1×10⁻⁶ M to 1.28×10⁻¹² M before the plates were incubated for further 5 days at 37°C and 5% CO₂. Cytotoxicity was assessed by using CellTiter-Glo^{®} 2.0 Cell Viability Assay of Promega.

Anti-GCC ADCs that were used as controls comprised the linker-payloads (IV.11), (IV.17) and (IV.19) which do not form part of the present invention.

### Example 26 - Study design in vivo animal models

Female NOD SCID mice were inoculated subcutaneously with 5 × 10⁶ GCC-overexpressing human embryonic kidney HEK293-GUCY2C-(HDP)-2B3 cells in 200 µL RPMI medium containing 50% Matrigel without phenol red per animal into their right flanks. Once a mean tumor volume of approximately 150 mm³ was reached, animals were allocated to control or treatment groups according to tumor size. On the same day (day 0) or the day after (day 1), the animals were treated with either a single intravenous dose of vehicle control or anti-GCC ADC conjugated to the respective modular linker compound of the invention, indicated as ("IV.x"), or once a week for three weeks. The tumor volume was measured twice per week by caliper and body weights were determined in parallel. Clinical signs and survival were monitored daily. The animals were sacrificed, and necropsy was performed when one or more termination criteria arose or at study termination. The indicated dosing of the respective ADCs corresponds to the dose of total ADC in mg/kg as indicated in each case.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

**1.** An antibody-drug conjugate (ADC) of Formula (I) 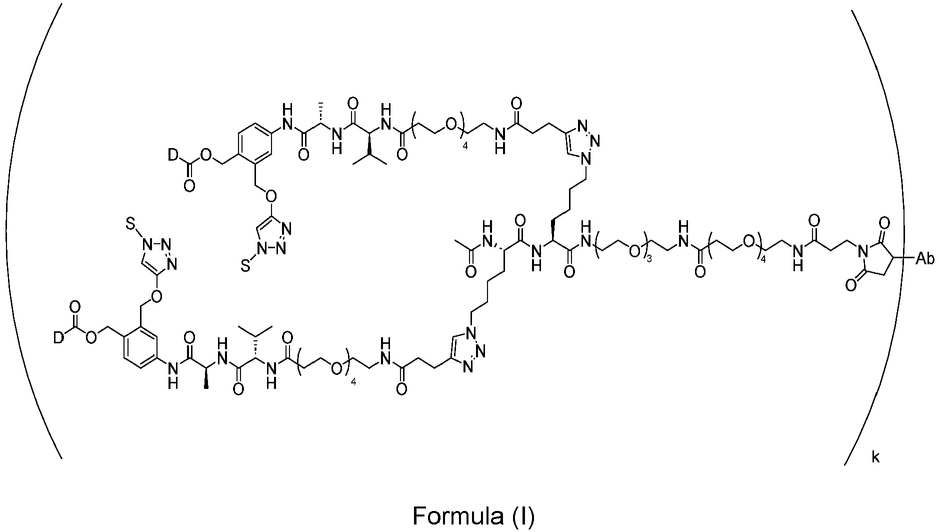 or a pharmaceutically acceptable salt or solvate thereof; wherein:
k is an integer from 2 to 4;
Ab is an antibody;
D is a pharmaceutically active substance selected from 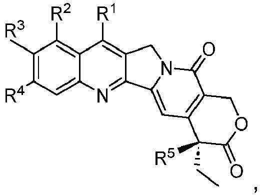 wherein:
- R² and R¹ together form 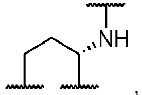 R³ is -CH₃, R⁴ is -F and R⁵ is -OH; or
- R¹ is -H, R² is -CH₂N(CH₃)₂ ,R³ -OH, R⁴ is -H and R⁵ is -O-; or
- R¹ is -CH₂CH₃, R² is -H ,R³ is 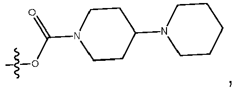 and R⁴ is -H and R⁵ is -O-; or
- R¹ is -H, R² is -NO₂ ,R³ -H, and R⁴ is -H and R⁵ is -O-; or
- R¹ is -CH₂CH₂NHCH(CH3)₂, R² is -H , R³ -H, and R⁴ is -H and R⁵ is -O-; or
- R¹ is -CH₂CH₃, R² is -H ,R³ -OH, and R⁴ is -H and R⁵ is -O-;
S is a solubility enhancing group and comprises an alpha cyclodextrin, a beta-cyclodextrin, a gamma-cyclodextrin, a polysarcosine, a PEG, or a polyalcohol made from carbohydrates.

**2.** The antibody-drug conjugate according to claim 1, wherein D is 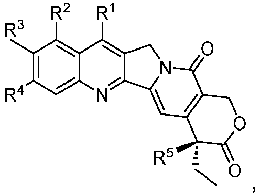 wherein:
- R² and R¹ together form 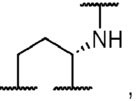 R³ is -CH₃, R⁴ is -F and R⁵ is -OH; or
- R¹ is -H, R² is -CH₂N(CH₃)₂, R³ -OH, R⁴ is -H and R⁵ is -O-; or
- R¹ is -CH₂CH₃, R² is -H ,R³ is 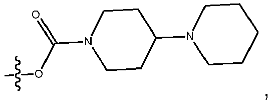 and R⁴ is - H and R⁵ is -O-.

**3.** The antibody-drug conjugate according to claim 1 or claim 2, wherein D is

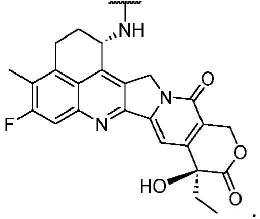

**4.** The antibody-drug conjugate according any one of claims 1 to 3, wherein S is selected from: 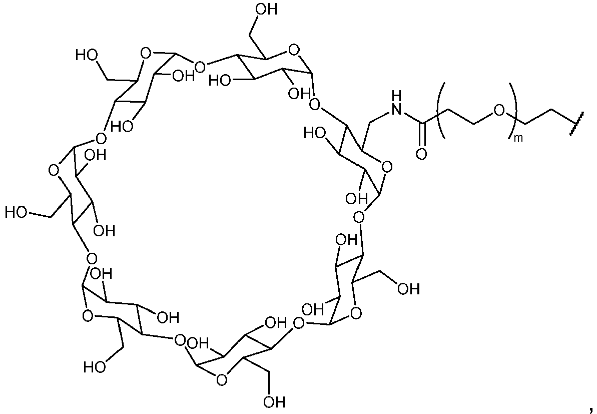 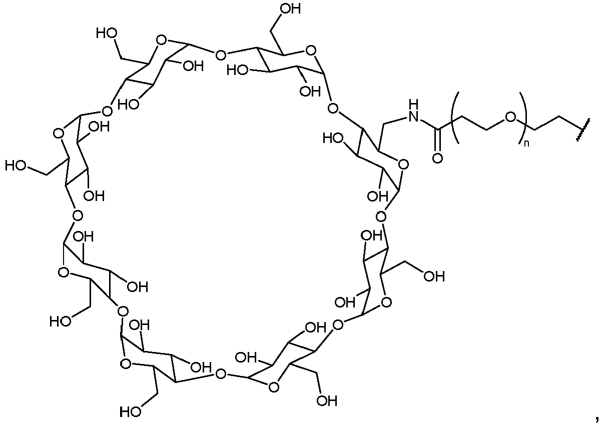 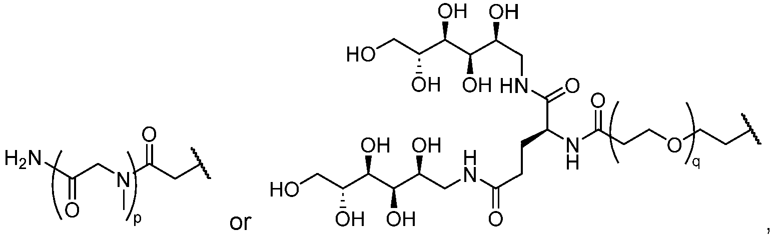 wherein
m is 2 to 12;
n is 2 to 12;
p is 1 to 20;
q is 2 to 12.

**5.** The antibody-drug conjugate according to claim 4, wherein S is: 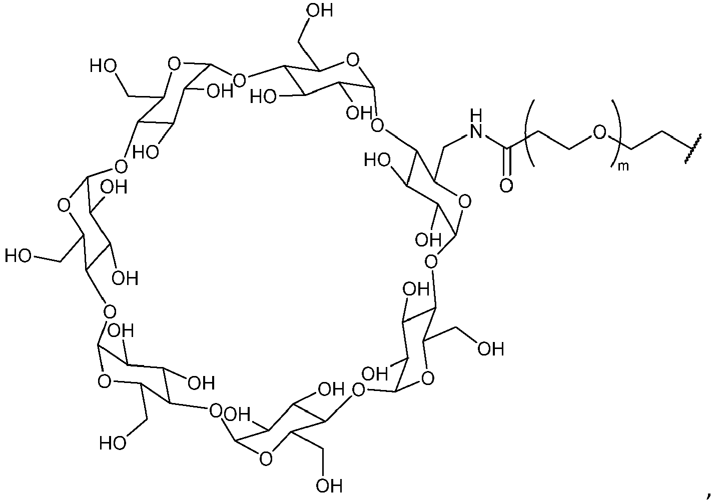 and m is 4 or 8.

**6.** The antibody-drug conjugate according to claim 4, wherein S is: 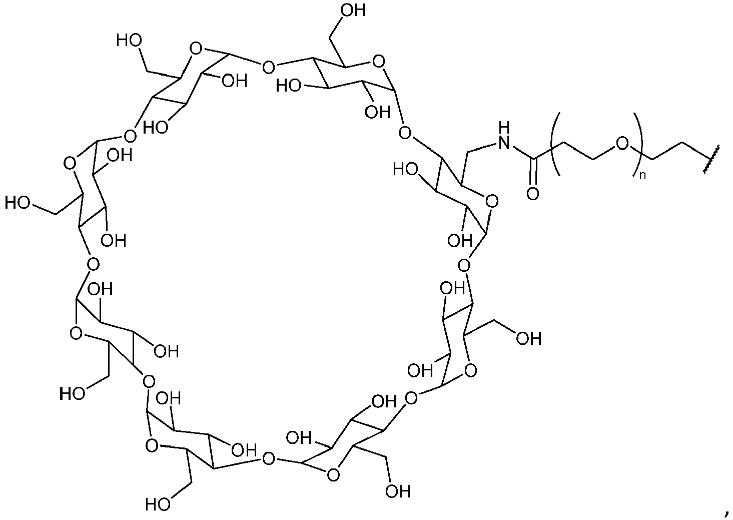 and n is 4.

**7.** The antibody-drug conjugate according to claim 4, wherein S is: 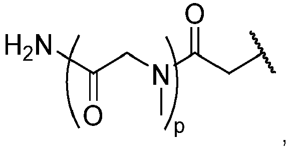 and p is 10.

**8.** The antibody-drug conjugate according to claim 4, wherein S is: 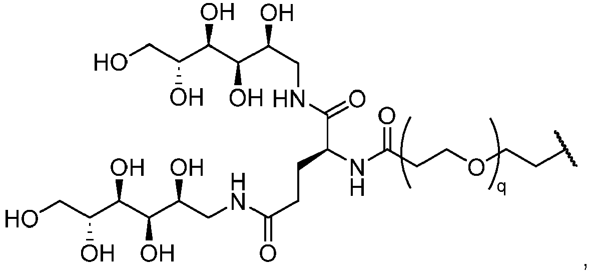 and q is 8.

**7.** The antibody-drug conjugate according to any one of claims 1 to 6, wherein the antibody specifically recognises and binds a target sequence or epitope of an antigen, preferably the antibody is one of:
- a monoclonal antibody;
- a functional antibody fragment or antibody derivative such as a single-chain variable fragment (scFv), an antigen-binding fragment such as a Fab fragment, a F(ab'), fragment, a F(ab')2 fragment or a bi- or tri-specific antibody construct;
- a Diabody,
- a Camelid Antibody,
- a Domain Antibody,
- a Nanobody,
- a bivalent homodimer with two chains consisting of scFvs,
- a shark antibody,
- an antibody consisting of new-world primate framework sequences plus nonnew world primate complementarity-determining regions (CDR) or
- a dimerised construct comprising a constant heavy chain 3 (CH₃) domain, a variable light chain (V_{L}) and a variable heavy chain (V_{H}).

**8.** The antibody-drug conjugate according to any one of claims 1 to 7, wherein the antibody is a monoclonal antibody that recognizes and binds a target sequence or epitope of Guanylate Cyclase 2C (GUCY2C), preferably human or cynomolgus Guanylate Cyclase 2C, more preferably human Guanylate Cyclase 2C.

**9.** The antibody drug conjugate according to any one of claims 1 to 8, wherein the antibody includes an antigen binding region comprising:
- an mAb1 light chain variable region (mAb1 V_{L}) complementarity-determining region (CDR_{L}) sequences CDR_{L}1 of SEQ ID NO: 1, CDR_{L}2 of SEQ ID NO: 2 and CDR_{L}3 of SEQ ID NO: 3 and
- an mAb1 heavy chain variable region (mAb1 V_{H}) CDR_{H}1 of SEQ ID NO: 4, CDR_{H}2 of SEQ ID NO: 5 and CDR_{H}3 of SEQ ID NO: 6.

**10.** The antibody drug conjugate according to any one of claims 1 to 8, wherein the antibody includes an antigen binding region comprising:
- an mAb8 light chain variable region (mAb8 V_{L}) complementarity-determining region (CDR_{L}) sequences CDR_{L}1 of SEQ ID NO: 1, CDR_{L}2 of SEQ ID NO: 2 and CDR_{L}3 of SEQ ID NO: 7 and
- an mAb8heavy chain variable region (mAb8 V_{H}) CDR_{H}1 of SEQ ID NO: 4, CDR_{H}2 of SEQ ID NO: 5 and CDR_{H}3 of SEQ ID NO: 6.

**11.** The antibody drug conjugate according to any one of claims 1 to 8, wherein the antibody includes an antigen binding region comprising:
- an mAb41 light chain variable region (mAb41 V_{L}) complementarity-determining region (CDR_{L}) sequences CDR_{L}1 of SEQ ID NO: 8, CDR_{L}2 of SEQ ID NO: 9 and CDR_{L}3 of SEQ ID NO: 10 and
- an mAb41 heavy chain variable region (mAb41 V_{H}) CDR_{H}1 of SEQ ID NO: 11, CDR_{H}2 of SEQ ID NO: 12 and CDR_{H}3 of SEQ ID NO: 13.

**12.** The antibody drug conjugate according to claim 9, wherein the antibody includes an antigen binding region comprising:
- the mAb1 V_{L} comprises a framework region 1 (FR_{L}1) of SEQ ID NO: 14, CDR_{L}1 of SEQ ID NO: 1, a FR_{L}2 of SEQ ID NO: 15, CDR_{L}2 of SEQ ID NO: 2, a FR_{L}3 of SEQ ID NO: 16, CDR_{L}3 of SEQ ID NO: 3 and a FR_{L}4 of SEQ ID NO: 17; and
- the mAb1 V_{H} comprises a framework region 1 (FR_{H}1) of SEQ ID NO: 18, CDR_{H}1 of SEQ ID NO: 4, a FR_{H}2 of SEQ ID NO: 19, CDR_{H}2 of SEQ ID NO: 5, a FR_{H}3 of SEQ ID NO: 20, CDR_{H}3 of SEQ ID NO: 6 and a FR_{H}4 of SEQ ID NO: 21.

**13.** The antibody drug conjugate according to claim 10, wherein the antibody includes an antigen binding region comprising:
- the mAb8 V_{L} comprises a framework region 1 (FR_{L}1) of SEQ ID NO: 22, CDR_{L}1 of SEQ ID NO: 1, a FR_{L}2 of SEQ ID NO: 15, CDR_{L}2 of SEQ ID NO: 2, a FR_{L}3 of SEQ ID NO: 23, CDR_{L}3 of SEQ ID NO: 7 and a FR_{L}4 of SEQ ID NO: 24; and
- the mAb8 V_{H} comprises a framework region 1 (FR_{H}1) of SEQ ID NO: 18, CDR_{H}1 of SEQ ID NO: 4, a FR_{H}2 of SEQ ID NO: 19, CDR_{H}2 of SEQ ID NO: 5, a FR_{H}3 of SEQ ID NO: 20, CDR_{H}3 of SEQ ID NO: 6 and a FR_{H}4 of SEQ ID NO: 21.

**14.** The antibody drug conjugate according to claim 11, wherein the antibody includes an antigen binding region comprising:
- the mAb41 V_{L} comprises a framework region 1 (FR_{L}1) of SEQ ID NO: 25, CDR_{L}1 of SEQ ID NO: 8, a FR_{L}2 of SEQ ID NO: 26, CDR_{L}2 of SEQ ID NO: 9, a FR_{L}3 of SEQ ID NO: 27, CDR_{L}3 of SEQ ID NO: 10 and a FR_{L}4 of SEQ ID NO: 28; and
- the mAb41 V_{H} comprises a framework region 1 (FR_{H}1) of SEQ ID NO: 29, CDR_{H}1 of SEQ ID NO: 11, a FR_{H}2 of SEQ ID NO: 30, CDR_{H}2 of SEQ ID NO: 12, a FR_{H}3 of SEQ ID NO: 31, CDR_{H}3 of SEQ ID NO: 13 and a FR_{H}4 of SEQ ID NO: 21.

**15.** The antibody drug conjugate according to claim 12, wherein the antibody includes an antigen binding region comprising:
- the mAb1 V_{L} of SEQ ID NO: 32 and
- the mAb1V_{H} of SEQ ID NO: 33.

**16.** The antibody drug conjugate according to claim 13, wherein the antibody includes an antigen binding region comprising:
- the mAb8 V_{L} of SEQ ID NO: 34 and
- the mAb8 V_{H} of SEQ ID NO: 35.

**17.** The antibody drug conjugate according to claim 14, wherein the antibody includes an antigen binding region comprising:
- the mAb41 V_{L} of SEQ ID NO: 36 and
- the mAb41 V_{H} of SEQ ID NO: 37.

**18.** The antibody drug conjugate according to any one of claims 1 to 17, wherein the antibody is: (a) a humanized or human antibody; and/or (b) an IgG type antibody, preferably an IgG1 antibody; and/or (c) a recombinant antibody.

**19.** The antibody according to claim 18, wherein the antibody comprises a heavy chain constant (Fc) region which comprises the amino acid substitution D265C, preferably said heavy chain constant (Fc) region comprises the amino acid substitutions L234A, L235A and D265C (according to EU numbering system).

**20.** The antibody according to claim 19, wherein the antibody comprises
- an mAb1-D265C heavy chain of SEQ ID NO: 39; or
- an mAb8-D265C heavy chain of SEQ ID NO: 40; or
- an mAb41-D265C of SEQ ID NO: 41.

**21.** The antibody according to claim 19, wherein the antibody comprises
- an mAb1-L234A-L235A-D265C heavy chain of SEQ ID NO: 42; or
- an mAb8-L234A-L235A-D265C heavy chain of SEQ ID NO: 43; or
- an mAb41-L234A-L235A-D265C of SEQ ID NO: 44.

**22.** The antibody-drug conjugate according to any one of claims 1 to 20, wherein the antibody-drug conjugate is:: 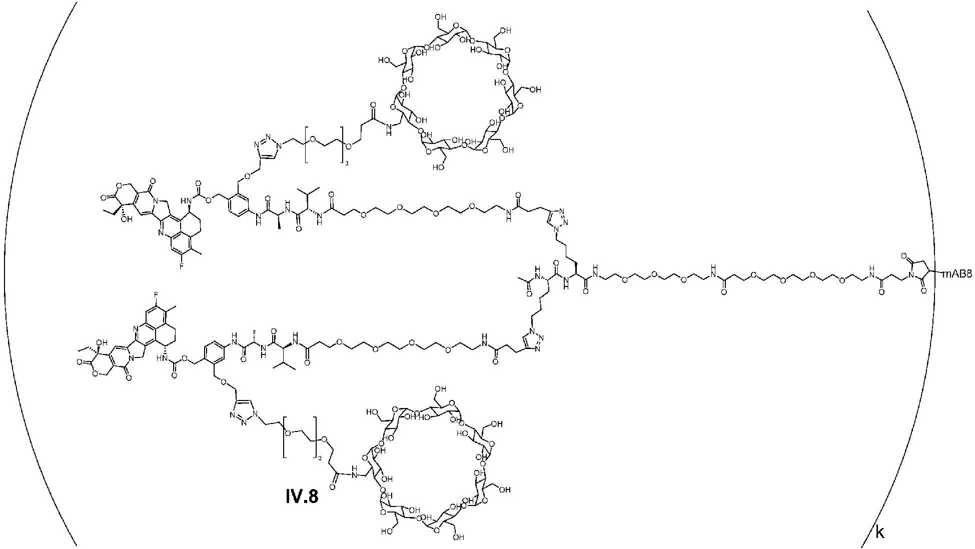
wherein k is 2; or 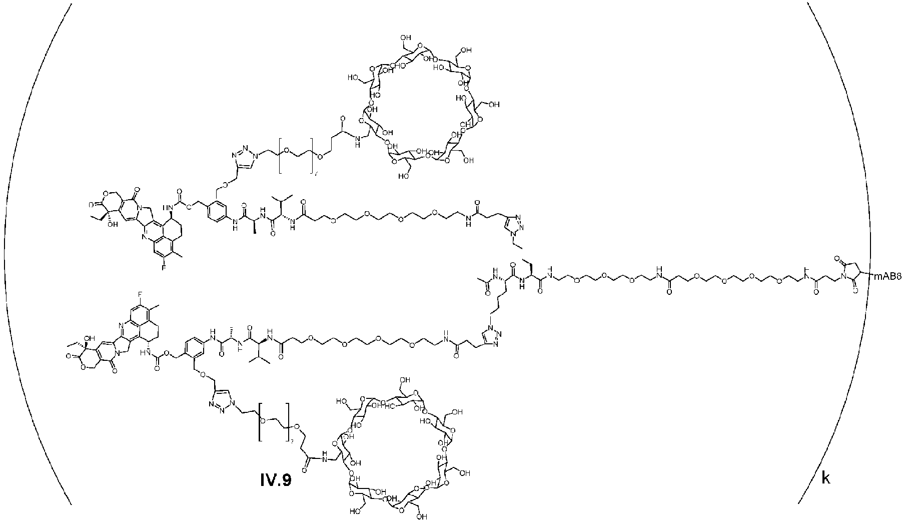
wherein k is 2; or 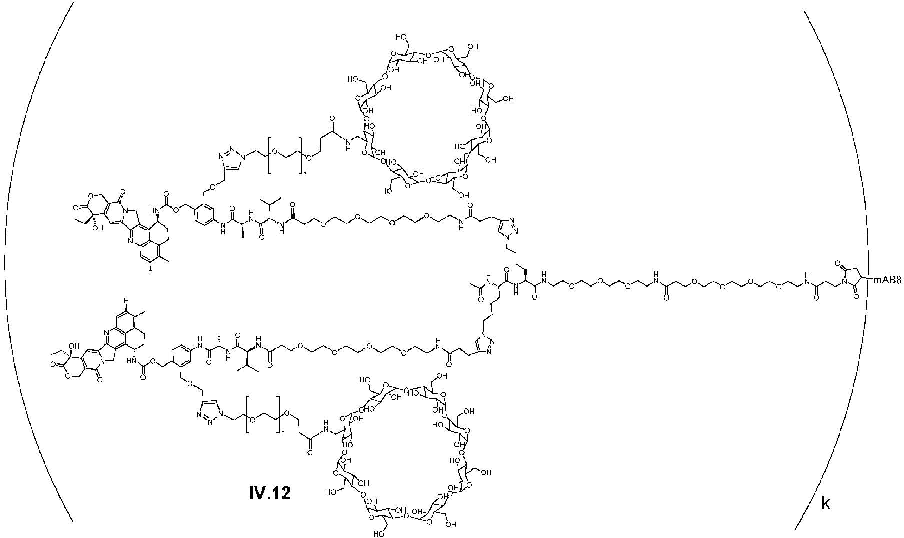
wherein k is 2; or 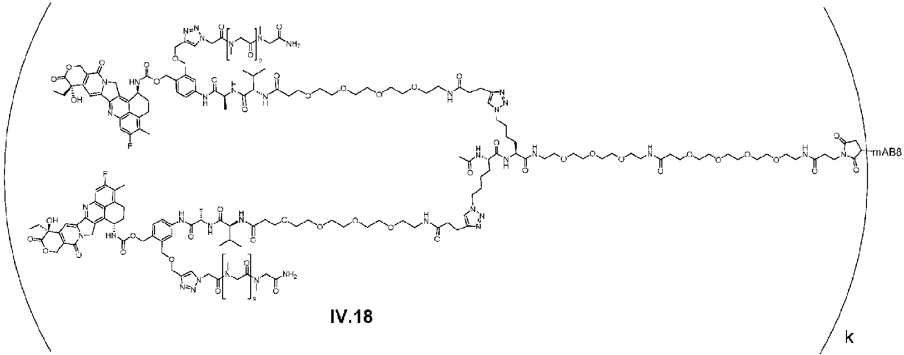
wherein k is 2; or 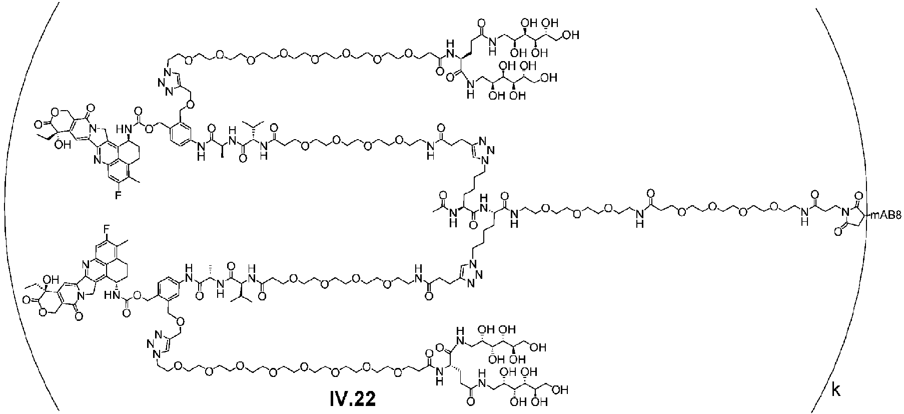
wherein k is 2.

**23.** The antibody-drug conjugate according to any one of claims 1 to 22 for use as a medicament, preferably for use in the treatment of cancer, more preferably for the treatment of gastrointestinal cancer, such as colorectal cancer (CRC), metastatic colorectal cancer (mCRC) or pancreatic cancer.

**24.** A pharmaceutical composition comprising the antibody-drug conjugate according to any one of claims 1 to 22.
